# EUROPÄISCHE PATENTSCHRIFT

(11) **EP 1 664 338 B1**
(45) Veröffentlichungstag und Bekanntmachung des Hinweises auf die Patenterteilung: **22.02.2012**
(21) Anmeldenummer: 04765182.3
(22) Anmeldetag: 14.09.2004
(51) Int. Cl.: C12Q 1/68, C12P 19/34, C12N 15/10

(54) **VERFAHREN ZUR SYNTHESE VON NUKLEINSÄUREN UND DEREN ANWENDUNG**
METHOD FOR SYNTHESIZING NUCLEIC ACIDS, AND APPLICATION THEREOF
PROCEDE DE SYNTHESE D'ACIDES NUCLEIQUES ET LEUR UTILISATION

(30) Priorität: 14.09.2003 DE 10342373
(43) Veröffentlichungstag der Anmeldung: 07.06.2006
(73) Patentinhaber: Noxxon Pharma AG, 10589 Berlin (DE); Vater, Axel, 13353 Berlin (DE)
(72) Erfinder: KLUSSMANN, Sven, 10709 Berlin (DE); JAROSCH, Florian, 13469 Berlin (DE); VATER, Axel, 13467 Berlin (DE)
(74) Vertreter: Bohmann, Armin K.
(86) Internationale Anmeldenummer: PCT/EP2004/010268
(87) Internationale Veröffentlichungsnummer: WO 2005/026374

(56) Entgegenhaltungen:
- EP-A- 0 799 834
- WO-A-00/71757
- US-A- 5 849 546

## Beschreibung

Die vorliegende Erfindung betrifft ein Verfahren zur Synthese von Nukleinsäuren.

### Funktionen von oligonukleotiden

Seit den Entdeckungen von Cech und Altman, die erstmals beschrieben, dass RNA in *C*. *elegans* nicht nur als Botenmolekül zwischen Erbgut, d. h. DNA, und Proteinsynthesemaschine, d. h. Ribosomen, dient, sondern katalytische Eigenschaften besitzt, wurden zahlreiche Arbeiten auf dem Gebiet der Ribozyme, d. h. der katalytischen RNA-Oligonukleotide, und Aptamere, d. h. Zielmolekül-bindende (Desoxy-)Oligonukleotide, publiziert. Beide Ansätze eignen sich allein oder in Kombination zum Einsatz als Therapeutika, Diagnostika, zur Target-Validierung oder als Affinitätschromatographiemedien oder als spezifische Adsorber.

Weitere Ansätze zur industriellen Verwendung von Oligonukleotiden stellen Antisense-Moleküle und auch siRNAs dar, die zu einer posttranskriptionellen Unterdrückung einer spezifischen Genexpression führen können.

### Stabitisierung von funktionellen Oligonukleotiden

Eine entscheidende Beschränkung für die Verwendung von Oligonukleotiden stellt deren schneller Abbau durch ubiquitäre RNasen und DNasen dar. Gerade in biologischen Systemen sind RNasen und DNasen in großen Mengen anzutreffen und führen zu Halbwertzeiten der RNA- und DNA-Oligonukleotide von wenigen Sekunden bis Minuten (Griffin et al., 1993; Jellinek et al, 1995; Lin et al., 1994).

Verfahren, Oligonukleotide gegen den Angriff von Exonukleasen zu stabilisieren, beruhen meist auf der Modifikation der Enden durch Anhängen von Schutzgruppen, wie z.B. ein terminales, invertiertes Nukleotid, d. h. 3'-3'- oder 5'-5'-Verknüpfung, oder anderer großer Gruppen, wie beispielsweise Polyethylenglycol (Bell et al., 1999). Vor Exo**-** und Endonukleasen gleichermaßen schützt auch der Austausch der natürlichen Substituenten, vor allem am 2'-Kohlenstoff der Ribose und am Phosphor. Unnatürliche, nukleaseresistentere Alternativen zu Ribose (2'-OH) oder Desoxyribose (2'-H) sind: 2'-Amino-, 2'-Fluoro-, 2'-Azido-, 2'-O-Methyl-, 2'-Alkyl-, 2'-Allyl und Arabino-Nukleotide (Eaton and Pieken, 1995). Die häufigste Phosphor-Modifikation ist der Austausch von Sauerstoff durch Schwefel, was zu sogenannten Phosphorothioaten führt.

Durch Maßnahmen dieser Art können signifikant höhere Halbwertzeiten in biologischen Flüssigkeiten, bis hin zu vielen Stunden, erzielt werden (Eaton and Pieken, 1995; Green et al., 1995; Jellinek et al., 1995; Lin et al., 1994).

Jede der hier genannten Modifikationen bringt jedoch gewisse Einschränkungen mit sich. Insbesondere ist die Herstellung der modifizierten Oligonukleotide mit DNA- oder RNA-Polymerasen meist nicht möglich. Dies ist jedoch häufig eine Bedingung zur Identifizierung funktionaler Oligonukleotide. Denn gerade durch die Oligonukleotiden eigene direkte Verknüpfung des Phänotyps (die Struktur und somit die Funktion) mit dem Genotyp (die Nukleotidsequenz) und ihre Eigenschaft kopierbar zu sein, ist es möglich, durch Selektion und Amplifikation geeignete Nukleinsäuresequenzen aus natürlichen Bibliotheken, wie sie das Genom oder das Transkriptom darstellen, oder synthetischen Bibliotheken, bspw. kombinatorischen Bibliotheken, so stark anzureichern, dass einzelne Sequenzen mit den gewünschten Eigenschaften isoliert werden können.

Solche Modifikationen, die nicht mit den enzymatischen Prozessen vereinbar sind, können erst im Anschluss an die eigentliche Identifizierung der funktionellen Oligonukleotide durch chemische Synthese der identifizierten Sequenzen eingefügt werden. Für diese ist zunächst die chemische Synthetisierbarkeit der identifizierten Sequenzen vonnöten. Daher müssen die RNA-Hybrid-Kandidaten, die zumeist eine Länge von etwa 60 - 90 Nukleotiden haben, zunächst auf 50 Nukleotide oder weniger verkürzt werden, um eine effiziente chemische Synthese zu gewährleisten. Anschließend werden die unmodifizierten Purine durch 2'-modifizierten Nukleotide ausgetauscht. Häufig wird dadurch die Funktion der Oligonukleotide beeinträchtigt (Kujau et al., 1997). Daher können in den seltensten Fällen alle Nukleotide nachträglich modifiziert werden. Dadurch weisen die meisten stabilisierten Oligonukleotide einige durch Nukleasen angreifbare Schwachstellen im Molekül auf, wodurch ihre Lebensdauer verringert wird.

### Bisherige enzymatische Verfahren zur Stabilisierung von Oligonukleotiden

Phosphorothioate können enzymatisch mittels RNA-Polymerasen eingebaut werden (Jhaveri et al., 1998) oder über Taq-DNA-Polymerase, wobei es jedoch nur möglich ist, bis zu drei Phosphorothioat-dNTPs einzubauen (King et al., 2002). Phosphorothioate weisen darüber hinaus den Nachteil auf, dass die zytotoxisch sind (Henry et al., 2001).

Durch Transkription können 2'-Fluoro- und 2'-Aminomodifikationen in RNA eingebracht werden. Eine Übersicht über modifizierte Aptamere wurde von Kusser zusammengestellt (Kusser, 2000). Auch der enzymatische Einbau von 2'-OMethyl- und 2'-Azidonukleosidtriphosphaten mit T7 RNA-Polymerase ist beschrieben (Lin et al., 1994; Padilla and Sousa, 1999; Padilla and Sousa, 2002). Der Einbau dermaßen modifizierter Nukleotide ist allerdings gegenwärtig auf modifizierte Cytidine und Uridine beschränkt (Aurup et al., 1992). Insbesondere 2'-modifizierte Guanosine werden nicht von bekannten RNA-Polymerasen toleriert. Die Ursache dafür liegt vermutlich in der Instabilität des Polymerase-DNA-Komplexes während der Initiationsphase der RNA-Polymerisation. Diese die Polymerisation der ersten 8-12 Nukleotide umfassende Phase muss möglichst zügig verlaufen, da die RNA-Polymerase sonst den Komplex schnell wieder verlässt (Lin et al., 1994; Milligan and Uhlenbeck, 1989).

Bedingung für eine erfolgreiche Initiation der RNA-Synthese ist jedoch mindestens ein Guanosin an den ersten beiden zu transkribierenden Positionen. Eine optimale Initiationssequenz sieht sogar drei konsekutive Guanosine an den Positionen 1-3 der RNA für T7 und T3-RNA-Polymerase vor (Milligan and Uhlenbeck, 1989) und GAAGNG für die SP6 RNA-Polymerase, wie beispielsweise dargestellt in Meador et al., 1995.

Daher ist es bisher praktisch nicht möglich, 2'-modifizierte Guanosinnukleotide enzymatisch in RNA-Moleküle einzubauen bzw. vollständig 2'-modifizierte Nukleinsäuren mittels RNA-Polymerasen herzustellen.

Die international Patentanmeldung WO 00/71757 betrifft Zusammensetzungen und Verfahren zum Markieren von Nukleinsäuremolekülen unter Verwendung von reversen Transkriptasen.

Die Europäische Patentanmeldung EP 0 799 834 offenbart modifizierte Nukleotide, die von reversen Transkriptasen akzeptiert werden.

Das US-Patent 5,849,546 beschreibt ein Verfahren zur Synthese eines Nukleinsäuremoleküls unter Verwendung einer mutierten Polymerase.

Der vorliegenden Erfindung lag somit die Aufgabe zugrunde, ein enzymatisches Verfahren bereitzustellen, welches den Einbau von modifizierten Nukleotiden und insbesondere von 2'-Fluoro-modifizierten Nukleotiden in eine Nukleinsäure erlaubt. Dabei war es insbesondere eine Aufgabe, ein Verfahren bereitzustellen, welches erlaubt, für alle fünf natürlicherweise vorkommenden Basen (A, C, G, T, U) Basenmodifikationen derselben und mögliche Universal-Basen, wie beispielsweise Inosin (I), als in Nukleinsäuren vorkommende Nukleosidphosphate in ihrer zuckermodifizierten Form in eine Nukleinsäure einzubauen.

Eine weitere der vorliegenden Erfindung zugrundeliegende Aufgabe war die Bereitstellung eines enzymatischen Syntheseverfahrens für Nukleinsäuren, wobei die Nukleinsäuren vollständig aus modifizierten Nukleosidphosphaten, insbesondere 2-Fluoro-modifizierten Nukleosidphosphaten bestehen.

Diese Aufgabe wird erfindungsgemäß durch die Verfahren gemäß den unabhängigen Ansprüchen gelöst. Bevorzugte Ausführungsformen ergeben sich aus den Unteransprüchen.

Genauer wird die Aufgabe in einem ersten Aspekt erfindungsgemäß gelöst durch ein Verfahren zur Synthese einer Nukleinsäure, wobei die Nukleinsäure modifizierte Nukleotide umfasst, umfassend die Schritte:
- Bereitstellen eines Matrizenstranges;
- Bereitstellen eines zumindest teilweise an dem Matrizenstrang hybridisierenden Primers;
- Bereitstellen von Nukleosidtriphosphaten, wobei ein Teil der Nukleosidtriphosphate modifizierte Nukleosidtriphosphate sind;
- Bereitstellen einer Polymerase-Aktivität; und
- Inkubation des Matrizenstranges, des Primers, der Nukleosidtriphosphate zur Synthese einer zum Matrizenstrang im wesentlichen komplementären Nukleinsäure,
dadurch gekennzeichnet, dass die Polymerase-Aktivität eine reverse Translaiptaseaktivität ist die ausgewählt aus der Gruppe, die Reverse Transkriptasen von Murine Moloney Leukemia Virus (MMLV), Avian Myeloblastosis Virus (AMV), thermostabile Reverse Transhiptasen, DNA-Polymerase von *Carboxydothermus hydrogenoformans* jeweilige Mutanten davon und Gemische davon umfasst, und die modifizierten Nukleosidtriphosphate ausgewählt sind aus der Gruppe, die 2'-Fluoro-modifizierte Nukleosidtriphosphate, 2'-Amino-modifizierte Nukleosidtriphosphate, 2'-Azido-modifizierte Nukleosidtriphosphate, 2'-O-Methyl-modifizierte Nukleosidtriphosphate, 2'-Alkyl-modifizierte Nukleosidtriphosphate, und 2'-Allyl-modifizierte Nukleosidtriphosphate umfasst.

In einer Ausführungsform sind die modifizierten Nukleosidtriphosphate 2'-Fluoro-Nukleosidtriphosphate.

In einer Ausführungsform sind die bereitgestellten Nukleosidtriphosphate ausschließlich modifizierte Nukleosidtriphosphate und die synthetisierte Nukleinsäure besteht bevorzugterweise im wesentlichen ausschließlich aus modifizierten Nukleotiden.

In einer Ausführungsform besteht der Matrizenstrang aus RNA.

In einer alternativen Ausführungsform besteht der Matrizenstrang aus DNA.

In einer weiteren alternativen Ausführungsform besteht der Matrizenstrang aus einer modifizierten Nukleinsäure, bevorzugterweise einer 2'-Fluoro-Nukleinsäure.

In einer alternativen Ausführungsform ist die Sequenz des Primers Teil der zu synthetisierenden Nukleinsäure.

In einer Ausführungsform ist die Sequenz des Primers von der zu synthetisierenden Nukleinsäure verschieden.

In einer Ausführungsform besteht der Primer aus modifizierten Nukleosidphosphaten, wobei die Modifikation der Nukleosidphosphate des Primers die gleiche Modifikation wie die der bereitgestellten Nukleosidtriphosphate ist.

In einer Ausführungsform besteht der Primer aus RNA.

In einer Ausführungsform besteht der Primer aus DNA, wobei mindestens das 3'-terminale Nukleotid des Primers ein Desoxyribonukleotid ist.

In einer Ausführungsform synthetisiert die Polymerase-Aktivität einen zum Matrizenstrang im wesentlichen komplementären Strang, wobei dieser bevorzugterweise mit dem Matrizenstrang basengepaart vorliegt.

In einer bevorzugten Ausführungsform wird die synthetisierte Nukleinsäure von dem Matrizenstrang getrennt.

In einer Ausführungsform wird der Primer oder ein Teil davon von der durch die Polymerase-Aktivität synthetisierten Nukleinsäure entfernt

In einer Ausführungsform wird der Matrizenstrang und/oder der Primer verdaut oder gespalten, bevorzugterweise nach der Synthese der zum Matrizenstrang im wesentlichen komplementären Nukleinsäure.

In einer bevorzugten erfolgt Ausführungsform das Trennen und/oder das Spalten durch alkalische Spaltung oder enzymatische Aktivität.

In einem zweiten Aspekt wird die Aufgabe erfindungsgemäß gelöst durch die Verwendung einer reversen Transkriptase zur Synthese einer Nukleinsäure, wobei die Nukleinsäure mindestens ein modifiziertes Nukleosidphosphat enthält die reverse Transkriptase ausgewählt aus der Gruppe, die Reverse Transkriptasen von Murine Moloney Leukemia Virus (MMLV), Avian Myeloblastosis Virus (AMV), thermostabile Reverse Transkriptasen, DNA-Polymerase von *Carboxydothermus hydrogenoformans,* Mutanten davon und Gemische davon umfasst, und das modifizierte Nukleosidtriphosphat ausgewählt ist aus der Gruppe, die 2'-Fluoro-modifizierte Nukleosidtriphosphate, 2'-Amino-modifizierte Nukleosidtriphosphate, 2'-Azido-modifizierte Nukleosidtriphosphate, 2'-O-Methyl-modifizierte Nukleosidtriphosphate, 2'-Alkyl-modifizierte Nukleosidtriphosphate und 2'-Allyl-modifizierte Nukleosidtriphosphate, umfasst.

In einer Ausführungsform besteht die Nukleinsäure im wesentlichen vollständig aus modifizierten Nukleosidphosphaten.

Ebenfalls offenbart wird ein Verfahren zur Selektion einer an ein Zielmolekül bindenden Nukleinsäure, insbesondere Aptamere, umfassend die Schritte:
(a) Bereitstellen einer heterogenen Population von Nukleinsäuren, insbesondere D-Nukleinsäuren, wobei eine jede Nukleinsäure einen Bereich mit einer randomisierten Sequenz und einer ersten konstanten Sequenz am 5'-Ende und einer zweiten konstanten Sequenz am 3'-Ende aufweist und sich die die Population ausbildenden Nukleinsäuren in der randomisierten Sequenz unterscheiden,
(b) Inkontaktbringen der Population von Nukleinsäuren mit dem Zielmolekül,
(c) Abtrennen der Nukleinsäuren, die nicht mit dem Zielmolekül in Wechselwirkung getreten sind,
(d) Abtrennen der Nukleinsäure(n), die mit dem Zielmolekül in Wechselwirkung getreten ist/sind, von dem Zielmolekül,
(e) optional Wiederholen der Schritte von (a) bis (d), wobei die Nukleinsäure(n) aus Schritt (d) die heterogene Population ausbilden oder in dieser enthalten sind,
(f) reverse Transkription der Nukleinsäure(n), die mit dem Zielmolekül in Wechselwirkung getreten ist/sind, um reverse Transkriptionsprodukte zu bilden,
(g) Durchführen einer Zweitstrangsynthese, wobei der zweite Strang im Wesentlichen komplementär ist zu den reversen Transkriptionsprodukten, wobei die Zweitstrangsynthese bevorzugterweise eine Amplifikationsreaktion und bevorzugterweise eine Polymerasekettenreaktion ist,
(h) Transkription, des Produktes aus (g), wobei der synthetisierte Zweitstrang als Matrizenstrang dient, um Transkriptionsprodukte zu erhalten,
(i) Synthese von Nukleinsäuren, die im wesentlichen komplementär zu den Transkriptionsprodukten sind,
(j) optional Wiederholen der Schritte (a) bis (i), wobei die Nukleinsäure(n) aus Schritt (i) die heterogene Population ausbilden oder in dieser enthalten sind, und
(k) optional Sequenzieren der aus Schritt (f) oder (g) erhaltenen Nukleinsäure(n),
dadurch gekennzeichnet, dass die Synthese gemäß Schritt (i) gemäß einem Verfahren nach dem ersten oder zweiten Aspekt durchgeführt wird.

Desweiteren wird offenbart ein Verfahren zur Selektion einer an ein Zielmolekül bindenden Nukleinsäure, insbesondere Aptamere, umfassend die Schritte:
(a) Bereitstellen einer heterogenen Population von Nukleinsäuren, insbesondere D-Nukleinsäuren, wobei eine jede Nukleinsäure einen Bereich mit einer randomisierten Sequenz und einer ersten konstanten Sequenz am 5'-Ende und einer zweiten konstanten Sequenz am 3'-Ende aufweist und sich die die Population ausbildenden Nukleinsäuren in der randomisierten Sequenz unterscheiden,
(b) Inkontaktbringen der Population von Nukleinsäuren mit dem Zielmolekül,
(c) Abtrennen der Nukleinsäure, die nicht mit dem Zielmolekül in Wechselwirkung getreten sind,
(d) Abtrennen der Nukleinsäure(n), die mit dem Zielmolekül in Wechselwirkung getreten ist/sind, von dem Zielmolekül,
(e) Optional Wiederholen der Schritte von (a) bis (d), wobei die Nukleinsäure(n) aus Schritt (d) die heterogene Population ausbilden oder in dieser enthalten sind,
(f) reverse Transkription der Nukleinsäure(n), die mit dem Zielmolekül in Wechselwirkung getreten ist/sind, um reverse Transkriptionsprodukte zu bilden,
(g) Durchführen einer Zweitstrangsynthese, wobei der zweite Strang im Wesentlichen komplementär ist zu den reversen Transkriptionsprodukten, wobei die Zweitstrangsynthese bevorzugterweise eine Amplifikationsreaktion und bevorzugterweise eine Polymerasekettenreaktion ist,
(h) Transkription, des Produktes aus Schritt (g), wobei der synthetisierte Zweitstrang als Matrizenstrang dient, um Transkriptionsprodukte zu erhalten
(i) Synthese von Nukleinsäuren, die im wesentlichen komplementär zu den Transkriptionsprodukten sind,
(j) optional Wiederholen der Schritte (a) bis (i), wobei die Nukleinsäure(n) aus Schritt (i) die heterogene Population ausbilden oder in dieser enthalten sind, und
(k) optional Sequenzieren der aus Schritt (f) oder (g) erhaltenen Nukleinsäure(n)
dadurch gekennzeichnet, dass zumindest der randomisierte Bereich der Nukleinsäure und/oder in Schritt (i) synthetisierte Nukleinsäure im wesentlichen vollständig aus modifizierten Nukleosidphosphaten besteht, bevorzugterweise aus 2'-FluoroNukleosidphosphaten.

Desweiteren wird offenbart ein Verfahren zur Selektion einer an ein Zielmolekül bindenden Nukleinsäure, insbesondere von Aptameren, insbesondere ein Verfahren wie hierin offenbart , umfassend die Schritte:
(a) Bereitstellen einer heterogenen Population von Nukleinsäuren, insbesondere D-Nukleinsäuren, wobei eine jede Nukleinsäure einen Bereich mit einer randomisierten Sequenz und einer ersten konstanten Sequenz am 5'-Ende und einer zweiten konstanten Sequenz am 3'-Ende aufweist und sich die die Population ausbildenden Nukleinsäuren in der randomisierten Sequenz unterscheiden,
(b) Inkontaktbringen der Population von Nukleinsäuren mit dem Zielmolekül,
(c) Abtrennen der Nukleinsäure, die nicht mit dem Zielmolekül in Wechselwirkung getreten sind,
(d) Abtrennen der Nukleinsäure(n), die mit dem Zielmolekül in Wechselwirkung getreten ist/sind, von dem Zielmolekül,
(e) optional Wiederholen der Schritte von (a) bis (d), wobei die Nukleinsäure(n) aus Schritt (d) die heterogene Population ausbilden oder in dieser enthalten sind,
(f) reverse Transkription der Nukleinsäure(n), die mit dem Zielmolekül in Wechselwirkung getreten ist/sind, um reverse Transkriptionsprodukte zu bilden,
(g) Durchführen einer Zweitstrangsynthese, wobei der zweite Strang im Wesentlichen komplementär ist zu den reversen Transkriptionsprodukten, wobei die Zweitstrangsynthese bevorzugterweise eine Amplifikationsreaktion und bevorzugterweise eine Polymerasekettenreaktion ist,
(h) Transkription des Produktes aus Schritt (g), wobei der synthetisierte Zweitstrang als Matrizenstrang dient, um Transkriptionsprodukte zu erhalten
(i) Synthese von Nukleinsäuren, die im wesentlichen komplementär sind zu den Transktiptionsprodukten,
(j) optional Wiederholen der Schritte (a) bis (i), wobei die Nukleinsäure(n) aus Schritt (i) die heterogene Population ausbilden oder in dieser enthalten sind, und
(k) optional Sequenzieren der aus Schritt (f) oder (g) erhaltenen Nukleinsäure(n),
   dadurch gekennzeichnet, dass
   - die erste konstante Sequenz der Nukleinsäure in Schritt (a) eine Forward-Primer-Sequenz und die zweite konstante Sequenz eine Reverse-Primer-Bindungsstelle umfasst, und
   - ein Reverse-Primer in der reversen Transkription gemäß Schritt (f) verwendet wird, der im wesentlichen komplementär zur Reverse-Primer-Bindungsstelle ist und an seinem 5'-Ende einen weiteren Teilbereich umfasst und das reverse Transkriptionsprodukt in 5' → 3'-Richtung eine Reverse-Primer-Sequenz, eine zur randomisierten Sequenz im Wesentlichen komplementäre Sequenz und eine Forward-Primer-Bindungsstelle umfasst.

Dabei ist vorgesehen, dass in der Zweitstrangsynthese der Reverse-Primer und ein Forward-Primer verwendet, wird wobei der Forward-Primer zumindest mit einem Teil der Forward-Primer-Bindungsstelte des reversen Transkriptionsproduktes im wesentlichen komplementär ist, wobei die Sequenz des synthetisierten Zweitstrangs mit der Sequenz der zu Nukleinsäure von Schritt (d) im wesentlichen identisch ist und am 3'-Ende zusätzlich eine weiteren Teilbereich des Reverse-Primers im Wesentlichen komplementäre Sequenz umfasst.

Dabei ist vorgesehen, dass der weitere Teilbereich des Reverse-Primers eine Promotor-Sequenz ist, wobei bevorzugterweise die Promotor-Sequenz ausgewählt ist aus der Gruppe, die Promotor-Sequenzen von T7-RNA-Polymerase, T3-RNA-Polymerase und SP6-Polymerase umfasst.

Es ist vorgesehen, dass der in der Zweitstrangsynthese synthetisierte Strang als Matrizenstrang einer Transkription unterzogen, wird wobei das Transkriptionsprodukt in 3' → 5'-Richtung die Forward-Primer-Bindungsstelle den komplementären randomisierten Bereich und die Reverse-Primer-Sequenz umfasst.

Es ist vorgesehen, dass das Transkriptions-Produkt mit einer reversen Transkriptase zusammen mit einem Forward-Synthese-Primer und modifizierten Nukleosidtriphosphaten, bevorzugterweise 2'-Fluoronukleosidphosphaten, umgesetzt wird, wobei der Forward-Synthese-Primer an der Forward-Primer-Bindungsstelle hybridisiert, um ein Syntheseprodukt zu erhalten, wobei das Syntheseprodukt modifizierte Nukleosidphosphate, bevorzugterweise 2'-Fluoro-Nukleosidphosphate umfasst.

Es ist vorgesehen, dass besteht der Forward-Synthese-Primer aus modifizierten Nukleosidtriphosphaten besteht.

Es ist vorgesehen, dass der Matrizenstrang einer alkalischen Behandlung unterzogen wird, um eine einzelsträngige Nukleinsäure zu erhalten, wobei die Nukleinsäure in 5' → 3'-Richtung die Forward-Primer-Sequenz, den randomisierten Bereich und die Reverse-Primer-Bindungsstelle umfasst.

Es ist vorgesehen, dass der Forward-Primer an seinem 5'-Ende einen weiteren Teilbereich und der synthetisierte Zweitstrang an seinem 5'-Ende eine dem weiteren Teilbereich entsprechende Sequenz umfasst.

Es ist vorgesehen, dass der in der Zweitstrangsynthese synthetisierte Strang als Matrizenstrang einer Transkription unterzogen wird, wobei das Transkriptionsprodukt in 3' → 5'-Richtung die Forward-Primer-Bindungsstelle einschließlich der zu dem weiteren Teilbereich des Forward-Primers komplementären Sequenz, den komplementären randomisierten Bereich und die Reverse-Primer-Sequenz an seinem 5'-Ende umfasst, wobei der Reverse-Primer-Sequenz bevorzugterweise eine zu dem weiteren Teilbereich des Reverse-Primers korrespondierende Sequenz fehlt.

Es ist vorgesehen, dass das Transkriptions-Produkt mit einer reversen Transkriptase zusammen mit einem Forward-Synthese-Primer und modifizierten Nukleosidtriphosphaten, bevorzugterweise 2'-Fluoronukleosidphosphaten, umgesetzt wird, wobei der Forward-Synthese-Primer an der Forward-Primer-Bindungsstelle hybridisiert, um ein Syntheseprodukt zu erhalten, wobei das Syntheseprodukt modifizierte Nukleosidphosphate, bevorzugterweise 2'-Fluoro-Nukleosidphosphate umfasst.

Es ist vorgesehen, dass der Forward-Synthese-Primer aus Ribonukleotiden oder aus Desoxyribonukleotiden mit mindestens einem Ribonukleotid an seinem 3'-Ende besteht.

Es ist vorgesehen, dass der Matrizenstrang einer alkalischen Spaltung unterzogen und der Forward-Synthese-Primer abgespalten wird, um eine einzelsträngige Nukleinsäure zu erhalten, wobei die Nukleinsäure in 5' → 3'-Richtung die Forward-Primer-Sequenz, den randomisierten Bereich und die Reverse-Primer-Bindungsstelle umfasst.

Es ist vorgesehen, dass der Forward-Primer und der Reverso-Primer aus DNA besteht

Es wird offenbart ein Verfahren zur Selektion einer an ein Zielmolekül bindenden Nukleinsäure, insbesondere von Aptameren, umfassend die Schritte
(a) Bereitstellen einer heterogenen Population von Nukleinsäuren, insbesondere D-Nukleinsäuren, wobei eine jede Nukleinsäure einen Bereich mit einer randomisierten Sequenz und einer ersten konstanten Sequenz am 5'-Ende und einer zweiten konstanten Sequenz am 3'-Ende aufweist und sich die die Population ausbildenden Nukleinsäuren in der randomisierten Sequenz unterscheiden,
(b) Inkontaktbringen der Population von Nukleinsäuren mit dem Zielmolekül,
(c) Abtrennen der Nukleinsäure, die nicht mit dem. Zielmolekül in Wechselwirkung getreten sind,
(d) Abtrennen der Nukleinsäure(n), die mit dem Zielmolekül in Wechselwirkung getreten ist/sind, von dem Zielmolekül,
(e) optional Wiederholen der Schritte von (a) bis (d), wobei die Nukleinsäure(n) aus Schritt (d) die heterogene Population ausbilden oder in dieser enthalten sind,
(f) reverse Transkription der Nukleinsäure(n), die mit dem Zielmolekül in Wechselwirkung getreten ist/sind, um reverse Transkriptionsprodukte zu bilden,
(g) Durchrühren einer Amplifikationsreaktion mit dem reversen Transkriptionsprodukt wobei die Amplifikationsreaktion bevorzugterweise eine Polymerasekettenreaktion ist, um ein amplifiziertes reverses Transkriptionsprodukt zu erhalten,
(h) Synthese von Nukleinsäuren, die im Wesentlichen komplementär sind zu den in (g) amplifizierten reversen Transktiptionsprodukten, um ein Syntheseprodukt zu erhalten,
(i) optional Wiederholen der Schritte (a) bis (h), wobei die Nukleinsäure(n) aus Schritt (h) die heterogene Population ausbilden oder in dieser enthalten sind,
(j) optional Sequenzieren der aus Schritt (f) oder (g) erhaltenen Nukleinsäure,
   dadurch gekennzeichnet, dass
   - die erste konstante Sequenz der Nukleinsäure in Schritt (a) eine Forward-Primer-Sequenz und die zweite konstante Sequenz eine Reverse-Primer-Bindungsstelle umfasst, und
   - ein Reverse-Primer in der reversen Transkription gemäß Schritt (f) verwendet wird, der im Wesentlichen komplementär zu Reverse-Primer-Bindungsstelle ist und das reverse Transkriptionsprodukt in 5' → 3'-Richtung eine Reverse-Primer-Sequenz, eine zur randomisierten Sequenz im Wesentlichen komplementäre Sequenz und eine zur Forward-Primer-Seqüenz im Wesentlichen komplementäre Forward-Primer-Bindungsstelle umfasst.

Es ist vorgesehen, dass in der Zweitstrangsynthese der Reverse-Primer und ein Forward-Primer verwendet wird wobei der Forward-Primer mit der Forward-Primer-Bindungsstelle im Wesentlichen komplementär ist, wobei die Sequenz des synthetisierten Zweitstranges mit der zu amplifizierenden Nukleinsäure im Wesentlichen identisch ist.

Es ist vorgesehen, dass in der Synthese nach Schritt (g) das amplifizierte reverse Transkriptionsprodukt mit einem Forward-Synthese-Primer, modifzierten Nukleosidtriphosphaten, bevorzugterweise 2'-Fluoro-Nukleosidtriphosphaten, und einer reversen Transkriptase umgesetzt wird, wobei der Forward-Synthese-Primer an der Forward-Primer-Bindungsstelle hybridisiert, um ein Syntheseprodukt zu erhalten, wobei das Syntheseprodukt modifizierte Nukleosidphosphate, bevorzugterweise 2'-Fluoro-Nukleosidphosphate umfasst.

Es ist vorgesehen, dass der Forward-Synthese-Primer aus modifizierten Nukleosidtriphosphaten besteht.

Es ist vorgesehen, dass der Matrizenshaag einem Verdau, bevorzugterweise einem enzymatischen Verdau, unterzogen wird, um eine einzelsträngige Nukleinsäure zu erhalten, wobei die Nukleinsäure in 5' → 3'-Richtung die Forward-Primer-Sequenz, den randomisierten Bereich und die Reverse-Primer-Bindungsstelle umfasst.

Es ist vorgesehen, dass in der Zweitstrangsynthese der Reverse-Primer und ein Forward-Primer verwendet werden, wobei der Forward-Primer mit der Forward-Primer-Bindungsstelle im Wesentlichen komplementär ist und an seinem 5'-Ende einen weiteren Teilbereich umfasst, wobei der Teilbereich bevorzugterweise eine Länge etwa von 10 bis 25 und bevorzugtsrerweise etwa von 10 bis 15 Nukleotide aufweist, wobei der Teilbereich bevorzugterweise eine Bindungsstelle oder einen Teil davon für einen Forward-Synthese-Primer ist und ein verlängertes reverses Transhiptionsprodukt erhalten wird, wobei das verlängerte reverse Transkriptionsprodukt dem reversen Transkriptionsprodukt entspricht, wobei das reverse Transkriptionsprodukt an seinem 3'-Ende um eine Sequenz ergänzt wird, wobei die Sequenz komplementär zur Sequenz des weiteren Teilbereichs des Forward-Primers ist.

Es ist vorgesehen, dass in der Synthese nach Schritt (g) das amplifizierten reverse Translaiptionsprodukt umgesetzt wird, mit einem Forward-Synthese-Primer, modifizierten Nukleosidtriphosphaten, bevorzugterweise 2'-Fluoro-Nukleosidtriphosphate, und einer reversen Transkriptase, wobei der Forward-Synthese-Primer an der Bindungsstelle für den Forwerd-Synthese-Primer hybridisiert, um ein Syntheseprodukt zu erhalten, wobei das Syntheseprodukt modifizierte Nukleosidphosphate, bevorzugterweise 2'-Fluoro-Nukleosidphosphate umfasst.

Es ist vorgesehen, dass der Forward-Synthese-Primer aus Ribonukleotiden oder aus Desoxynbonukleotiden mit mindestens einem Ribonukleotid an seinem 3'-Ende besteht.

Es ist vorgesehen, dass der Matrizenstrang einem Verdau unterzogen wird, bevorzugterweise einem enzymatischen Verdau, um eine einzelsträngige Nukleinsäure zu erhalten, wobei die Nukleinsäure in 5' → 3'-Richtung die Forward-Primer-Sequenz, den randomisierten Bereich und die Reverse-Primer-Bindungsstelle umfasst.

Es ist vorgesehen, dass der Forward-Primer und der Reverse-Primer aus DNA besteht.

Es wird offenbart ein Verfahren zur Selektion einer an ein Zielmolekül bindenden Nukleinsäure, insbesondere von Aptameren, umfassend die Schritte:
(a) Bereitstellen einer heterogenen Population von Nukleinsäuren, insbesondere D-Nukleinsäuren, wobei eine jede Nukleinsäure einen Bereich mit einer randomisierten Sequenz und einer ersten konstanten Sequenz am 5'-Ende und einer zweiten konstanten Sequenz am 3'-Ende aufweist und sich die die Population ausbildenden Nukleinsäuren in der randomisierten Sequenz unterscheiden,
(b) Inkontaktbringen der Population von Nukleinsäuren mit dem Zielmolekül,
(c) Abtrennen der Nukleinsäure, die nicht mit dem Zielmolekül in Wechselwirkung getreten sind,
(d) Abtrennen der Nukleinsäure(n), die mit dem Zielmolekül in Wechselwirkung getreten ist/sind, von dem Zielmolekül,
(e) optional Wiederholen der Schritte von (a) bis (d), wobei die Nukleinsäure(n) aus Schritt (d) die heterogene Population ausbilden oder in dieser enthalten sind,
(f) Amplifikation der Nukleinsäure von Schritt (a) umfassend den Schritt:
   Umsetzen der Nukleinsäure von Schritt (a) mit einer reversen Transkriptase, einem Reverse-Primer, einem Forward-Primer und Nukleosidphosphaten, bevorzugterweise modifizierten Nukleosidphosphaten und bevorzugtererweise 2'-F-Nukleosidphosphaten,
   wobei der Reverse-Primer im Wesentlichen komplementär zu der Reverse-Primer-Bindungsstelle ist und mit dieser hybridisiert und eine Markierung trägt, wobei die Markierung eine Wechselwirkung zwischen dem Primer und dem Wechselwirkungspartner vermittelt und
   wobei der Forward-Primer im Wesentlichen identisch ist mit der Forward-Primer-Sequenz der Nukleinsäure von Schritt (a),
   um ein doppelsträngiges Amplifikationsprodukt zu erhalten, wobei ein Strang im Wesentlichen der Nukleinsäure von Schritt (a) entspricht und ein Strang dazu komplementär ist, wobei der komplementäre Strang die Markierung trägt,
(g) Entfernen des komplementären Stranges vom Amplifikationsprodukt, um eine Nukleinsäure zu erhalten, die im Wesentlichen der Nukleinsäure von Schritt (a) entspricht,
(h) optional Wiederholen der Schritte (a) bis (g), wobei die Nukleinsäure aus Schritt (g) die heterogene Population ausbilden oder in dieser enthalten sind,
(i) optional Sequenzieren der aus Schritt (d), (f) oder (g) erhaltenen Nukleinsäure(n), wobei im Falle der Sequenzierung bevorzugterweise noch die folgenden Schritte durchgerührt werden:
   (ia) reverse Transkription unter Verwendung des Reverse-Primer, wobei der Reverse-Primer aus DNA besteht und-keine Markierung trägt,
   (ib) Amplifizieren des reversen Transkriptionsproduktes aus Schritt (ia) unter Durchführung einer Zweitstrangsynthese zur Amplifikation, wobei der Reverse-Primer und der Forward-Primer verwendet werden, wobei der Reverse-Primer keineMarkierung aufweist und der Forward-Primer aus DNA besteht.

Es ist vorgesehen, dass der komplementäre Strang in Schritt (g) durch Wechselwirkung zwischen der Markierung und dem Wechselwirkungspartner entfernt wird.

Es ist vorgesehen, dass der Wechselwirkungspartner an einer Oberfläche immobilisiert ist.

Es ist vorgesehen, dass das Amplifikationsprodukt durch die Wechselwirkung zwischen Markierung und Wechselwirkungspartnem an der Oberfläche immobilisiert ist.

Es ist vorgesehen, dass die beiden Stränge des Amplifikationsproduktes voneinander getrennt werden wobei bevorzugterweise der komplementäre Strang immobilisiert bleibt.

Es ist vorgesehen, dass die Markierung ausgewählt aus der Gruppe ist die Biotin, Digoxigenin und Linker mit reaktiven funktionellen Gruppen umfasst, und die reaktiven funktionellen Gruppen bevorzugterweise ausgewählt sind aus der Gruppe, die Amino-, Carboxy, Epoxy und Thiol umfasst.

Es ist vorgesehen, dass der Wechselwirkungspartner ausgewählt ist aus der Gruppe, die Streptavidin, Avidin, Neutravidin und Anti-Digoxigenin-Antikörper und komplementäre funktionelle Gruppen umfasst, und die reaktiven funktionellen Gruppen bevorzugterweise ausgewählt sind aus der Gruppe, die Amino-, Carboxy, Epoxy und Thiol umfasst..

Es ist vorgesehen dass, die Markierung am 5'-Ende des reversen Primers angeordnet ist

Es ist vorgesehen dass, der Forward-Primer modifizierte Nukleosidphosphate, insbesondere 3'-Fluoro-Nukleosidphosphate umfasst.

Es ist vorgesehen dass, der Reverse Primer Desoxynukleosidphosphate umfasst.

Es wird offenbart ein Verfahren zur Selektion einer an ein Zielmolekül bindenden Nukleinsäure, insbesondere von Aptameren, umfassend
(a) Bereitstellen einer heterogenen Population von Nukleinsäuren, insbesondere D-Nukleinsäuren, wobei eine jede Nukleinsäure einen Bereich mit einer randomisierten Sequenz und einer ersten konstanten Sequenz am 5'-Ende und einer zweiten konstanten Sequenz am 3'-Ende aufweist und sich die die Population ausbildenden Nukleinsäuren in der randomisierten Sequenz unterscheiden, wobei die Nukleinsäure modifizierte Nukleosidphosphate umfasst, bevorzugterweise 2'-Fluoro-modifizierte Nukleosidphosphate und die konstanten Sequenzen jeweils 4-6 Nukleotide umfassen,
(b) Inkontaktbringen der Population von Nukleinsäuren mit dem Zielmolekül,
(c) Abtrennen der Nukleinsäuren, die nicht mit dem Zielmolekül in Wechselwirkung getreten sind,
(d) Abtrennen der Nukleinsäure(n), die mit dem Zielmolekül in Wechselwirkung getreten ist/sind, von dem Zielmolekül,
(e) Optional Wiederholen der Schritte von (a) bis (d), wobei die Nukleinsäure(n) aus Schritt (d) die heterogene Population ausbilden oder in dieser enthalten sind,
(f) Modifizieren der Nukleinsäure aus Schritt (a) oder (d) durch folgende Schritte:
   (f0) 5'-Phosphorylieren des 5'-terminalen Nukleotids der Nukleinsäure von
      Schritt (a), bevorzugterweise durch eine Kinasierung, unter der Voraussetzung, dass das 5'-terminale Nukleotid nicht schon eine Phosphatgruppe am 5'-Ende aufweist,
   (fa) Bereitstellen eines ersten Adaptermoleküls, wobei das erste Adaptermolekül aus einer doppelsträngigen Nukleinsäure aus einem ersten und einem zweiten Nukleinsäurestrang besteht und wobei der erste Nukleinsäurestrang und der zweite Nukleinsäurestrang unabhängig voneinander eine Desoxyribonukleinsäure, eine Ribonukleinsäure oder eine FNA ist und das 5'-Ende des zweiten Nukleinsäurestranges einen Überhang liefert, wobei der Überhang zumindest teilweise zu der ersten konstanten Teilsequenz der Nukleinsäure aus Schritt (a) und/oder (d) oder einem Teil davon komplementär ist,
   (fb) Bereitstellen eines zweiten Adaptermoleküls, wobei das zweite Adaptermolekül aus einer doppelsträngigen Nukleinsäure aus einem ersten und einem zweiten Nukleinsäurestrang besteht, wobei der erste Nukleinsäurestrang ein 5'-Phosphat trägt und der erste und der zweite Nukleinsäurestrang unabhängig voneinander eine Desoxyribonukleinsäure, eine Ribonukleinsäre oder eine FNA ist und das 3'-Ende des zweiten Nukleinsäurestranges einen Überhang liefert, der zumindest teilweise komplementär ist zu der zweiten konstanten Teilsequenz der Nukleinsäure aus Schritt (a) und/oder (d) oder einem Teil davon,
   (fc) Ligieren der ersten Nukleinsäurestränge des ersten und des zweiten Adaptermoleküls an die Nukleinsäure aus Schritt (a) und/oder (d), um ein Ligationsprodukt als Reaktionsprodukt zu erhalten,
(g) Reverse Transkription des Ligationsproduktes unter Verwendung des im Ligationsansatz vorliegenden zweiten Stranges des zweiten Adaptermoleküls als Primer, um ein reverses Transkriptionsprodukt zu erhalten,
(h) Durchführen einer Zweitstrangsynthese, wobei der zweite Strang im Wesentlichen komplementär ist zu den reversen Transkriptionsprodukten, wobei die Zweitstrangsynthese bevorzugtererweise eine Amplifikationsreaktion und bevorzugterweise eine Polymerasekettenreaktion ist,
(i) Transkription, des Produktes aus (h), wobei der synthetisierte Zweitstrang als Matrizenstrang dient um Transkriptionsprodukte zu erhalten, wobei ein Transkriptionsprodukt erhalten wird, das komplementär ist zu
   der Sequenz des ersten Nukleinsäurestranges des ersten Adaptermoleküls, der ersten konstanten Teilsequenz,
   dem randomisierten Bereich, und
   der zweiten konstanten Teilsequenz; und
(j) Durchführen einer Nukleinsäuresynthese, wobei das Transkriptionsprodukt aus Schritt (i) mit einem Forward-Synthese-Primer, modifizierten Nukleosidtriphosphaten, bevorzugterweise 2'-Fluoronukleosidtriphosphaten, und einer reversen Transkriptase umgesetzt wird, wobei der Primer mit der Komplementär-Sequenz des ersten Nukleinsäurestranges des ersten Adaptermoleküls hybridisiert und der Primer aus RNA besteht oder einer Kombination von RNA und DNA besteht, unter der Voraussetzung, dass bei der Kombination von RNA und DNA zumindest das 3'-Ende durch ein Ribonukleotid gebildet wird,
(k) Abspalten des Transkriptionsproduktes nach Schritt (j) und der Forward-Primer-Sequenz des in Schritt (j) synthetisierten Nukleinsäuremolekül, um eine Nukleinsäure zu erhalten, die im Wesentlichen identisch ist mit der Nukleinsäure von Schritt (a) oder (d),
(l) optional Wiederholen der Schritte (a) bis (k), wobei die Nukleinsäure aus Schritt (k) die heterogene Population ausbilden oder in dieser enthalten ist, und
(m) optional Sequenzieren der aus Schritt (h) erhaltenen Nukleinsäure.

Es ist vorgesehen, dass die Spaltung in Schritt (k) durch alkalische Spaltung und/oder RNase-Verdau erfolgt.

Es ist offenbart ein Verfahren zur Selektion einer an ein Zielmolekül bindenden Nukleinsäure, insbesondere von Aptameren, umfassend
(a) Bereitstellen einer heterogenen Population von Nukleinsäuren, insbesondere D-Nukleinsäuren, wobei eine jede Nukleinsäure einen Bereich mit einer randomisierten Sequenz und einer ersten konstanten Sequenz am 5'-Ende und einer zweiten konstanten Sequenz am 3'-Ende aufweist und sich die die Population ausbildenden Nukleinsäuren in der randomisierten Sequenz unterscheiden, wobei die Nukleinsäure modifizierte Nukleosidphosphate umfasst, bevorzugterweise 2'-Fluoro-modifizierte Nukleosidphosphate, die konstanten Sequenzen jeweils 4-6 Nukleotide umfassen und die Nukleinsäure am 3'-Ende eine OH-Gruppe trägt.
(b) Inkontaktbringen der Population von Nukleinsäuren mit dem Zielmolekül,
(c) Abtrennen der Nukleinsäuren, die nicht mit dem Zielmolekül in Wechselwirkung getreten sind,
(d) Abtrennen der Nukleinsäure(n), die mit dem Zielmolekül in Wechselwirkung getreten ist/sind, von dem Zielmolekül,
(e) optional Wiederholen der Schritte von (a) bis (d), wobei die Nukleinsäure(n) aus Schritt (d) die heterogene Population ausbilden oder in dieser enthalten sind,
(f) Modifizieren der Nukleinsäure(n) aus Schritt (a) oder (d) durch folgende Schritte:
   (fa) Phosphorylieren des 5'-Endes der Nukleinsäure, sofern die Nukleinsäure kein Phosphat am 5'-Ende aufweist,
   (fb) Bereitstellen eines ersten Adaptermoleküls, wobei das erste Adaptermolekül aus einer doppelsträngigen Nukleinsäure aus einem ersten und einem zweiten Nukleinsäurestrang besteht und wobei der erste Nukleinsäurestrang und der zweite Nukleinsäurestrang unabhängig voneinander eine Desoxyribonukleinsäure, eine Ribonukleinsäure oder eine FNA ist und das 5'-Ende des zweiten Nukleinsäurestranges einen Überhang liefert, wobei der Überhang zumindest teilweise zu der ersten konstanten Teilsequenz der Nukleinsäure aus Schritt (a) und/oder (d) oder einem Teil davon komplementär ist,
   (fc) Bereitstellen eines zweiten Adaptermoleküls, wobei das zweite Adaptermolekül aus einer doppelsträngigen Nukleinsäure aus einem ersten und einem zweiten Nukleinsäurestrang besteht, wobei der erste Nukleinsäurestrang ein 5'-Phosphat trägt, der erste und der zweite Nukleinsäurestrang eine unabhängig voneinander Desoxyribonukleinsäure, eine Ribonukleinsäure oder eine FNA ist und das 3'-Ende des zweiten Nukleinsäurestranges einen Überhang liefert, der zumindest teilweise komplementär ist zu der zweiten konstanten Teilsequenz der Nukleinsäure aus Schritt (a) und/oder (d) oder einem Teil davon und wobei der zweite Nukleinsäurestrang eine Spaltstelle aufweist, die bei Spaltung des Nukleinsäurestranges ein erstes Spaltprodukt und ein zweites Spaltprodukt liefert, wobei das erste Spaltprodukt das 3'-Ende des zweiten Nukleinsäurestranges des zweiten Adaptermoleküls ist, das zumindestens teilweise komplementär ist zu der zweiten konstanten Teilsequenz der Nukleinsäure aus Schritt (a) und/oder (d),
   (fd) Ligieren der ersten Nukleinsäurestränge des ersten und des zweiten Adaptermoleküls an die Nukleinsäure aus Schritt (a) und/oder (d), um ein Ligationsprodukt als Reaktionsprodukt zu erhalten,
(g) Reverse Transkription des Ligationsproduktes unter Verwendung des im Ligationsansatz vorliegenden zweiten Stranges des zweiten Adaptermoleküls als Primer, um ein reverses Transkriptionsprodukt zu erhalten,
(h) Durchführen einer Zweitstrangsynthese, wobei der zweite Strang im Wesentlichen komplementär ist zu den reversen Transkriptionsprodukten, wobei die Zweitstrangsynthese bevorzugterweise eine Amplifikationsreaktion und bevorzugtererweise eine Polymerasekettenreaktion ist, und ein amplifiziertes reverses Transkriptionsprodukt liefert,
(i) Abbau des reversen Transkriptionsproduktes, insbesondere des amplifizierten reversen Transkriptionsproduktes, wobei eine Nukleinsäure bereitgestellt ist, die in 3'→ 5'-Richtung umfasst:
   - die dem Forward Primer komplementäre Sequenz oder die Forward Primer Bindungstelle,
   - den zum randomisierten Bereich komplementären Bereich, sowie
   - den Bereich des zweiten Stranges des zweiten Adaptermoleküls, der teilweise komplementär ist zu der zweiten konstanten Sequenz am 3'-Ende der Nukleinsäure von Schritt (a) und/oder (d),
(k) Durchführen einer Nukleinsäuresynthese, wobei die in (j) bereitgestellte Nukleinsäure mit einem Forward-Synthese-Primer, modifizierten Nukleosidtriphosphaten, bevorzugterweise 2'-Fluoro-Nukleosidtriphosphaten, und einer reversen Transkriptase umgesetzt wird, wobei der Primer mit der Komplementärsequenz des ersten Nukleinsäurestranges des ersten Adaptermoleküls hybridisiert und der Primer aus RNA besteht oder aus einer Kombination aus DNA und RNA, wobei der Primer aus einer Kombination aus DNA und RNA zumindest ein Ribonukleotid an seinem 3'-Ende aufweist, um ein Syntheseprodukt zu erhalten,
(l) Abspalten des reversen Transkriptionsproduktes von dem Syntheseprodukt nach Schritt (k) und der Forward-Synthese-Primer-Sequenz des Syntheseproduktes nach Schritt (k), um eine Nukleinsäure zu erhalten, die im wesentlichen identisch ist mit der Nukleinsäure von Schritt (a) oder (d),
(m)Optional Wiederholen der Schritte (a) bis (l), wobei die Nukleinsäure aus Schritt (a) die heterogene Population ausbildet oder in dieser enthalten ist, und
(n) Optional Sequenzieren der in Schritt (h) erhaltenen Nukleinsäure.

Es ist vorgesehen, dass das Phosphorylieren in Schritt (fa) durch eine Kinasierung erfolgt.

Es ist vorgesehen, dass die Spaltstelle durch eine Restriktionsenzymschnittstelle bereit gestellt wird und das Spalten durch ein Restriktionsenzym erfolgt.

Es ist vorgesehen, dass die Spaltstelle durch ein Ribonukleotid bereit gestellt wird und das Spalten durch alkalische Spaltung oder durch RNasen erfolgt.

Es ist vorgesehen, dass das Abspalten gemäß Schritt (1) enzymatisch, insbesondere durch DNase erfolgt, und/oder die Forward-Synthese-Primer-Sequenz durch eine RNase entfernt wird.

Es ist vorgesehen, dass die Nukleinsäure von Schritt (a) eine einzelsträngige Nukleinsäure ist, die aus modifizierten Nuleosidphosphaten, insbesondere 2'-Fluoro-modifizierten Nukleosidphosphaten besteht.

Es ist offenbart ein Verfahren zur Selektion einer an ein Zielmolekül bindende Nukleinsäure, insbesondere von Aptameren, umfassend die Schritte,
(a) Bereitstellen einer heterogenen Population von Nukleinsäuren, insbesondere D-Nukleinsäuren, wobei eine jede Nukleinsäure einen Bereich mit einer randomisierten Sequenz und einer ersten konstanten Sequenz am 5'-Ende und einer zweiten konstanten Sequenz am 3'-Ende aufweist und sich die die Population ausbildenden Nukleinsäuren in der randomisierten Sequenz unterscheiden, wobei die erste konstante Sequenz eine Forward-Primer-Sequenz und die zweite konstante Sequenz eine Reverse-Primer-Bindungsstelle umfasst,
(b) Inkontaktbringen der Population von Nukleinsäuren mit dem Zielmolekül,
(c) Abtrennen der Nukleinsäuren, die nicht mit dem Zielmolekül in Wechselwirkung getreten sind,
(d) Abtrennen der Nukleinsäure(n), die mit dem Zielmolekül in Wechselwirkung getreten ist/sind, von dem Zielmolekül,
(e) optional Wiederholen der Schritte von (a) bis (d), wobei die Nukleinsäure(n) aus Schritt (d) die heterogene Population ausbilden oder in dieser enthalten sind,
(f) Zweitstrangsynthese eines zur Nukleinsäure von Schritt (a) und/oder (d) komplementären zweiten Stranges und Amplifizieren des zweiten Stranges sowie des zu der Nukleinsäure von Schritt (a) und/oder (d) korrespondierenden Nukleinsäuren durch Zugabe eines Reverse-Primers und eines Forward-Primers, wobei der Reverse-Primer einen ersten und einen zweiten Teilbereich umfasst, wobei der erste Teilbereich an die Reverse-Primer-Bindungsstelle bindet und der zweite Teilbereich am 5'-Ende des Reverse-Primers angeordnet ist und eine Promotorsequenz für eine RNA-Polymerase umfasst, wobei die durch die Zweitstrangsynthese erhaltene Syntheseprodukt der Nukleinsäure von Schritt (a) und/oder (d) entspricht und an ihrem 3'-Ende zusätzlich eine Sequenz aufweist, die komplementär ist zum zweiten Teilbereich des Reverse-Primers,
(g) Transkription des Syntheseproduktes von Schritt (f), wobei die Transkription unter Zugabe von Nukeosidphosphaten und RNA-Polymerase erfolgt und das Transkriptionsprodukt einem DNase-Verdau unterzogen wird, um ein Transkriptionsprodukt zu erhalten, welches in 3'→ 5'-Richtung die Forward-Primer-Bindungsstelle, einen zum randomisierten Bereich der Nulcleinsäure von Schritt (a) komplementären Bereich sowie den ersten Teilbereich des Reverse-Primers umfasst
(h) Synthese einer Nukleinsäure ausgehend von dem verkürzten Transkriptionsprodukt von Schritt (g), wobei das verkürzt Transkriptionsprodukt mit einem Forward-Synthese-Primer, dNTPs und reverser Transkriptase umgesetzt wird, wobei der Forward-Synthese-Primer aus Desoxyribonukleotiden besteht,
(i) alkalischer Verdau des Reaktionsansatzes von Schritt (h) zum Verdau des Transkriptionsproduktes, um eine Nukleinsäure zu erhalten, die im wesentlichen identisch ist zur Nukleinsäure von Schritt (a) und (d).
(j) Optional Wiederholen der Schritte (a) bis (i), wobei die Nukleinsäure(n) aus Schritt (i) die heterogene Population ausbilden oder in dieser enthalten sind und
(k) optional Sequenzieren der aus Schritt (f) oder (d) erhaltenen Nukleinsäure(n).

Es ist vorgesehen, dass die Nukleinsäure von Schritt (a) eine Desoxyribonukleinsäure ist.

Es ist vorgesehen, dass die Promotor-Sequenz ausgewählt ist aus der Gruppe, die Promotor-Sequenzen von T7-RNA-Polymerase, T3-RNA-Polymerase und SP6-Polymerase umfasst.

Im Rahmen der offenbarten Verfahren ist die selektierte Nukleinsäure(n) ausgewählt aus der Gruppe, die Aptamer, Ribozyme, Aptazyme, Antisense-Moleküle und siRNA umfasst.

Die vorliegenden Erfinder haben überraschenderweise festgestellt, dass mittels einer reversen Transkriptase-Aktivität, hierin im folgenden auch als reverse Transkriptase bezeichnet, vollständig modifizierte Oligonukleotide und insbesondere vollständig 2'-F-modifizierte Oligonukleotide hergestellt werden können. Dazu wird lediglich ein Revers-komplementärer Gegenstrang der zu synthetisierenden Sequenz aus RNA oder DNA oder 2'-Amino-modifizierter Nukleinsäure oder FNA, d. h. 2'-Fluoro-modifizierte Nukleinsäure, oder aus einem Gemisch aus diesen und ein geeigneter Primer für die FNA-Synthese benötigt, der an den Gegenstrang hybridisieren kann und hierin auch als Forward-Synthese-Primer oder FS-Primer bezeichnet wird. Bevorzugterweise ist dieser Primer ebenfalls aus FNA. Sollte ein solcher Primer nicht verfügbar sein, kann auch ein Primer aus RNA verwendet werden, der nach der FNA-Synthese durch RNasen oder alkalische Hydrolyse zerstört wird. In diesem Falle muss der Primer upstream, d.h. am 5'-Ende von der zu synthetisierenden Sequenz an den Gegenstrang, der ggf. zusätzliche Nukleotide am 3'-Ende aufweisen muss, hybridisieren. Alternativ zum FS-Primer aus RNA kann auch ein in seiner Sequenz identischer Primer aus DNA benutzt werden, dessen 3'-terminales Nukleotid ein Ribonukleotid ist, das nach der FNA-Synthese durch RNasen oder alkalische Hydrolyse zerstört wird. Optional kann der Primer weitere Ribonukleotidbausteine beinhalten.

Unter Anwendung des erfindungsgemäßen Verfahrens zur Synthese einer Nukleinsäure können grundsätzlich Nukleinsäuren unabhängig von ihrer Sequenz und Funktion hergestellt werden. Funktionale Nukleinsäure, die unter Anwendung des erfindungsgemäßen Verfahrens zur Synthese von Nukleinsäuren hergestellt werden können, umfassen insbesondere auch Aptamere, Ribozyme, Antisense-Moleküle und RNAi-Moleküle. Den unter Anwendung des erFindungsgemäßen Verfahrens hergestellten Nukleinsäuren ist dabei eigen, dass es sich infolge der Modifikation um nukleaseresistente Nukleinsäuren handelt, was für deren Anwendung in biologischen Systemen oder Prozessen besonders vorteilhaft ist. Unter biologischen Systemen werden hierin in besonderen Ausführungsformen unter anderem verstanden: Reaktionsansätze mit biologischem Material, Zell-, Gewebe- und Organaufschlüsse, Zellen, Gewebe, Organe und Organismen, einschließlich aber nicht darauf beschränkt einzellige und mehrzellige Organismen, Menschen, Tieren und Pflanzen und Proben davon.

Das erfindungsgemäße Verfahren ermöglicht auch dessen Implementation in den sog. SELEX-Prozess, wie er beispielsweise in dem europäischen Patent EP 533 83 8 beschrieben ist. Somit ist es unter Anwendung des erfindungsgemäßen Verfahrens zur Synthese von Nukleinsäuren möglich, mit dem SELEX-Prozess ein Zielmolekül bindende Oligonukleotide, nukleaseresistente Aptamere und Ribozyme zu generieren, die vollständig aus 2'-modifizierten Nukleotiden bestehen. Dies verlängert ihre Lebensdauer in Milieus, die RNasen und DNasen enthalten, wie z.B. biologischen Flüssigkeiten, und ermöglicht eine Erweiterung ihrer Einsatzmöglichkeiten, beispielsweise längere Halbwertszeit im Blut und anderen biologischen Flüssigkeiten, im Magen-Darm-Trakt, in Zellen und Geweben.

Nukleaseresistenz ist auch für Antisense-Moleküle, die zum Gen-silencing beitragen, von Vorteil (Manoharan, 1999). Antisense-Moleküle werden häufig einfach chemisch synthetisiert. Es gibt allerdings auch Verfahren, die mit Hilfe von Enzymen potenziell erfolgreiche Antisense-Moleküle identifizieren (Xu et al., 2003; Zhang et al., 2003). Durch Modifikation eines solchen Verfahrens lassen sich nun auch 2'-F-modifizierte Antisense-Moleküle identifizieren. Überdies haben 2'-F-Sequenzen einen höheren Schmelzpunkt, was zu festeren RNA-FNA- oder FNA-DNA-Duplexen führt (Cummins et al., 1995).

Das gleiche gilt auch für siRNAs. Auch hier ist Nukleaseresistenz von Vorteil, damit die siRNAs undegradiert ihr Ziel, d.h. den RISC-Komplex im Cytoplasma ihrer Ziel-Zellen, erreichen (Chiu and Rana, 2003). Durch den Einsatz der hier beschriebenen Verfahren lassen sich so auf enzymatischem Wege bei Wahl geeigneter Template oder Matrizen vollständig 2'-modifizierte siRNAs herstellen.

Die solchermaßen hergestellten vollständig 2'-modifizierten Nukleinsäuren eignen sich auch beispielsweise für die massenspektrometrische Analyse von Nukleinsäuren. Bei der massenspektrometrischen Analyse wurde festgestellt, dass es zu einem Verlust einzelner Basen kommen kann, wobei dieser Verlust durch Verwendung von FNA verringert werden kann. Infolgedessen können auch längere Sequenzen hochauflösend gemessen werden,wenn diese als 2'-modifizierte Nukleinsäuren vorliegen.

Insgesamt erlauben die erfindungsgemäßen Verfahren eine Synthese von Nukleinsäuren mit bzw. aus modifizierten Nukleosidphosphaten, insbesondere 2'-F-modifizierten Nukleosidphosphaten, wobei der Anteil an Abbruchprodukten sehr gering ist bei einem sehr hohen Anteil an Vollängenprodukten. Ein weiterer Vorteil der erfindungsgemäßen Verfahren ist darin zu sehen, dass die Mutationsrate gering ist und sie den Einbau aller und nicht nur einzelner modifizierter Nukleotide erlauben.

Obwohl die verschiedenen Ausführungen hierin insbesondere am Beispiel der Verwendung von 2'-Fluoro-modifizierten Nukleosidphosphate dargestellt wurden, sind die Verfahren gemäß der vorliegenden Erfindung nicht auf diese beschränkt, sondern sind grundsätzlich auf alle hierin beschriebenen modifizierten Nukleosidphosphate anwendbar.

Die im Rahmen der vorliegenden Erfindung verwendeten reversen Transkriptasen sind dabei in bevorzugten Ausführungsformen RNA-abhängige reverse Transkriptasen, die im Rahmen der vorliegenden Erfindung auch in Verbindung mit DNA-Matrizen oder FNA-Matrizen verwendet werden.

Wie hierin in bevorzugten Ausführungsformen verwendet, bezeichnet der Begriff "reverse Transkriptase" eine jegliche enzymatische Aktivität, die in der Lage ist, ausgehend von einem (+) RNA Strang, der als Templat verwendet wird, einen komplementären (-) DNA Strang zu synthetisieren. Sie ist eine RNA-gerichtete DNA-Polymerasse (Stryer, 1995). Für die Durchführung der hierin offenbarten Verfahren geeignete reverse Transkriptasen sind insbesondere auch jene, wie sie in der nachstehenden Tabelle angegeben sind.

| **Name** | **Hersteller** | **Organismus** | **Temp-Optimum** |
|---|---|---|---|
| GeneAmpr rTth | ABI | Thermus thermophilus | 70°C |
| Superscript II | Invitrogen | MMLV, RNase H⁻ | 42°C |
| Superscript III | Invitrogen | MMLV, RNase H⁻ | 50°C |
| ThermoScript | Invitrogen | AMV, RNAse H⁻ | 70°C |
| C. therm | Roche | Carboxydothermus hydrogenoformans | 60-70°C |
| MMLV | Epicentre | MMLV | |
| M-MuLV RT | Fermentas | MMLV | |
| AMVRT | CPG | AMV | |

Dabei ist es im Rahmen der vorliegenden Erfindung, dass auch DNA-Polymerasen, sofern sie die in den Verfahren erforderliche enzymatische Aktivität, d.h. insbesondere die Aktivität einer reversen Transkriptase, aufweisen, im Rahmen der erfindungsgemäßen Verfahren verwendet werden können. Derartige DNA-Polymerasen sind beispielsweise bakterielle DNA-Polymerasen mit reverser Transkriptase-Aktivität wie beispielsweise die in der obigen Tabells angeführten Enzyme von Thermus thermophilus und Carboxyhydrothermus hydrogenoformans. Weitere besonders bevorzugte reverse Transkriptasen sind solche, welche keine RNAseH-Aktivität aufweisen..

Hinsichtlich der Verwendung der verschiedenen hierin offenbarten reversen Transkriptasen ist anzumerken, dass Mischungen der einzelnen reversen Transkriptasen verwendet werden können.Dabei ist es im Rahmen der vorliegenden Erfindung, dass die einzelne reverse Transkriptase in ihrer Wildtyp-Form oder in mutierter Form oder einer Mischung davon entweder alleine oder mit einer oder mehreren anderen reversen Transkriptasen, sei es in der Wildtyp-Form, in einer mutierten Form oder einer Mischung davon, verwendet werden.

Wie hierin verwendet, bezeichnet die Begrifflichkeit "im Wesentlichen" im Zusammenhang mit der Beschreibung einer Nukleinsäure, die im Wesentlichen aus einer bestimmten Spezies von Nukleosidphosphaten besteht, in einer Ausführungsform dass die jeweilige Nukleinsäure vollständig aus den jeweiligen Nukleosidphosphaten bestehen kann, aber auch in bestimmtem Umfang andere Nukleosidphosphate, beispielsweise solche, die eine andere oder keine Modifikation aufweisen, in der Nukleinsäure enthalten sein können. Beispiele dafür, dass eine bestimmte Nukleinsäure aus lediglich im Wesentlichen bestimmten modifizierten Nukleosidphosphaten besteht, können dadurch begründet werden, dass die Ausgangsmaterialien, d. h. die einzelnen eingesetzten Nukleosidphosphate infolge Verunreinigungen nicht in einem jeden Falle die entsprechende Modifikation aufweisen. Darüber hinaus ist es im Rahmen der Kenntnisse des Fachmanns auf dem Gebiet zu bestimmten, wie umfangreich der Gehalt an jenen Nukleosidphosphaten in einer Nukleinsäure vorhanden sein dürfen, die verschieden sind von der Mehrzahl der in der Nukleinsäure enthaltenen modifizierten Nukleosidphosphaten.

Im Zusammenhang mit der vorliegenden Erfindung können die Nukleosidtriphosphate alle modifiziert sein oder nur jeweils eine Spezies oder auch ein bestimmter Anteil einer einzelnen Spezies modifiziert sein oder mehrere Spezies anteilig modifiziert sein. Bevorzugte Nukleosidtriphosphate sind dabei 2'-F-ATP, 2'-F-CTP, 2'-F-GTP, 2'-F-TTP und 2'-F-UTP sowie das Nukleosidtriphosphat der Universalbase Inosin 2-F-ITP. Diese können beliebig gegen 2'-dNTPs ausgetauscht werden.

Weiterhin soll hierin in einer bevorzugten Ausführungsform der Begriff "im Wesentlichen komplementär" bezeichnen, dass die solchermaßen bezeichneten Nukleinsäuren in der Lage sind, miteinander zu hybridisieren, wobei besonders bevorzugt ist, dass dies unter Bedingungen mittlerer Stringenz und insbesondere Bedingungen hoher Stringenz miteinander hybridisieren. Bedingungen für eine mittlere Stringenz bzw. hohe Stringenz sind beispielsweise beschrieben in Current Protocols oder Maniatis et al. Die Stringenz lässt sich dabei durch die Wahl der Inkubationstemperatur und der Kationenkonzentration eistellen.

Wie hierin verwendet bezeichnet der Begriff "Alkyl" eine Methyl-, Ethyl- und eine PropylGruppe. Darüber hinaus umfasst der Begriff Alkyl bzw. "Allyl" auch eine Ethenyl- und eine Propenyl-Gruppe sowie eine Methoxy-, Ethoxy- und Propoxy-Gruppe.

Wie hierin verwendet, bezeichnet der Begriff "Nukleosidphosphate" in Ausführungsformen der vorliegenden Erfindung auch Derivate davon, insbesondere Nukleosidthiophosphate, ist aber nicht darauf beschränkt.

Es ist im Rahmen der hierin offenbarten Verfahren zur Selektion einer an ein Zielmolekül bindenden Nukleinsäure, dass unter anderem Aptamere selektiert werden können. Des weiteren ist es möglich, dass unter Anwendung dieser Verfahren auch Ribozyme, Antisense-Moleküle und RNAi- Moleküle selektiert werden, wobei sich das Zielmolekül entsprechend unterscheidet. Im Falle von Ribozymen besteht die Eigenschaft des Ribozyms darin, an ein Zielmolekül zu binden und dieses oder sich selbst darauf hin zu modifizieren. Die Modifizierung kann das Knüpfen oder Spalten einer oder mehrerer chemischer Bindungen oder beides sein. Im Falle der Anwendung der erfindungsgemäßen Selektionsverfahren für die Selektion von Antisense-Molekülen ist das Zielmolekül eine Nukleinsäure, insbesondere eine mRNA oder Vorläuferstufen hierzu.

In einem Aspekt betrifft die vorliegende Erfindung auch ein Verfahren zur enzymatischen Synthese von einzelsträngiger DNA und insbesondere deren Anwendung in einem Verfahren zur Selektion einzelsträngiger DNA oder DNA-Oligonukleotide.

Einzelsträngige DNA-Oligonukleotide können - in Analogie zu einzelsträngigen RNAs - Zielstrukturen erkennen und an sie binden (Bock et al., 1992; Green et al., 1996; Leva et al., 2002). Man bezeichnet sie daher auch als DNA-Aptamere. DNA-Aptamere haben im Gegensatz zu solchen Aptameren, welche Ribonukleotide enthalten oder aus diesen aufgebaut sind, den Vorteil, dass sie laugenstabil und autoklavierbar sind. Dies ist von großem Vorteil, wenn das Aptamer beispielsweise in Chromatographiesäulen als Affinitätsmatrix eingesetzt werden soll, die durch ein *"Cleaning in* Place"-Verfahren regeneriert werden soll. Hier kommen in allgemeinen verdünnte Laugen zum Einsatz. Für Anwendungen im medizinischen Bereich, wie z.B. extrakorporale Adsorber zur Blutwäsche, ist Sterilität eine unverzichtbare Bedingung. Daher ist hier eine Sterilisation, evtl. auch zur Wiederverwendung nötig. Die gängigste Methode ist dabei die Autoklavierung. Diese wird erfahrungsgemäß von DNA-Aptameren intakt überstanden, während sich RNA-Aptamere hydrolysieren.

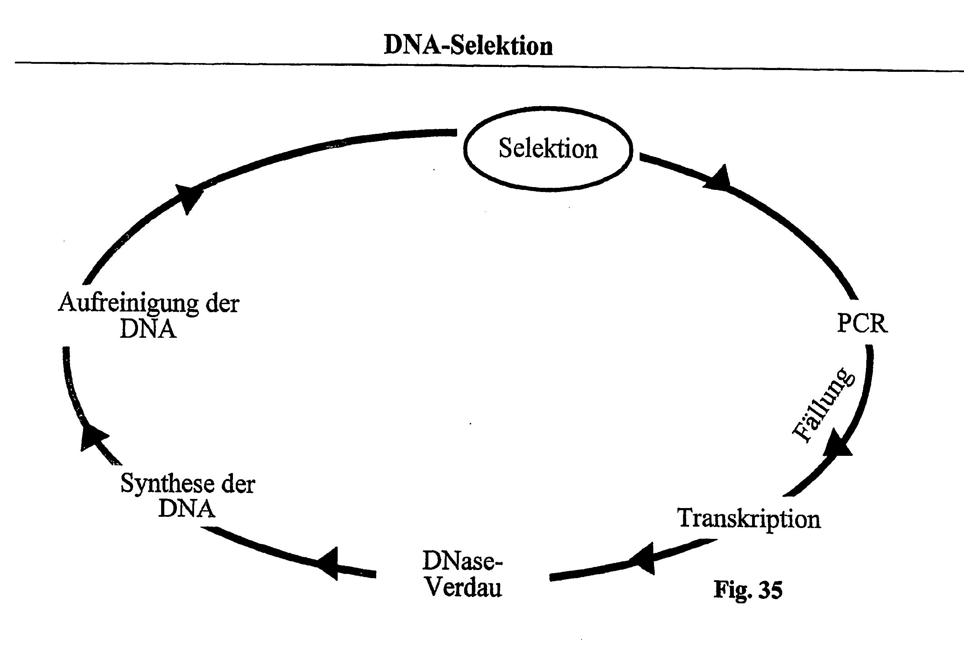

Ein Vorteil der Verwendung eines DNA-Oligonukleotids in einem Amplifikationsschritt in Kontext des SELEX-Verfahrens, wie in Fig. 35-38 detaillierter dargestellt, besteht beispielsweise darin, dass im Vergleich zum Stand der Technik (Williams et al., 1997) nicht die PCR als letzter Amplifikationsschritt vorgesehen ist. Daher ist keine präparative Gelreinigung der DNA, also Gelreinigung und Eluieren aus dem Gel und Fällung, wie im Stand der Technik beschrieben, nötig. Üblicherweise werden die beiden DNA-Stränge, die in der PCR geschrieben werden, voneinander getrennt, indem ein Strang vorher durch Spaltung eines Ribo-Primers verkürzt wird. Die nach dem Stand der Technik erforderlichen und nicht gut automatisierbaren präparative Gelreinigungen werden bei der Durchführung des erfindungsgemäßen Verfahrens zur Amplifikation und Selektion von DNA-Oligonukleotiden, insbesondere einzelsträngigen DNA-Oligonukleotiden nicht erforderlich, was deren besondere Eignung zur Verwendung in automatisierten Verfahren zur Durchführung von Selektionsverfahren wie dem SELEX-Verfahren begründet.

Alternativ zur Trennung der in der PCR erhaltenen DNA-Stränge durch Gelreinigung ist in Stand der Technik die Verwendung eines biotinylierten Primers beschrieben. Das PCR-Produkt wird dann auf Streptavidin-Partikeln immobilisiert und der Strang, der den biotinylierten Primer nicht enthält, wird mittels Natronlauge, Erhitzen o.ä. eluiert. Die Kapazität der Partikeln ist oft recht beschränkt, da auch biotinylierte aber nicht-inkorporierte Primer die vorhandenen Biotinbindungsstellen besetzen. Außerdem leiden die Partikeln (engl. beads) häufig unter den alkalischen Bedingungen bzw. Hitze, so dass Streptavidin oder andere Partikeln-Bestandteile zusammen mit dem DNA-Strang eluiert werden. Diese müssen dann erst wieder abgetrennt werden, z.B. durch eine nicht gut automatisierbare Phenol/Chloroform-Extraktion.

Ein weiterer Vorteil dieses erfindungsgemäßen Verfahrens besteht darin, dass die DNA für die jeweils folgende Selektionsrunde nicht nur durch präparative PCR, sondern durch PCR und in vitro Transkription hergestellt wird. Daher werden weniger PCR-Zyklen benötigt. Da die PCR einen starken Selektionsdruck ausübt und bei hoher Zyklenzahl häufig PCR-Artefakte (Murphy et al., 2003), sog. Amplifikationsparasiten, auftreten ist dies von großem Nutzen.

Die vorliegende Erfindung soll im folgenden anhand der folgenden Figuren und Beispiele veranschaulicht werden, wobei sich daraus weitere Merkmale, Ausführungsformen und Vorteile ergeben. Insbesondere wird darauf hingewiesen, dass einzelne Merkmale, wie sie bei den konkreten nachfolgend beschriebenen Figuren bzw. Beispielen im Kontext weiterer Merkmale offenbart sind, als solche einzeln auch in anderen Ausführungsformen der vorliegenden Erfindung verwendet oder angewandt werden können.
- Figs. 1 bis 3: zeigen schematisch Abläufe verschiedener Ausführungsformen des erfindungsgemäßen Verfahrens zur Synthese von Nukleinsäuren;
- Figs. 4 bis 38: zeigen schematische Abläufe verschiedener Ausführungsformen des erfindungsgemäßen Verfahrens zur Selektion einer an ein Zielmolekül bindenden Nukleinsäure unter Anwendung des erfindungsgemäßen Verfahrens zur Synthese von Nukleinsäuren;
- Fig. 39: zeigt schematisch die Durchführung eines Amplifikationszyklusses unter Anwendung des erfindungsgemäßen Verfahrens zur Synthese von Nukleinsäuren und das Ergebnis einer Analyse des Verfahrens mittels PAGE;
- Fig. 40: zeigt das Ergebnis einer PAGE-Analyse zur Stabilität von enzymatisch hergestellten kombinatorischen FNA- und DNA-Bibliotheken,
- Fig. 41: zeigt das Ergebnis einer PAGE-Analyse zur Stabilität von enzymatisch hergestellten kombinatorischen FNA- und 2'-F-Pyrimidin-RNA -Bibliotheken gegenüber RNase T1 und RNase I,
- Fig. 42: zeigt das Ergebnis einer PAGE-Analyse zur Stabilität von enzymatisch hergestellten kombinatorischen FNA- und RNA -Bibliotheken in menschlichem Serum, und
- Fig. 43: zeigt den Selektionsverlauf der in Beispiel 1 beschriebenen *in vitro* Selektion.

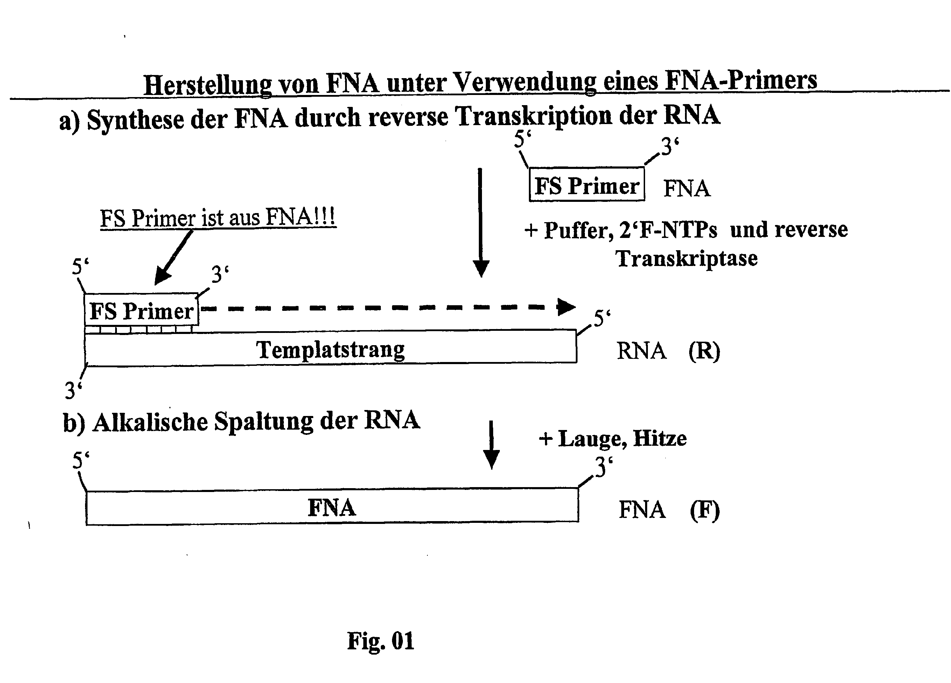

Fig. 1 zeigt den grundsätzlichen Ablauf einer Synthese von Nukleinsäuren, insbesondere FNA, unter Anwendung einer reversen Transkriptase. Genauer gesagt wird ausgehend vom einem Templat- oder Matrizen-Strang aus RNA ein Primer an das 3'-Ende des aus RNA bestehenden Matrizenstranges annealt oder hybridisiert, so dass ein freies 3'-OH-Ende zur Verfügung steht, das unter dem Einfluss der reversen Transkriptase und der im konkreten Beispiel eingesetzten 2'-F-NTPs und Zugabe eines geeigneten Puffers entsprechend bis zum 5'-Ende des Matrizenstranges verlängert wird. Bei dem Primer handelt es sich im vorliegenden Fall um eine FNA. Das solchermaßen erhaltene doppelsträngige Nukleinsäuremolekül wird anschließend weiter behandelt, um den Matrizenstrang zu entfernen. Diese Behandlung kann dabei eine Spaltung unter alkalischen Bedingungen und Hitze (300 mM Natronlauge, 10 min. bei 95°C) sein, was zu einem Verdau der RNA-Bestandteile der doppelsträngigen Nukleinsäure führt und lediglich den synthetisierten Nukleinsäurestrang, der im vorliegenden Falle vollständig aus FNTP besteht, zurücklässt.

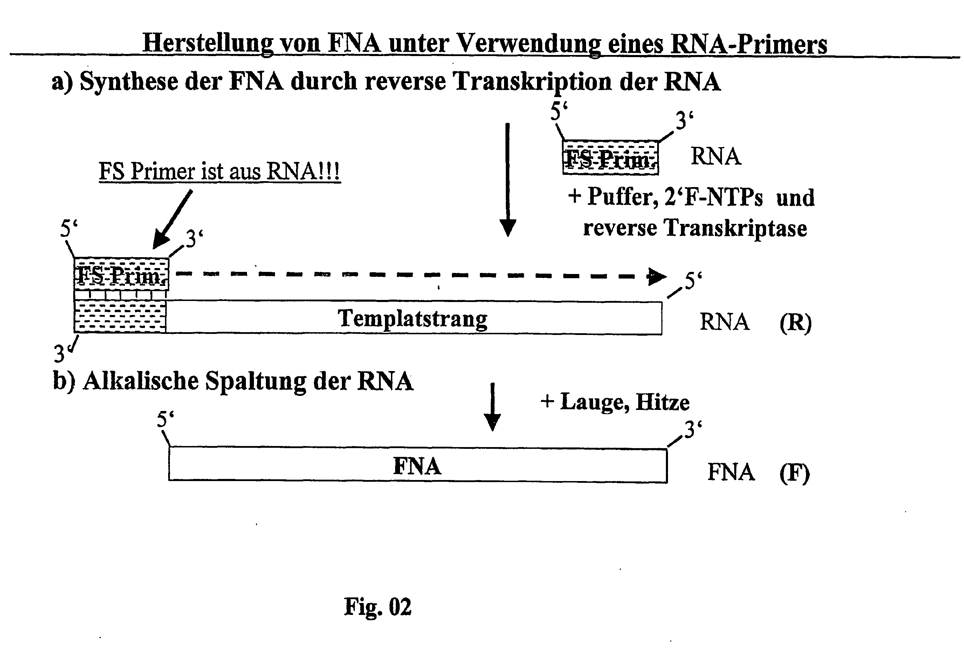

Fig. 2 zeigt ein gegenüber Fig. 1 alternatives Verfahren zur Synthese einer Nukleinsäure, speziell einer FNA. Dabei wird in dieser Ausführungs form ein Primer aus RNA verwendet. Die Matrizensequenz aus RNA muss in diesem Falle an ihrem 3'-Ende um weitere Nukleotide verlängert sein, um den RNA-Primer das Anhybridisieren zu gestatten. Bevorzugterweise umfasst diese Verlängerung einen Bereich von 10-25 Nukleotiden. Nach erfolgter reverser Transkription mittels einer reversen Transkriptase wird der Matrizenstrang und der am synthetisierten Nukleinsäurestrang vorhandene RNA-Primer beispielsweise durch alkalische Hydrolyse oder einen RNAase-Verdau entfernt.

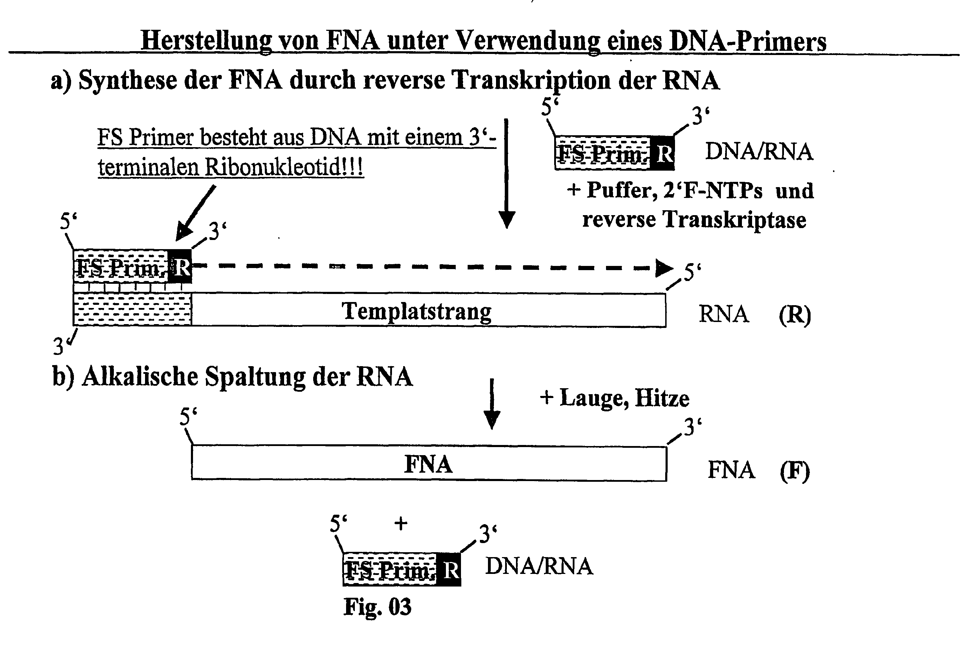

Fig. 3 zeigt eine weitere Ausführungsform des erfindungsgemäßen Verfahrens zur Synthese einer Nukleinsäure, wie dies in Fig. 1 dargestellt ist. Dabei wird ähnlich dem in Fig. 2 beschriebenen Verfahren ein Primer eingesetzt, der verschieden ist von einem FNA-Primer. Speziell wird ein DNA-Primer mit mindestens einem Ribonukleotid eingesetzt. Der DNA-Primer kann darüberhinaus weitere Ribonukleotide enthalten, wobei jedoch essentiell ist, dass am 3'-Ende des DNA-Primers ein Ribonukleotid vorhanden ist. Auch in diesem Falle umfasst der Matrizenstrang aus RNA eine weitere Sequenz an seinem 3'-Ende, die das Anhybridisieren des Primers erlaubt. Der RNA-Matrizenstrang wird durch die im Zusammenhang mit Figs. 1 und 2 beschriebenen Verfahren und Maßnahmen entfernt. Der Primer wird infolge alkalischer Hydrolyse an dem 3'-Ende angeordneten Ribonukleotid von dem neu synthetisierten FNA-Strang abgetrennt und kann nachfolgend entfernt werden, beispielsweise durch Gelreinigung, Molekularsieb oder Gelfiltration.

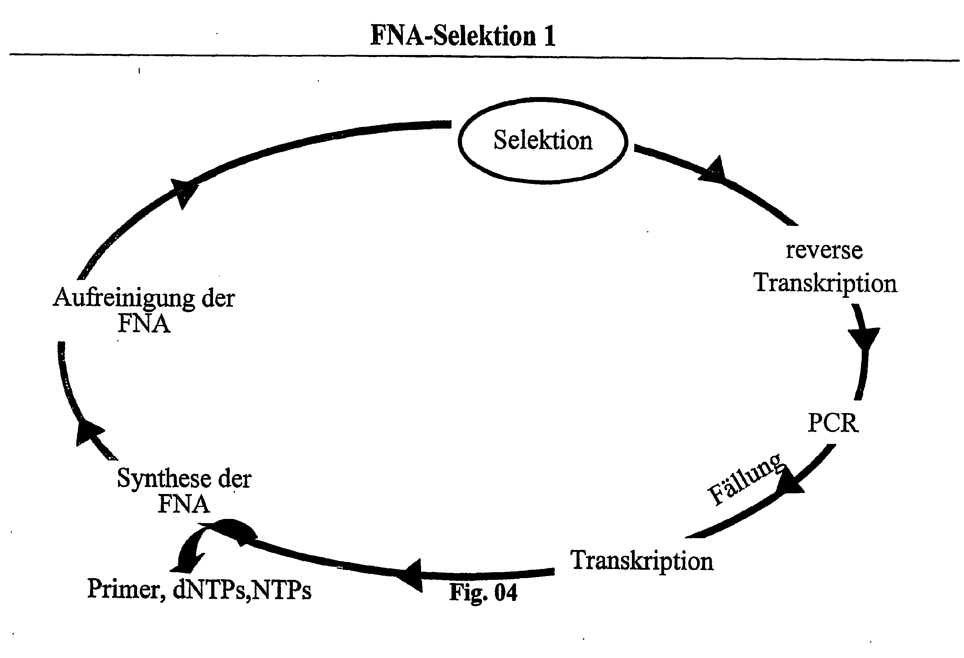

Fig. 4 zeigt ein Schema zur Durchführung eines *in vitro* Selektionsprozesses, bei dem das erfindungsgemäße Verfahren zur Synthese einer Nukleinsäure integriert ist. Nach der Selektion geeigneter FNA-Spezies werden diese durch eine Reverse Transkription mit dNTPs in komplementäre DNA (cDNA) umgeschrieben. Zur Amplifikation der Sequenzen erfolgt dann eine PCR und eine *in vitro* Transkription. Vor der sich daran anschließenden FNA-Synthese werden die aus der PCR und *in vitro* Transkription kommenden, die FNA-Synthese störenden dNTPs, NTPs Primer entfernt. Nach der FNA-Synthese wird die FNA aufgereinigt beispielsweise durch denaturierende PAGE oder alkalische Hydrolyse des Templat-Stranges. Bei der alkalischen Hydrolyse des Templat-Stranges kann zugleich der Forward-Synthese-Primer von der FNA-abgespalten werden, falls dieser nicht auch aus FNA besteht.

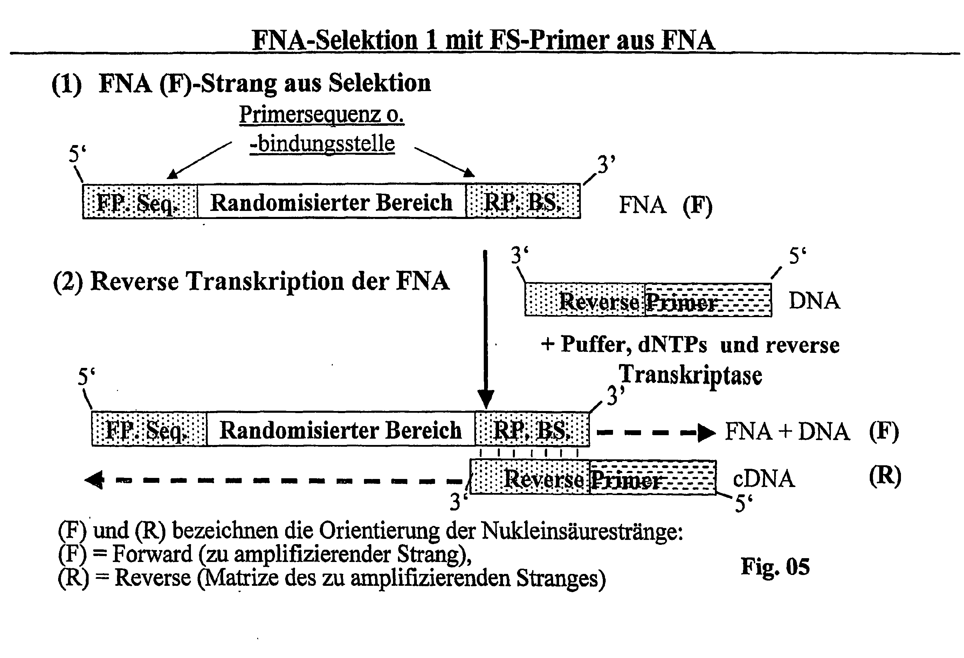

Fig. 5 bis 8 zeigen den Verlauf eines Amplifikationsverfahrens mit einem Transkriptionsschritt, wie es beispielsweise in einem *in vitro* Selektionsprozess, wie er in Fig. 4 dargestrellt ist, verwendet werden kann.

Insbesondere zeigt Fig. 5 die Herstellung von cDNA mittels einer Reversen Transkriptase wie beispielsweise Superscript II (Invitrogen) ausgehend von selektierten 2'-F-Oligonukleotiden. Diese 2'-F-Oligonukleotide können die heterogene Population von Nukleinsäuren darstellen, wie sie in einem *in* vitro-Selektionsprozess verwendet werden. Die hierin auch als Ausgangnukleinsäure bezeichneten Oligonukleotide weisen eine erste konstante Sequenz am 5'-Ende auf und eine zweite konstante Sequenz am 3'-Ende, die den randomisierten Bereich oder die randomisierte Sequenz flankieren. Die erste konstante Sequenz der Nukleinsäure umfasst eine Forward-Primer-Sequenz und die zweite konstante Sequenz umfasst eine Reverse-Primer-Bindungsstelle. Dabei ist bevorzugt, dass die erste konstante Sequenz die Forward-Primer-Sequenz ist und die zweite konstante Sequenz die Reverse-Primer-Bindungsstelle. Der aus DNA aufgebaute Reverse-Primer besteht aus zwei Teilbereichen. Der erste Teilbereich enthält oder umfasst die Sequenz, die mit der Reverse-Primer-Bindungsstelle bindet, der zweite Teilbereich enthält oder umfasst einen Promotor für eine RNA-Polymerase. In einem Reaktionsansatz umfassend den Reverse-Primer, Puffer sowie dNTPs und eine reverse Transkriptase wird so ein cDNA-Strang gebildet und der Ausgangsstrang, d. h. der Fluoro-Strang, mit dNTPs verlängert.

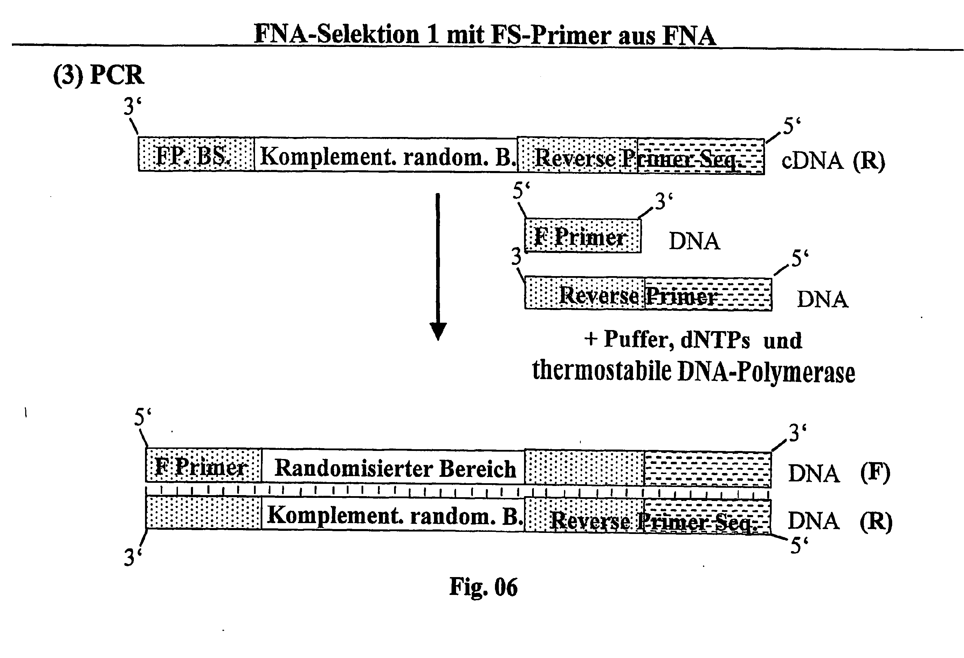

Fig. 6 zeigt den Ablauf eines Zweitstrangsyntheseschrittes, der hier als Polymerasekettenreaktion ausgeführt ist. Dabei kommt es zu einer Amplifikation der cDNA und insbesondere auch einer Amplifikation des reversen Transkriptionsproduktes, das in Fig. 5 auch als "R"-Strang bezeichnet wird. Dabei wird neben dem Reverse-Primer noch ein Forward-Primer und eine DNA-Polymerase, insbesondere eine thermostabile DNA-Polymerase, eingesetzt. Der F-Primer bindet dabei an die F-Primer-Bindesequenz des reversen Transkriptionsproduktes und erlaubt davon ausgehend die Synthese des Zweitstranges.

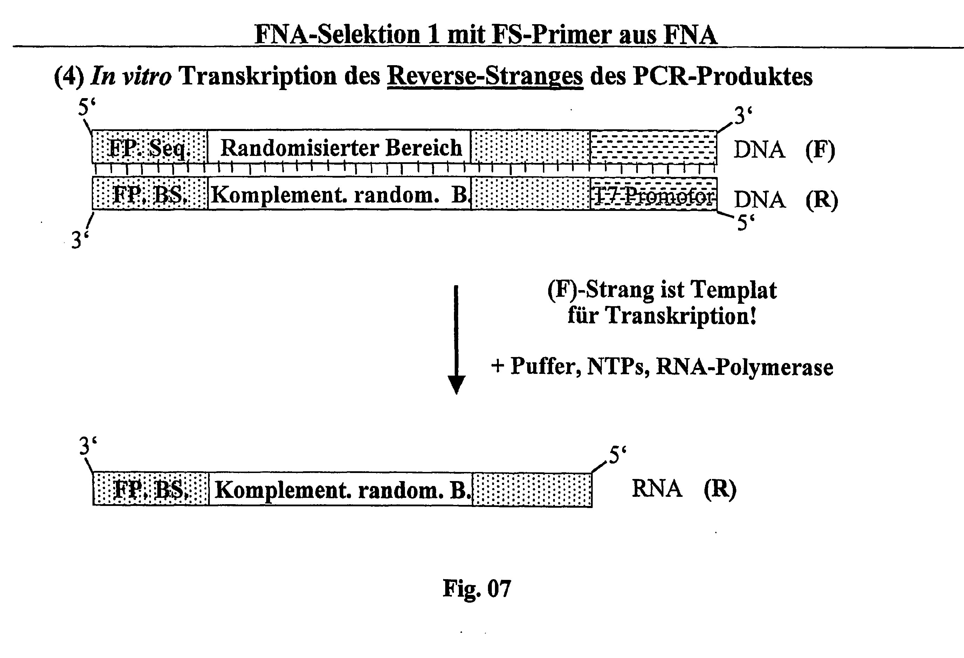

Fig. 7 zeigt die *in vitro*-Transkription des Reverse-Stranges des PCR-Produktes, dessen Herstellung in Fig. 6 beschrieben ist, in RNA. Als Vorlage für diese Transkription dient der mit "F" bezeichnete Forward-Strang des PCR-Produktes. Dabei wird der RNA-Polymerasen-Promotorbereich, im vorliegenden Bereich ein T7-Promotor, nicht transkribiert. Als Ergebnis wird eine Ribonukleinsäure erhalten, die komplementär ist zu der eingesetzten Nukleinsäure, wie sie in Teilschritt (1) von Fig. 5 dargestellt ist, d.h. der Ausgangsnukleinsäure.

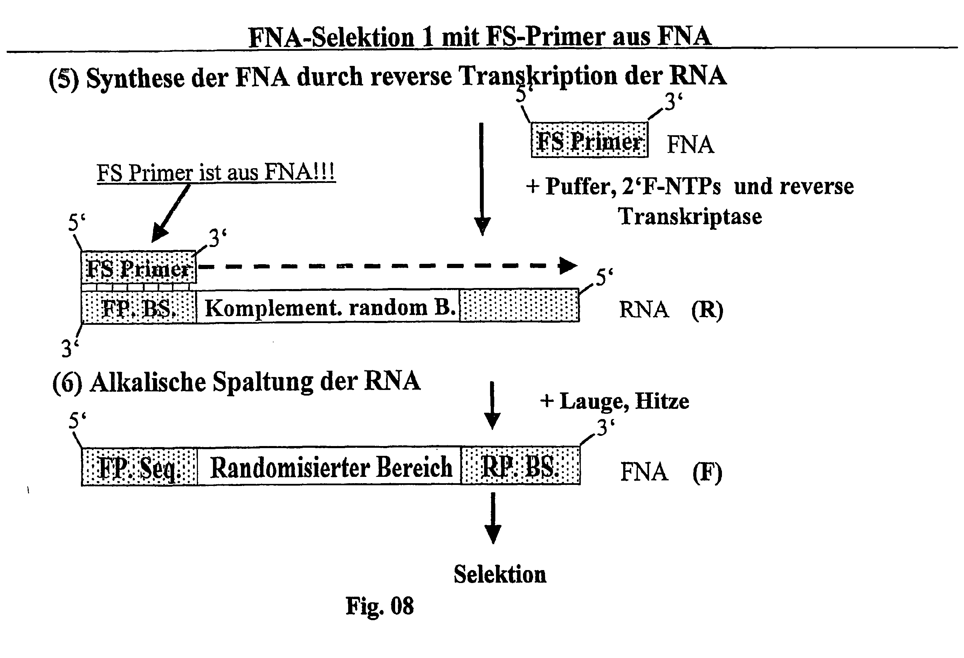

Fig. 8 schließlich zeigt die FNA-Synthese mit einem FNA-Primer, der hierin als FS-Primer bezeichnet wird. Der Reaktionsansatz enthält neben dem FS-Primer noch 2'-F-NTPs sowie eine reverse Transkriptase, wie beispielsweise Superskript II von Invitrogen. Der Forward-Synthese-Primer hybridisiert an die Forward-Primer-Bindungsstelle des aus RNA aufgebauten und als Matrizenstrang dienenden Transkriptionsproduktes (Fig. 7) und erlaubt die Synthese eines FNA-Nukleinsäurestranges, der im Wesentlichen komplementär ist zur Sequenz des zwischenzeitlich amplifizierten reverse Transkriptase-Produktes. Der Matrizenstrang wird im vorliegenden Falle mittels einer alkalischen Hydrolyse oder eines RNAse-Verdaus entfernt. Optional schließen sich FNA-Reinigung und Markierung sowie gegebenenfalls eine weitere Selektionsrunde an.

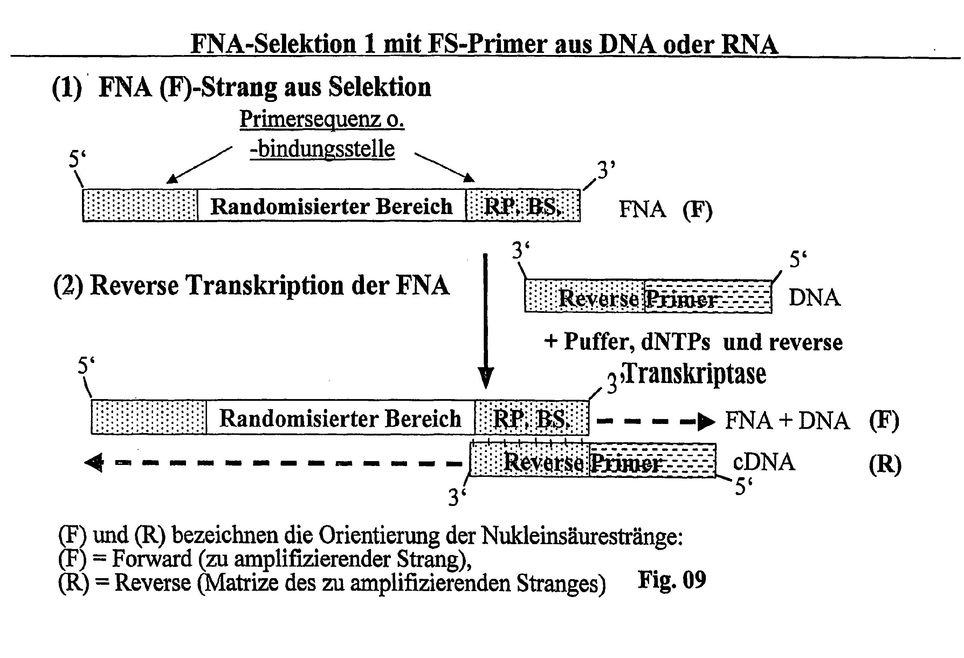

Die Figs. 9 bis 12 zeigen ein Verfahren für eine mögliche Amplifikation mit einem Transkriptionsschritt, wobei hier anstelle eines aus FNA bestehenden Primers mit einem RNA oder RNA-DNA-Primer gearbeitet wird. Dabei ist der Reaktionsablauf im Wesentlichen gleich zu dem in den Figs. 5 bis 8 dargestellten Reaktionsablauf, wobei sich jedoch einige im folgenden dargestellte Unterschiede ergeben.

Fig. 9 zeigt die reverse Transkription der selektierten FNA-Moleküle, d.h. der Ausgangsnukleinsäure, die unter den gleichen Bedingungen wie die in Fig. 5 dargestellte Reaktion abläuft.

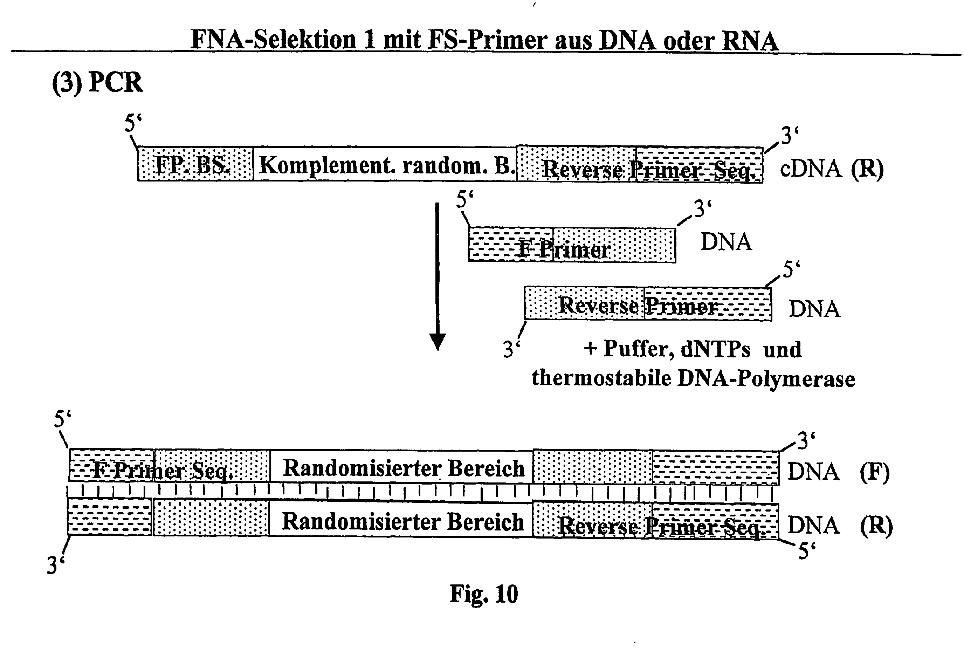

Fig. 10 zeigt die PCR-Amplifikation der cDNA mit dem Reverse-Primer und einem Forward-Primer, der zum einen, am 3'-Ende, eine Sequenz unzfasst, die im Wesentlichen komplementär ist zur Forward-Primer-Bindungsstelle des reversen Transkriptes, und darüber hinaus am 5'-Ende einen weiteren Teilbereich umfasst, wobei dieser weitere Teilbereich der Sequenz des Forward-Synthese-Primers, wie er im Rahmen der für die FNA-Synthese verwendeten reversen Transkription verwendet wird, entspricht.

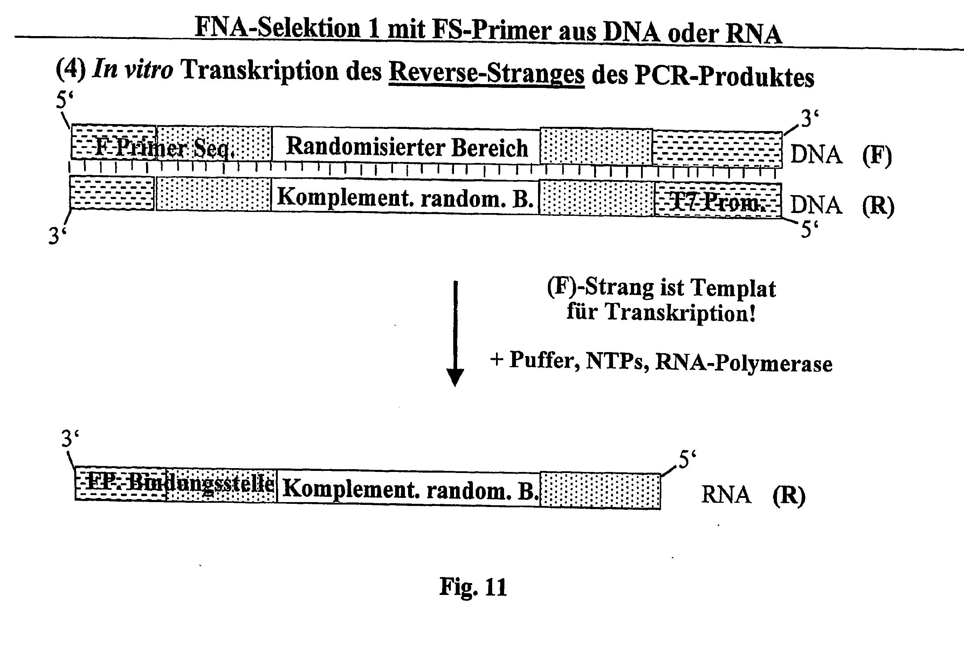

Fig. 11 zeigt die *in vitro*-Transkription des PCR-Produkt-Gegenstranges. Als Matrize dient der (F)-Strang. Eine geeignete RNA-Polymerase, wie beispielsweise T7-RNA-Polymerase und NTPs werden dem Reaktionsansatz zugesetzt und ein Transkriptionsprodukt erhalten, welches in 3' → 5'-Richtung eine Forward-Primer-Bindungsstelle, den zum randomisierten Bereich komplementären Bereich sowie eine Reverse-Primer-Sequenz umfasst. Daraus ergibt sich, dass dieser Strang gegenüber dem komplementären Strang der in Fig. 9 eingesetzten Nukleinsäure (Schritt (1), (F)) an seinem 3'-Ende um jenen Bereich verlängert ist, der zu dem weiteren Bereiche des Forward-Primers komplementär ist, und dagegen jener Bereich des Reverse-Primers, der am 5'-Ende über den zur Reverse-Primer-Bindungsstelle komplementären Bereich hinausgeht, in dem Molekül nicht enthalten ist.

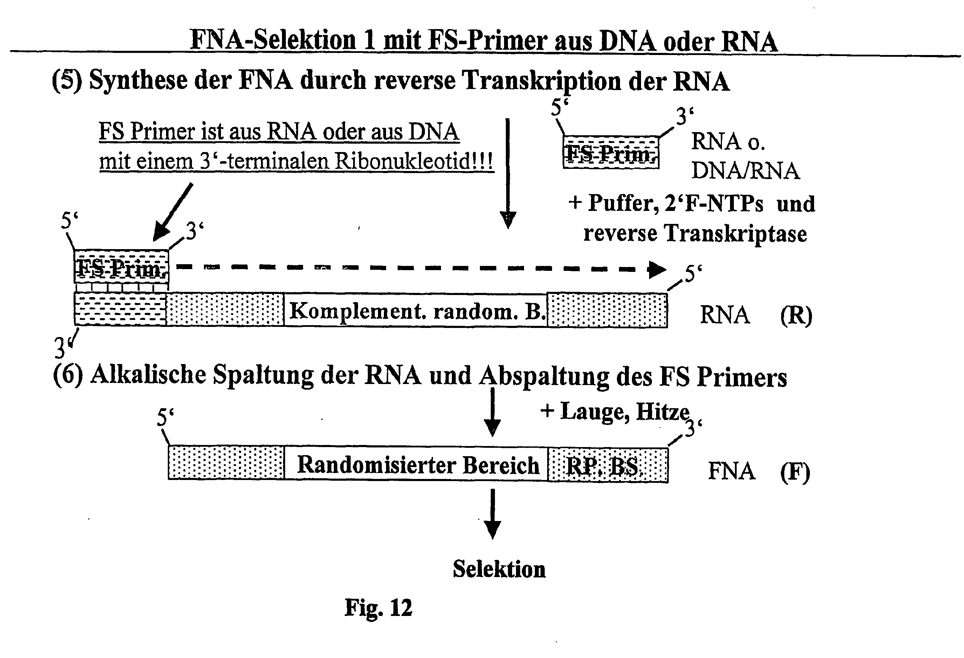

Fig. 12 zeigt die FNA-Synthese mit einer reversen Transkriptase wie beispielsweise Superskript II (Invitrogen) und 2'-F-NTPs. Als Primer dient entweder ein Ribonukleotid oder ein Desoxyribonukleotide mit mindestens einem 3'-terminalen Ribonukleotid, dessen Sequenz identisch ist mit der Sequenz des 5'-überhängenden Bereich des Forward-Primers, d. h. des weiteren Teilbereiches des Forward-Primers. Durch alkalische Hydrolyse wird der Matrizenstrang und der Primer von der FNA abgetrennt. Diese einzelsträngige FNA-Nukleinsäure kann sodann in weiteren Selektionsrunden verwendet werden.

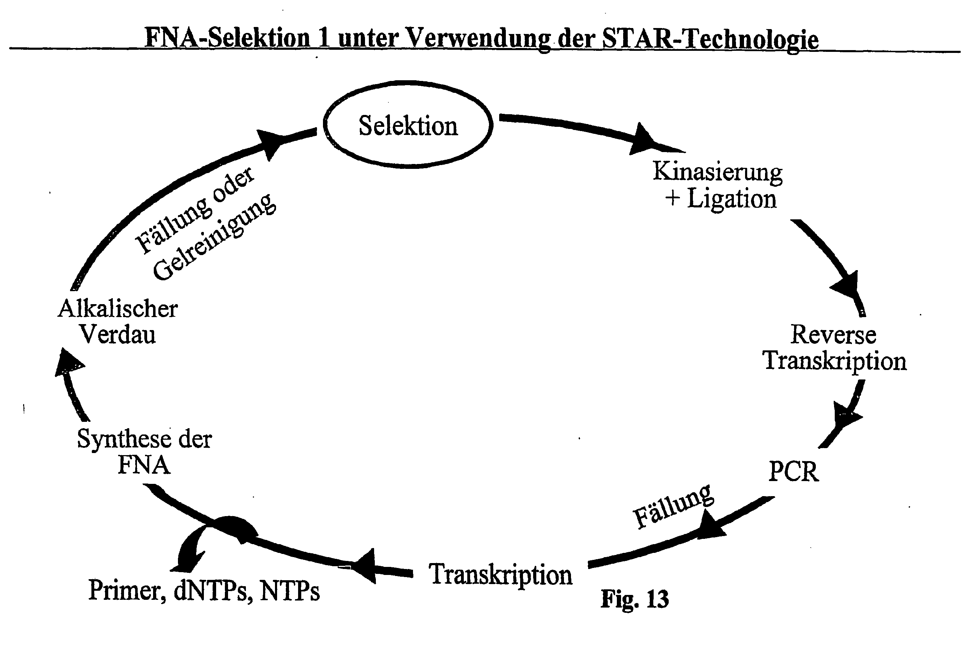

Figs. 13-18 zeigen die Vorgehensweise zur Amplifikation von FNAs in einem Selektionsschema, bei dem Ausgangnukleinsäure neben dem randomisierten Bereich nur vergleichsweise kurze, diesen flankierende Bereich aufweist, was bei der Selektion von Vorteil ist und insbesondere die Verkürzung der in einer Selektion primär erhaltenen an das Zielmolekül bindenden Nukleinsäuren ermöglicht. Diese spezielle Ausführungsform des Selektionsverfahrens wird hierin auch als Primer-Ligation bezeichnet (STAR-FNA-Selektion 1).

Fig. 13 zeigt einen Überblick über den Kreisprozess bestehend aus Selektion, Primerligation, Amplifikation, FNA-Synthese und -Reinigung.

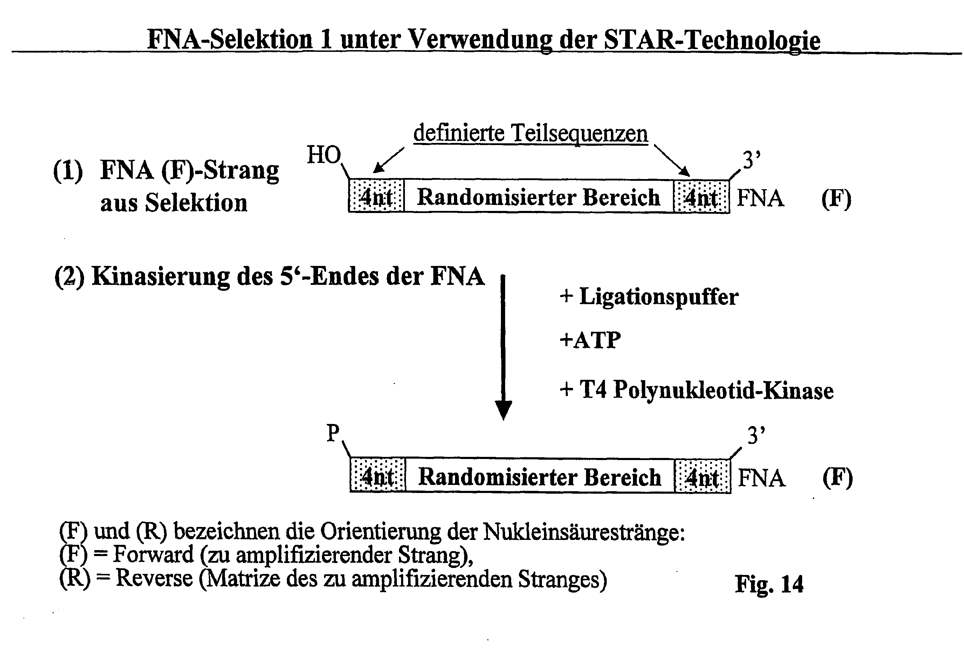

Fig. 14 zeigt in (1) die Nukleinsäure, wie sie in einem Selektionsprozess typischer Weise als Ausgangnukleinsäure verwendet wird. Die Nukleinsäure besteht im vorliegenden Falle aus FNA und weist neben dem randomisierten Bereich am 5'-Ende einen Teilbereich auf, der eine erste konstante Sequenz umfasst und am 3'-Ende einen weiteren Teilbereich, der eine zweite konstante Sequenz aufweist. Die Längen dieser Sequenzen sind in den hierin beschriebenen Verfahren grundsätzlich nicht beschränkt, jedoch ist eine Länge von 4, 5 oder 6 Nukleotiden bevorzugt, wobei bevorzugtererweise die Längen der beiden konstanten Sequenzen gleich sind; grundsätzlich können diese jedoch auch unterschiedlich sein. Die die heterogene Population ausbildenden Nukleinsäuren unterscheiden sich in dem randomisierten Bereich. Die Nukleinsäure kann dabei an ihrem 5'-Ende eine OH-Gruppe aufweisen oder ein Phosphat. Das Phosphat ist für die nachfolgenden Schritte von zentraler Bedeutung und wird typischerweise nach dem Selektionsschritt. durch Phosphorylierung am 5'-Ende der Nukleinsäure eingeführt. Die 5'-Phosporylierung erfolgt in einem geeigneten Ligationspuffer unter Zusatz von ATP und beispielsweise einer T4-Polynukleotid-Kinase, wobei auch andere Enzyme für eine derartige Kinasierung geeignet sind. Um möglichste hohe Ausbeuten in den nachfolgenden Schritten zu erhalten wird eine möglichst vollständige Kinasierung angestrebt.

Die am 5'-Ende mit einer Phosphatgruppe versehene Nukleinsäure wird sodann einer Modifizierung unterzogen. Die Modifizierung verwendet dabei zwei Adaptermoleküle. Das erste Adaptermolekül besteht aus einer doppelsträngigen Nukleinsäure aus einem ersten und einem zweiten Nukleinsäurestrang, wobei. die ersten Stränge des ersten und des zweiten Adaptermoleküls aus DNA, RNA oder FNA sind., bevorzugterweise aus DNA der zweite Nukleinsäurestrang eine Desoxyribonukleinsäure ist und das 5'-Ende des zweiten Nukleinsäurestranges einen Überhang liefert, wobei der Überhang zumindest teilweise zu der ersten konstanten Teilsequenz der Nukleinsäure aus Schritt (a) und/oder (d), d.h. der Ausgangsnukleinsäure, komplementär ist. Das zweite Adaptermolekül besteht ebenfalls aus einer doppelsträngigen Nukleinsäure und umfasst ebenfalls einen ersten und einen zweiten Nukleinsäurestrang, wobei der erste Nukleinsäurestrang ein 5'-Phosphat trägt und der zweite Nukleinsäurestrang eine Desoxyribonukleinsäure ist und das 3'-Ende des zweiten Nukleinsäurestranges einen Überhang liefert, der zumindest teilweise komplementär ist zu der zweiten konstanten Teilsequenz der in Fig. 14(1) bzw. Fig. 14(2) dargestellten Ausgangnukleinsäure ist. Bevorzugterweise sind sowohl der erste als auch der zweite Nukleinsäurestrang sowohl des ersten wie auch des zweiten Adaptermoleküls aus Desoxyribonukleinsäure hergestellt.

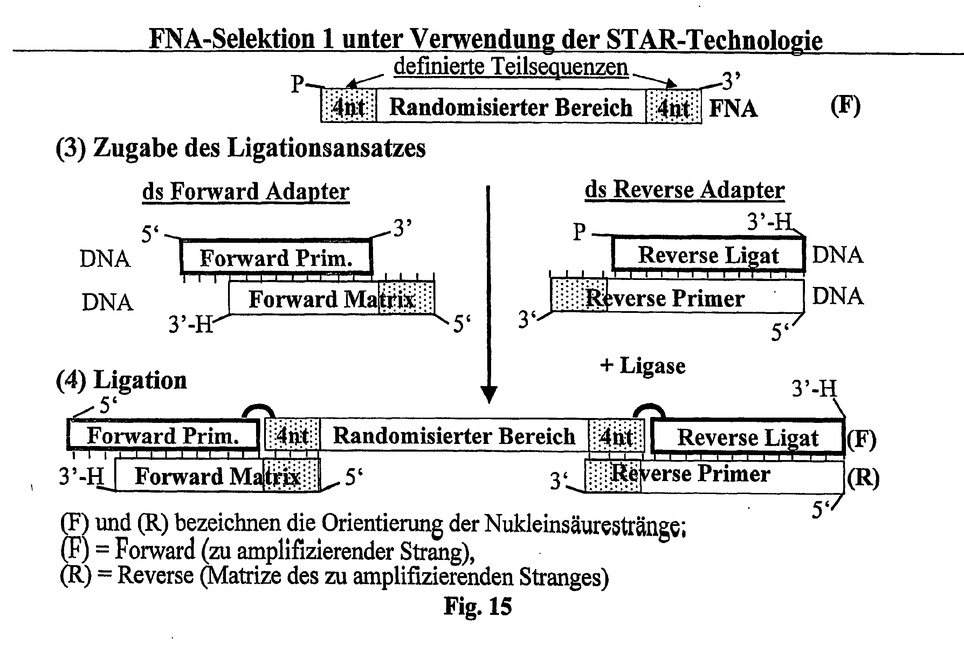

Fig. 15 zeigt die Ligation des ersten und zweiten Adaptermoleküls an die Nukleinsäure, die in dem Selektionsprozess als Ausgangsnukleinsäure verwendet wird. Dabei wird der erste Nukleinsäurestrang des ersten und des zweiten Adaptermoleküls an die Ausgangsnukleinsäure ligiert unter Anwendung einer Ligase. Eine geeignete Ligase ist beispielsweise die T4-DANN-Ligase (MBI Fermentas). Das solchermaßen erhaltene Ligationsprodukt besteht aus der Ausgangsnukleinsäure, an dessen 5'-Ende der erste Nukleinsäure des ersten Adaptermoleküls hierin auch als Forward-Primer bezeichnet, kovalent gebunden ist und an dessen 3'-Ende der erste Nukleinsäurestrang des zweiten Adaptermoleküls, hierin auch als Reverse Ligat bezeichnet, gebunden ist. Die jeweiligen zweiten Nukleinsäurestränge des ersten und zweiten Adaptermoleküls sind entsprechend ihrer Komplementarität an den jeweilige ersten Nukleinsäurestrang des ersten und zweiten Adaptermoleküls und mit ihren entsprechenden Überhängen an den korrespondierenden ersten und zweiten Teilsequenzen der Ausgangsnukleinsäure hybridisiert. Der Reverse-Primer enthält einen Promotor für eine RNA-Polymerase der 5' von der überhängenden Sequenz, die im vorliegenden Falle vier Nukleotide umfasst, liegt.

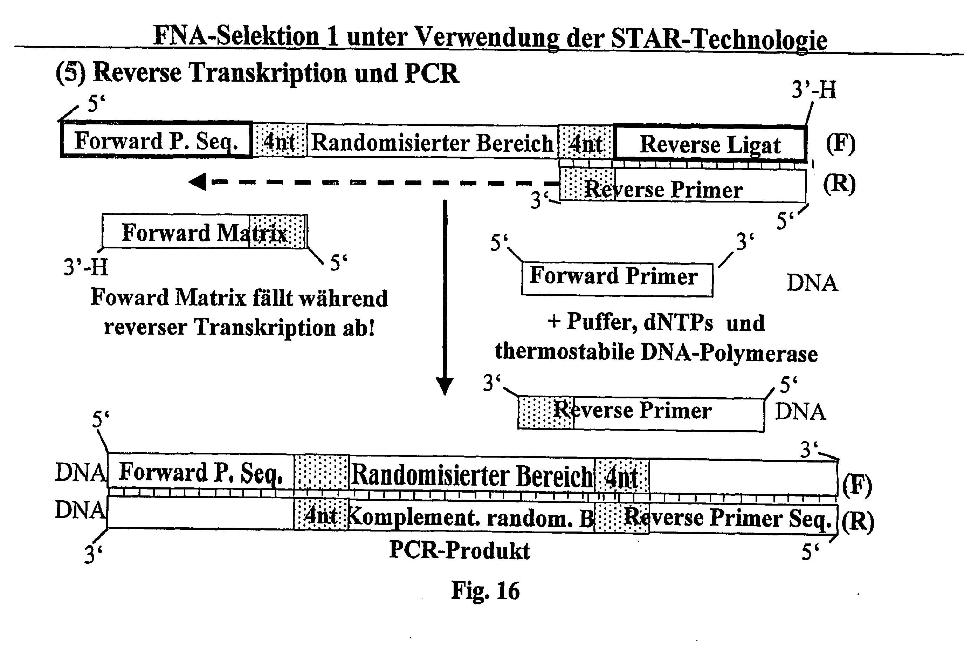

Fig. 16 zeigt die reverse Transkription des (F)-Stranges, d.h. des durch die jeweils ersten Nukleinsäurestränge der ersten und zweiten Adapters verlängerten Ausgangsnukleinsäure. Als geeignete reverse Transkriptasen sind alle hierin beschriebenen reversen Transkriptasen geeignet, so z.B. Superscript II (Invitrogen). Die Synthese zu dem durch die beiden jeweils ersten Nukleinsäuresträngen verlängerten Ausgangsnukleinsäure erfolgt ausgehend vom 3'-Ende des Reverse-Primers. Der zweite Nukleinsäurestrang des ersten Adaptermoleküls fällt während der reversen Transkription ab. Anschließend wird eine Amplifikationsreaktion wie beispielsweise eine Polymerase-Kettenreaktion mit geeigneten DNA-Polymerasen unter Zugabe geeigneter Puffer sowie dNTPs durchgeführt. Besonders geeignet sind hierbei thermostabile DNA-Polymerasen, wie z.B. die Taq-DNA-Polymerase (Roche). Diese Amplifikationsreaktion führt zur Amplifikation sowohl der verlängerten Ausgangsnukleinsäure als auch des infolge der reversen Transkription hierzu komplementären Nukleinsäurestranges. Beide Stränge liegen typischerweise in hybridisierter Form vor.

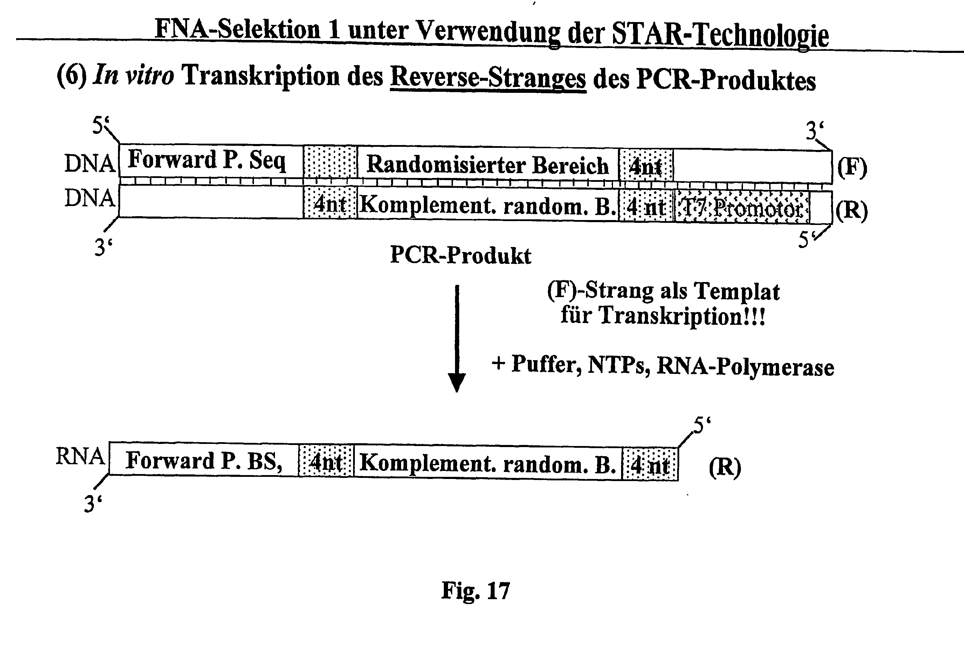

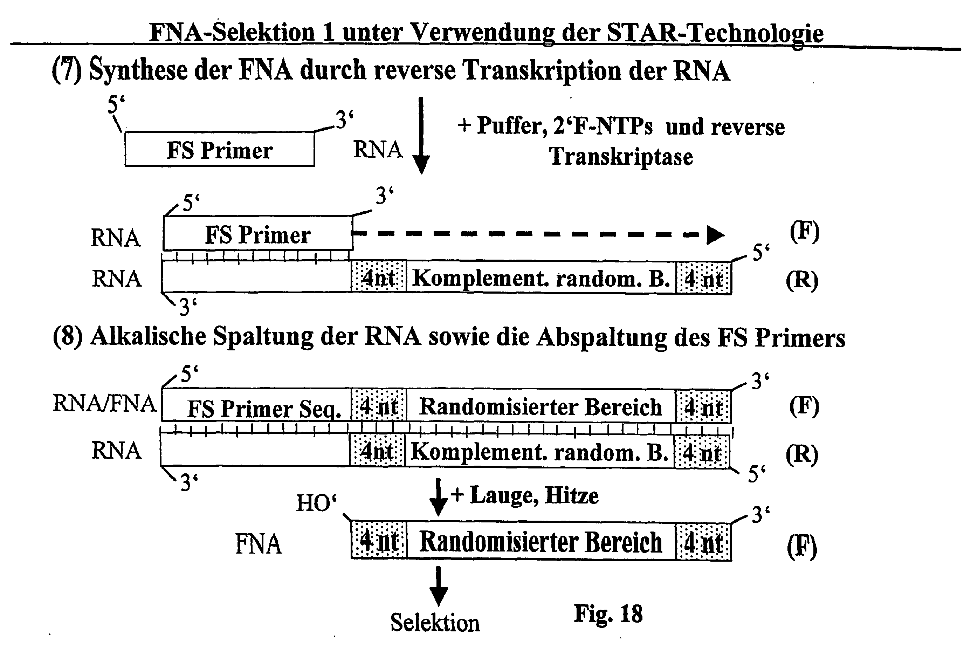

Der reverse Strang des doppelsträngigen Amplifikationsproduktes wird, wie in Fig. 17 dargestellt, einer in vitro-Transkription unterzogen. Die verlängerte Ausgangsnukleinsäure dient dabei als Matrize für die Transkription, die unter Zugabe geeigneter Puffer, NTPs sowie einer RNA-Polymerase durchgeführt wird. Am Ende wird ein Transkriptionsprodukt erhalten, welches in 3' → 5'-Richtung eine Sequenz aufweist, die komplementär ist zum ersten Strang des ersten Adaptermoleküls, eine Sequenz, die komplementär ist zu der ersten konstanten Teilsequenz der Ausgangsnukleinsäure, eine Sequenz, die komplementär ist zum randomisierten Bereich der Ausgangsnukleinsäure, und einen Bereich der komplementär ist zu der zweiten konstanten Teilsequenz der Ausgangs-Nukleinsäure. Dieses Produkt wird sodann einer Nukleinsäuresynthese unterzogen (Fig. 18) unter Zugabe eines aus RNA bestehenden Forward-Synthese-Primers, hierin auch als FS-Primer bezeichnet, der die Sequenz des ersten Nukleinsäurestranges des ersten Adaptermoleküls aufweist, von modifizierten Nukleosidphosphaten, im vorliegenden Falle 2'-F-NTPs, sowie reverser Transkriptase, wobei als reverse Transkriptase alle hierin offenbarten reversen Transkriptasen grundsätzlich geeignet sind. Am Ende der Reaktion liegt eine doppelsträngige Nukleinsäure vor, wobei ein Strang dem Transkriptionsprodukt entspricht und der andere hierzu komplementär ist. Das Transkriptionsprodukt besteht aus Ribonukleinsäure, wohingegen das durch die reverse Transkriptase synthetisierte Syntheseprodukt im Bereich des FS-Primers aus RNA besteht und der nachfolgende Bereich aus modifizierten Nukleosidphosphaten, insbesondere 2'-F-Nukleosidphosphaten. Alternativ zum FS-Primer aus RNA kann auch ein in seiner Sequenz identischer Primer aus DNA benutzt werden, dessen 3'-terminales Nukleotid ein Ribonukleotid ist. Durch Verdau der RNA, beispielsweise durch alkalische Spaltung oder RNase-Aktivität werden die RNA-Bestandteile dieser doppelsträngigen Nukleinsäure verdaut und am Ende eine einzelsträngige Nukleinsäure erhalten, die der Ausgangsnukleinsäure entspricht bzw. durch mögliche Einbaufehler der verwendeten Enzyme im wesentlichen entspricht. Die solchermaßen erhaltene Nukleinsäure kann in den Selektionsprozess wieder eingegliedert bzw. dort als Ausgangsnukleinsäure verwendet werden.

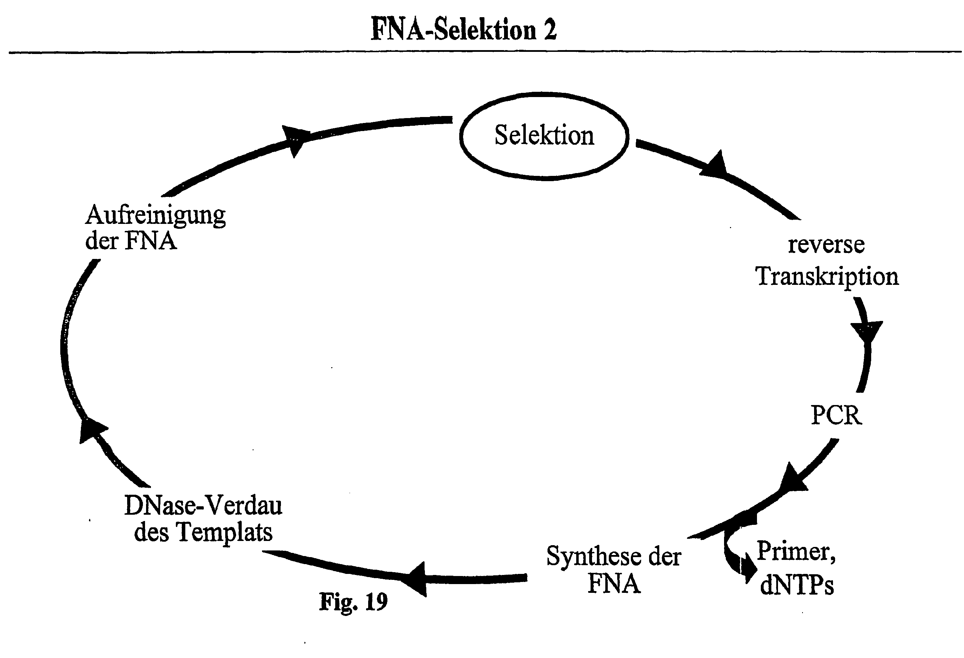

Fig. 19-25 zeigt eine alternative Vorgehensweise zur Amplifikation von FNAs unter Anwendung des erfindungsgemäßen Verfahrens zur Synthese von Nukleinsäuren, insbesondere auch, wie ein derartiges Verfahren in einem *in vitro* Selektionsprozess eingebaut wird. Gegenüber der in den Figuren 5 bis 12 dargestellten Ausführungsform wird in dieser Ausführungsform auf die *in vitro-Transkription* verzichtet.

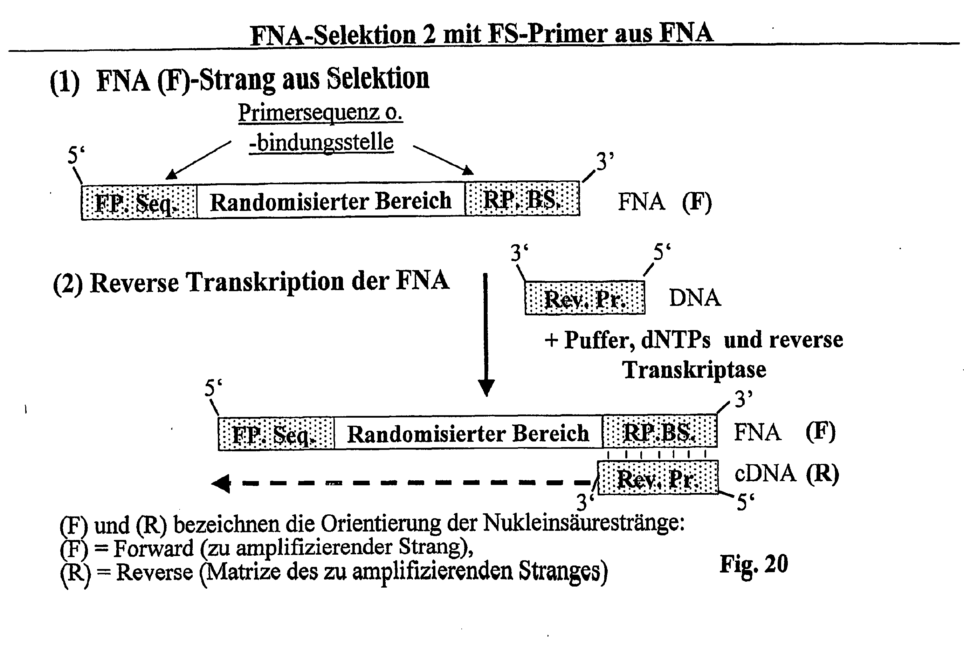

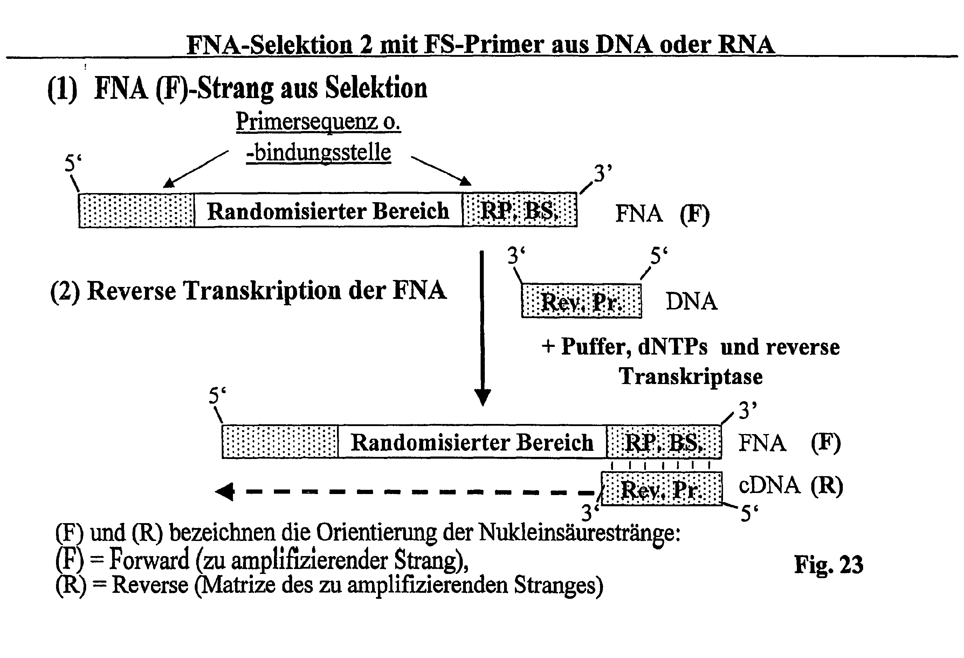

Fig. 19 zeigt einen Überblick über den Kreisprozess, der die folgenden Schritte umfasst: Selektion, reverse Transkription, PCR-Amplifikation, FNA-Synthese und FNA-Isolierung. Dabei zeigen die Figs. 20 bis 22 das Verfahren unter Verwendung eines Forward-Synthese-Primers aus FNA und die Figs. 23 bis 25 das Verfahren unter Verwendung eines Forward-Synthese-Primers aus DNA oder RNA.

Grundsätzlich ist anzumerken, dass das Verfahren in ähnlicher Weise durchgeführt wird, wie das in den Figs. 5 bis 12 beschriebene Verfahren, wobei sich aus dem Verzicht auf den Transkriptionsschritt einige im folgenden genauer dargestellte Änderungen ergeben.

Fig. 20 zeigt die Herstellung von cDNA mittels einer Reversen Transkriptase ausgehend von selektierten 2'-F-Oligonukleotiden, d.h. der Ausgangsnukleinsäure, wobei hier ein Revers-Primer verwendet ist, der mit der Revers-Primer-Bindungsstelle der zu amplifizierenden Nukleinsäure hybridisiert. Im Unterschied zu dem in den Figs. 5 bis 8 dargestellten Verfahren fehlt hier dem Primer jener Bereich, der als RNA-Polymerase-Promotor fungiert. Als Reaktionsprodukt der Reversen Transkriptase wird ein cDNA-Strang erhalten, der komplementär zu dem eingesetztem FNA-Strang ist.

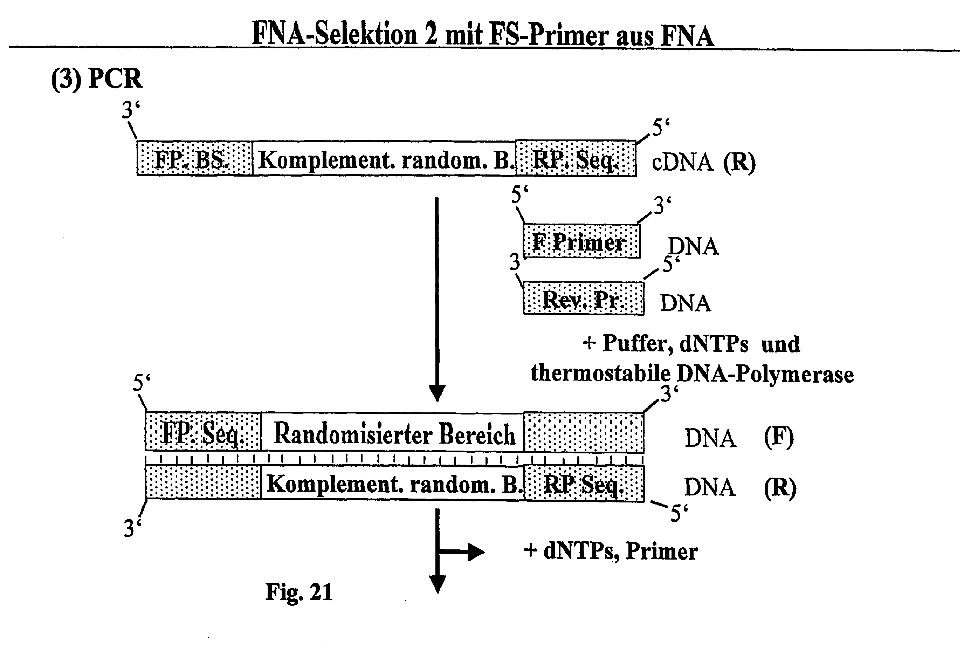

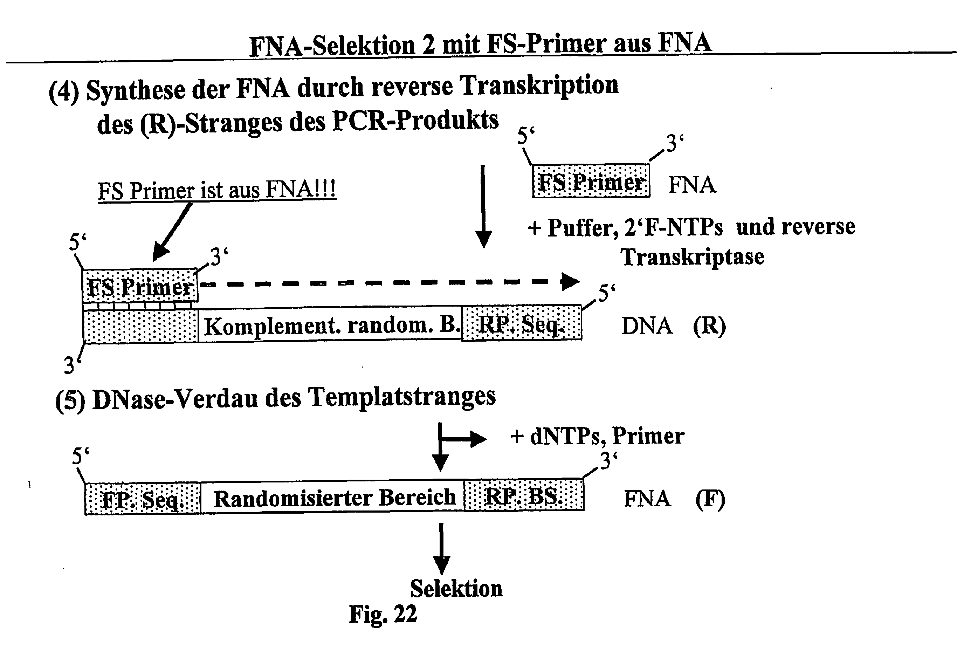

In der in Fig. 21 dargestellten Polymerasekettenreaktion die neben der Zweitstrangsynthese auch der Amplifikation dient, wird der Reverse-Primer sowie ein Forward-Primer eingesetzt, der grundsätzlich ähnlich jenem ausgebildet ist, wie er im Rahmen der Polymerasekettenreaktion des Verfahrens gemäß Fig. 6 verwendet wird. Die cDNA, d. h. das Reaktionsprodukt der reversten Transkriptase wird dadurch amplifiziert. Die in der PCR-Reaktion eingesetzten überschüssigen Primer sowie Nukleotide werden anschließend entfernt, beispielsweise durch einen Molekularsieb. Die FNA-Nukleinsäure wird, wie in Fig. 22 dargestellt, anschließend durch reverse Transkription des aus DNA bestehenden (R)-Stranges des PCR-Produktes vorgenommen, wobei dieser R-Strang im wesentlichen komplementär ist zu der in Schritt 1 von Fig. 20 dargestellten zu amplifizierenden Nukleinsäure, d.h. der Ausgangnukleinsäure. Als Matrize dient der R-Strang der PCR. Die Synthese der FNA erfolgt mittels einer reversen Transkriptase, eines FNA-Primers und 2'-F-NTPs. Der Matrizen-Strang wird durch DNase verdaut und die Bruckstücke bzw. Nukleotide entfernt. Das solchermaßen erhaltene Reaktionsprodukt entspricht der zu amplifizierenden Nukleinsäure und kann entsprechend in eine nächste Selektionsrunde eingeführt werden.

Fig. 23 zeig die Herstellung von cDNA mittels einer reversen Transkriptase ausgehend von selektierten 2'-F-Oligonukleotiden, d.h. den Ausgangnukleinsäuren. Als Reaktionsprodukt wird ein cDNA-Strang erhalten. Im Unterschied zu der in Fig. 9 dargestellten Vorgehensweise, bei der ein Transkriptionsschritt noch vorgesehen ist, ist bei dieser Ausführungsform der Erfindung vorgesehen, dass der Reverse-Primer lediglich aus dem Teilbereich besteht, welcher mit dem der Reverse-Primer-Bindungsstelle hybridisiert (Fig. 14). Ein Bereich der einen Promotor für eine RNA-Polymerase fehlt typischerweise.

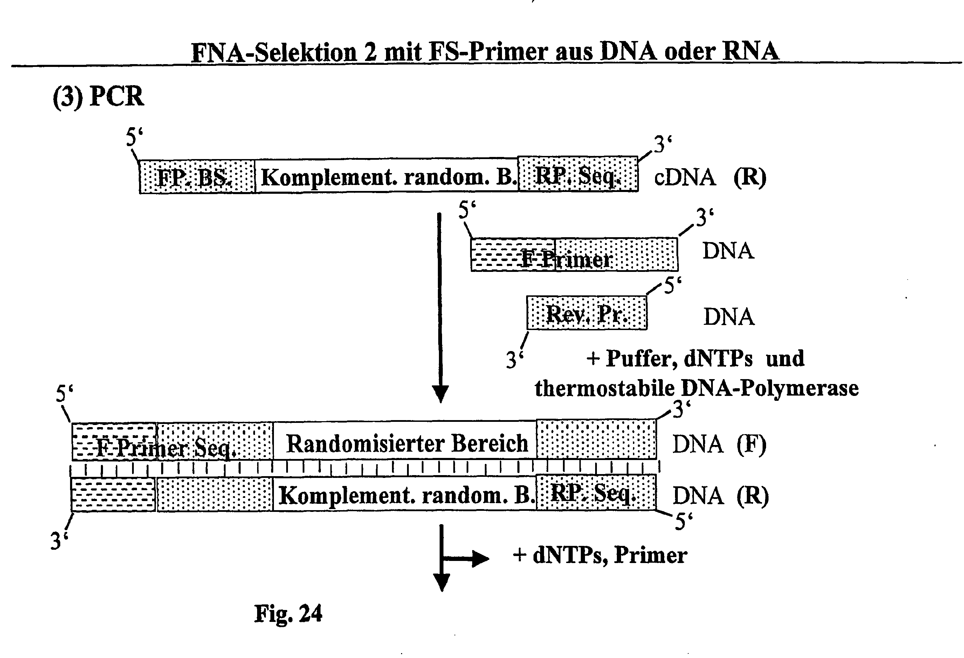

Bei der sich anschließenden Polymerasekettenreaktion (Fig. 24), die der Amplifikation der cDNA dient, werden zwei Primer eingesetzt, nämlich ein Forward-Primer und der Reverse-Primer. Der Forward-Primer weist an seinem 5'-Ende einen weiteren Teilbereich auf, der dem später, in dem Syntheseschritt mittels Reverser Transkriptase verwendeten Forward-Synthese-Primer entspricht. Die im Überschuss zugegebenen dNTPs sowie Primer werden im Anschluss an die PCR beispielsweise durch Molekularsieb entfernt.

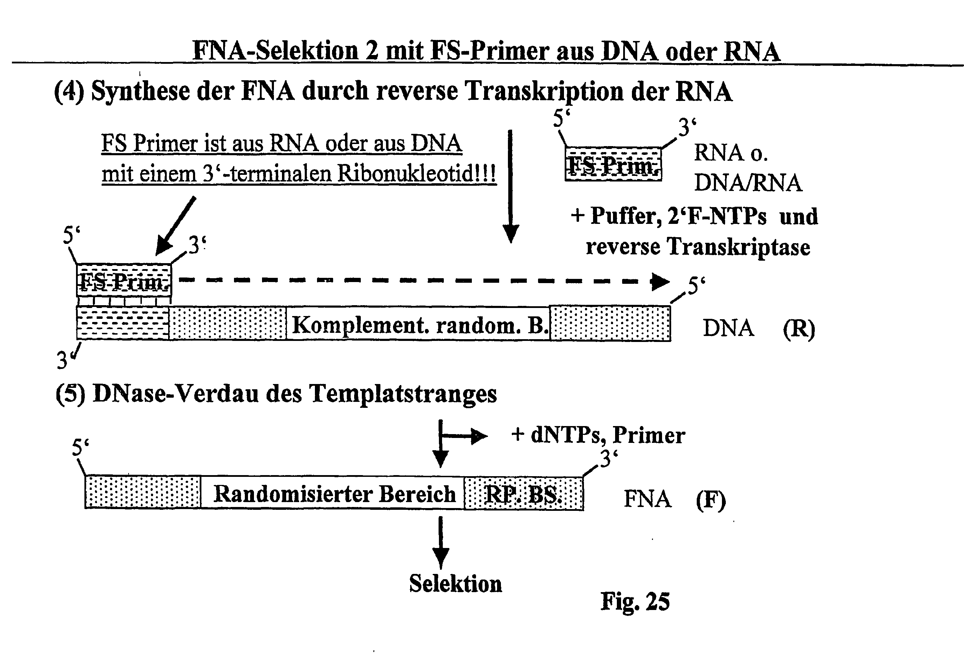

Fig. 25 zeigt schließlich die Synthese der FNA durch Reverse Transkription. Dabei wird in dieser Ausführungsform der Forward-Synthese-Primer aus RNA oder einer Mischung von DNA/RNA bestehen, wobei das 3'-Ende des Forward-Synthese-Primers ein Ribonukleotid aufweisen muss. Der Primer muss zumindest am 3'-Ende identisch sein mit dem weiteren Teilbereich des Forward-Primers. Nach der Reversen Transkriptionsreaktion erfolgt eine alkalische Hydrolyse oder ein RNase-Verdau, um die RNA-Anteile des Doppelstranges zu entfernen und somit die einzelsträngige FNA zu erhalten. Sofern der Forward-Synthese-Primer aus DNA besteht, ist dieser durch eine separate Behandlung, beispielsweise durch DNase-Behandlung zu entfernen, um zu einem Amplifikationsprodukt zu gelangen, welches dem in Schritt 1 von Fig. 23 gezeigten, zu amplifizierendem Nukleinsäure-Molekül entspricht.

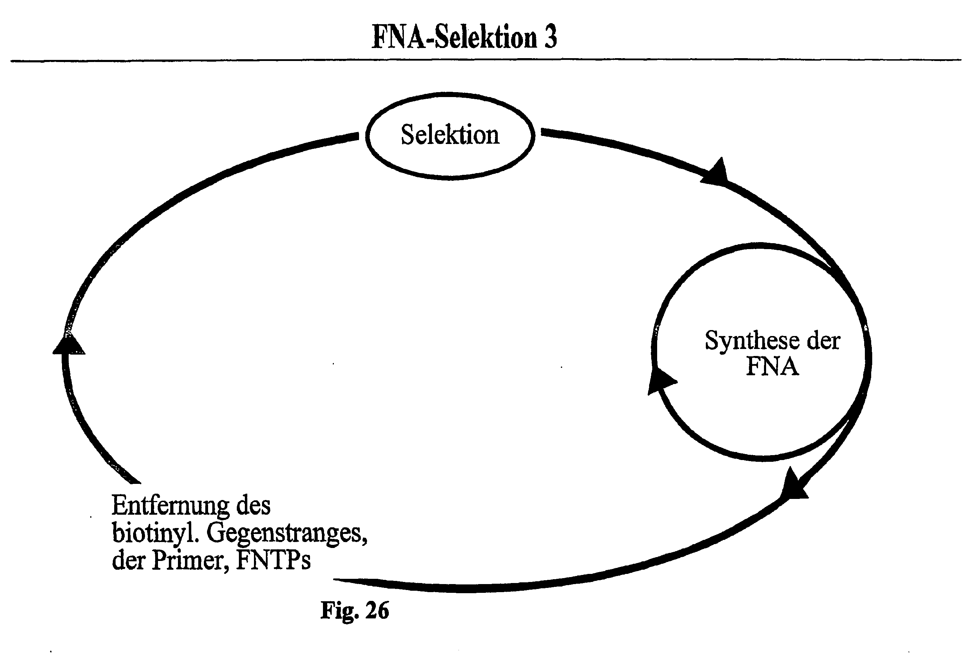

Figs. 26 bis 29 zeigen ein weiteres Amplifikationsverfahren für modifizierte Oligonukleotide, insbesondere 2'-Fluoro-Oligonukleotide unter Anwendung des erfindungsgemäßen Verfahrens zur Synthese von Nukleinsäure, dabei wird im wesentlichen eine Reverse Transkriptase-Aktivität sowie ein Thermocyclisieren realisiert.

Fig. 26 zeigt die Reaktionsfolge zur Amplifikation von 2'-Fluoro-Oligonukleotiden innerhalb eines *in vitro* Selektionsprozesses. Sie besteht lediglich aus dem wiederholten Kopieren der FNA mittels reverser Transkriptase, bevorzugterweise einer thermostabilen reversen Transkriptase, wie beispielsweise der DNA-Polymerase von Thermus thermophilus Alternativ kann auch eine Vorrichtung bereitgestellt werden, bei der Reverse Transkreptase immobilisiert vorliegt, wobei ein Denaturierung der FNA-Stränge erfolgt und die Primer-abhängige Synthese neuer Stränge bei niedriger Temperatur von dieser räumlich trennt. Dies kann beispielsweise dadurch bewerkstelligt werden durch ein System aus zwei unabhängigen Inkubationsgefäßen, die miteinander verbunden sind. Im diesem System werden die synthetisierten FNA, das heißt sowohl das Produkt wie Edukt, in Puffer in Anwesenheit von FNTPs und Primern, dem Forward-Synthese-Primer und dem biotinylierten Reverse Primer, zirkuliert, bzw. zwischen den Gefäßen hin- und herbewegt. Im auf Reaktionstemperatur (z.B. 42-51°C) temperierten ersten Gefäß befindet sich die immobilisierte reverse Transkriptase, die die vorhandenen FNA-Moleküle kopiert. Die resultierenden Doppelstränge werden dann im zweiten Gefäß bei hoher Temperatur von 95-99°C voneinander getrennt und auf dem Weg zum ersten Reaktionsgefäß abgekühlt. Alternativ zur Immobilisierung kann auch ein Molekularsieb am Ausgang des Reaktionsgefäßes, in dem sich das Enzym befindet, angebracht werden, dass die Reverse Transkriptase zurückhält.

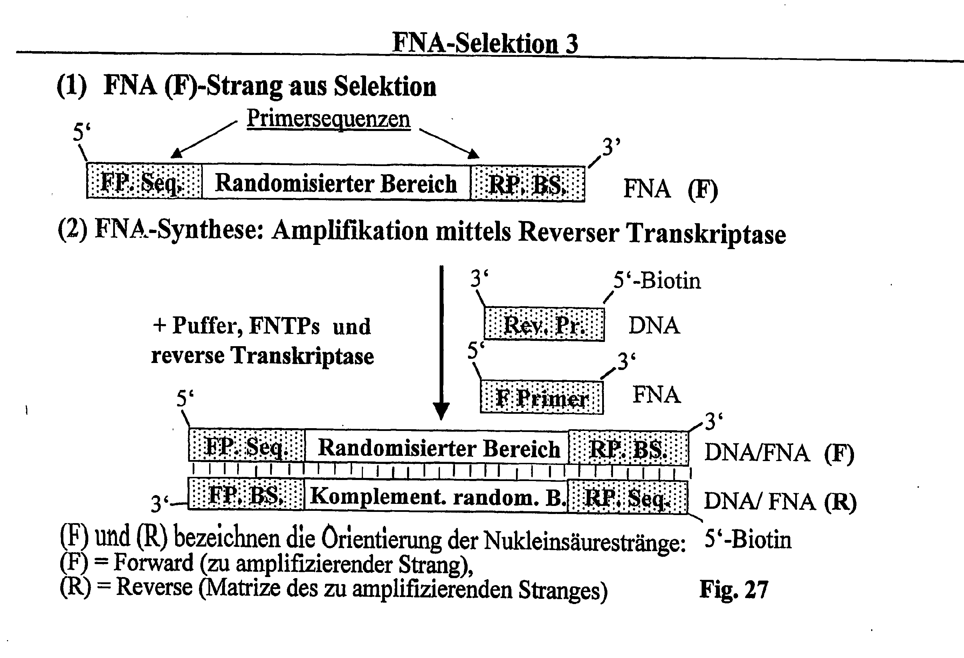

Fig. 27 zeigt die Amplifikation von FNA mittels Thermocyclieren mit FNTPs, einer Reversen Transkriptase und einem geeigneten Forward Primer aus FNA und einem Reverse Primer aus DNA. Der Reaktionsansatz enthält neben dem Primern noch 2'-F-NTPs sowie eine reverse Transkriptase, wie beispielsweise Superskript II von Invitrogen. Der Reverse-Primer besteht aus DNA und kann infolge seiner Komplementarität zur Reverse-Primer-Bindungsstelle der zu amplifizierenden Nukleinsäure, d.h. der Ausgangsnukleinsäure, an diese hybridisieren. Der Reverse-Primer weist im vorliegenden Falle am 5'-Ende ein Markierung auf, die eine Immobilisierung an einer Oberfläche erlaubt. Im vorliegenden Falle handelt es sich bei der Markierung um Biotin, das mit an einer Oberfläche immobilisiertem Streptavidin wechselwirken kann und somit den Primer bzw. damit verbundene Moleküle oder Molekülteile an der Oberfläche immobilisieren kann. Das Ergebnis der reversen Transkription ist ein doppelsträngiges Nukleinsäuremolekül, wobei ein Strang, hierin auch als (F)-Strang bezeichnet, dem zu amplifizierenden Nukleinsäuremolekül entspricht und der zweite Strang hierzu komplementär ist und als (R)-Strang bezeichnet wird. Der FNA-Primer hybridisiert dabei an die

Forward-Primer-Bindungsstelle des aus FNA aufgebauten und als Matrizenstrang dienenden (R)-Stranges der ersten bzw. folgend kopierten (F)-Stranges.

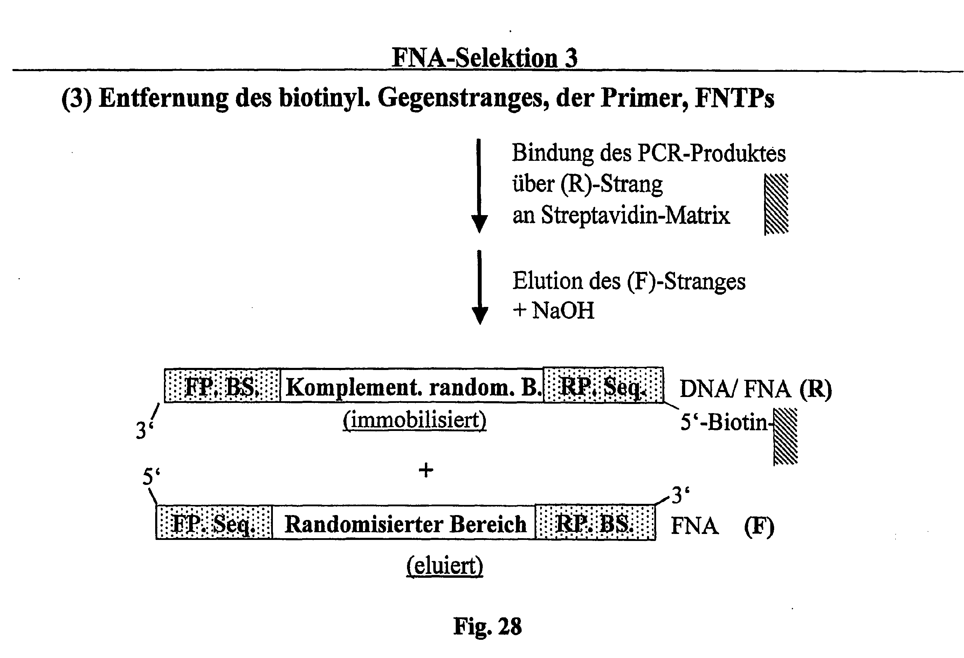

Das solchermaßen erhaltene doppelsträngige Produkt wird mittels des Markers an dem komplementären Strang an eine Streptavidin aufweisende Matrix immobilisiert, wie in Fig. 28 dargestellt. Alternativ zu Streptavidin kann beispielsweise Neutravidin verwendet werden. Geeignete Oberflächen sind beispielsweise Agarosen, die Oberfläche von Gefäßen oder die Oberflächen von Partikeln, insbesondere magnetischen Partikeln. Nicht eingebaute Forward-Primer und NTPs können auf diese Weise aus dem Reaktionsansatz weggewaschen werden. Zur Freisetzung des erwünschten amplifizierten Nukleinsäure-Moleküls, welches dem in Fig. 27 Teilschritt (1) dargestellt ist, entspricht, erfolgt eine Elution beispielsweise mittels NaOH des erwünschten Amplifikationsproduktes, d. h. der Stränge, die der in Fig. 27 in Teilschritt (1) dargestellte Nukleinsäure entsprechen.

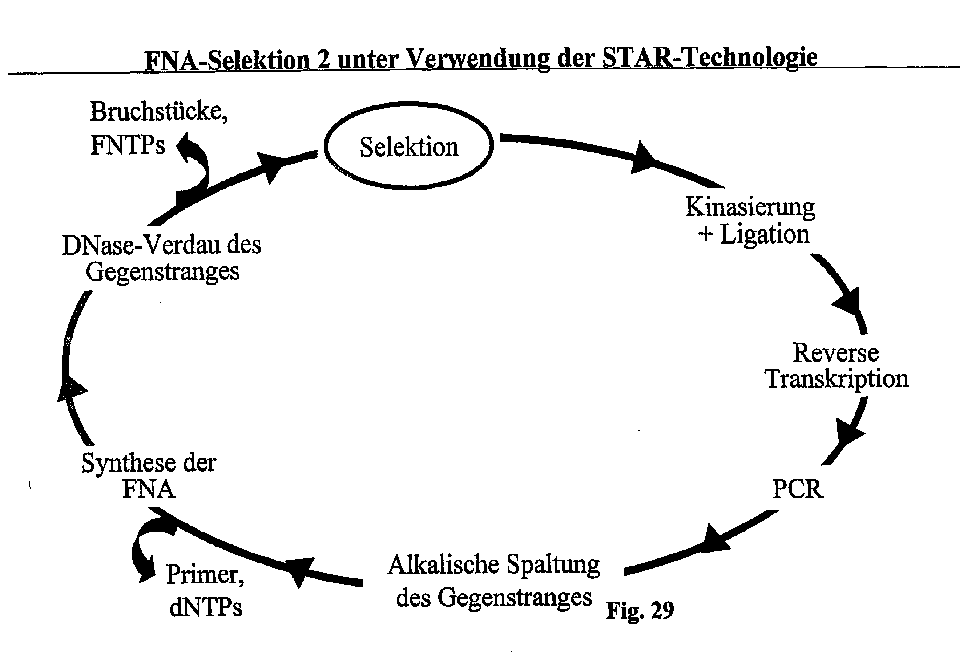

Figs. 29 bis 34 zeigen die Amplifikation von FNA-Sequenzen mit nur wenigen, d.h. typischerweise vier bis sechs bekannten Nukleotiden an jedem Ende des randomisierten Bereiches unter Anwendung der in den Figs. 13 bis 18 beschriebenen STAR-Technologie und stelle eine weitere Ausführungsform hiervon dar, wobei in dieser Ausführungsform der in vitro-Transkriptionsschritt nicht durchgeführt wird. Die konkreten Schritte bestehen aus einer Kinasierung und Ligation, reverser Transkription, Polymerase-Kettenreaktion, alkalische Spaltung des Gegenstranges, Synthese der FNA sowie DNase-Verdau des Gegenstranges, um eine Nukleinsäure zu erhalten, die hinsichtlich ihres grundsätzlichen Aufbaues der Ausgangsnukleinsäure, wie sie in der Selektion verwendet wird, entspricht.

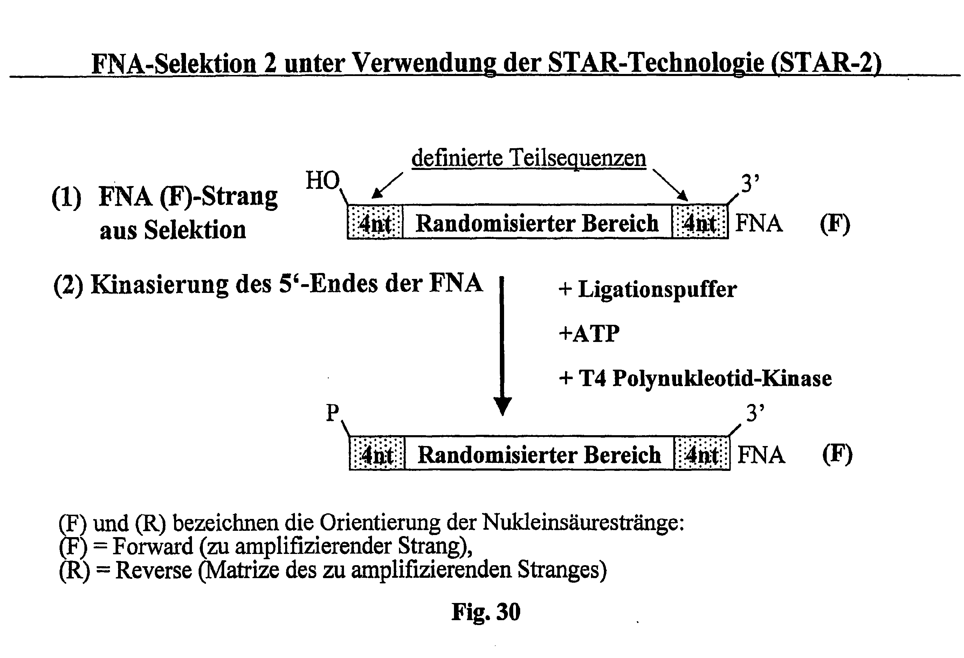

Fig. 30 (1) zeigt nochmals den Aufbau der im Rahmen der in vitro-Selektion als Ausgangpopulation verwendeten Nukleinsäuren, wie er auch in Fig. 14(1) beschrieben ist. Der weitere gezeigte Schritt der 5'-Phosphorylierung dieser Ausgangsnukleinsäure entspricht dem im Zusammenhang mit Fig. 14 beschriebenen Verfahren.

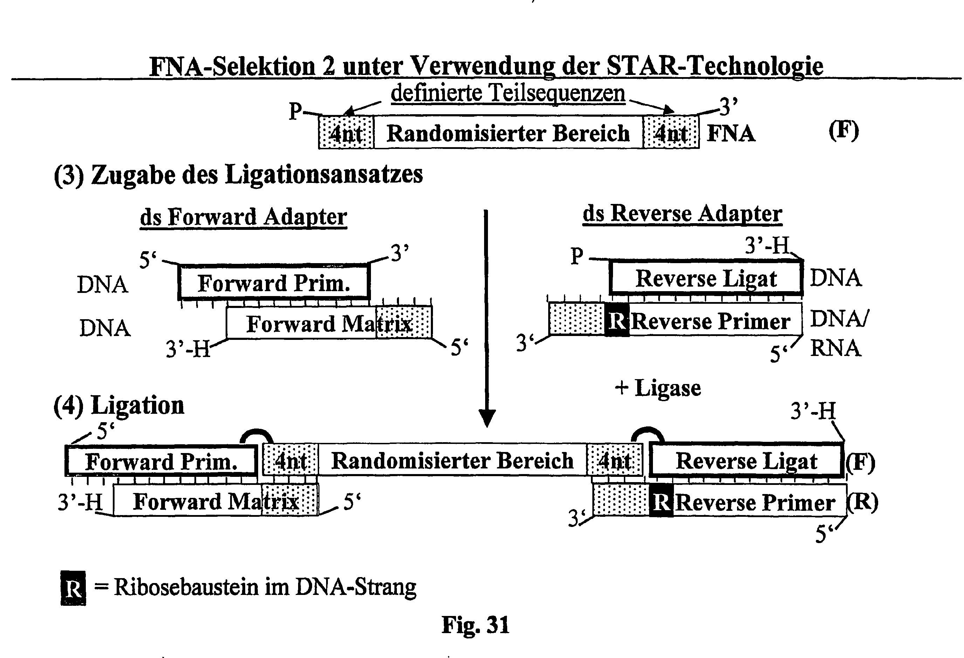

Das in Fig. 31 dargestellte Ligationsverfahren zeigt die Ligation der ersten und zweiten Adaptermoleküle, die jeweils aus einem ersten und zweiten Nukleinsäurestrang aufgebaut sind. Im vorliegenden Falle bestehen beide Adaptermoleküle aus DNA, wobei der zweite Strang des zweiten Adaptermoleküls eine Spaltungsstelle in Form eines Ribonukleotides aufweist, das eine Spaltung des Nukleinsäurestranges unter dem Einfluss von Alkali und gegebenenfalls Wärme erlaubt. Die Ausgestaltung des ersten und zweiten Adaptermoleküles bzw. der jeweiligen Nukleinsäurestränge entspricht ansonsten der im Zusammenhang mit Fig. 15 beschriebenen Ausgestaltung. Ebenso wie im Zusammenhang mit dem in Fig. 15 beschriebenen Verfahren kann als Ligase eine DNA-Ligase, bevorzugterweise eine T4-DNA-Ligase verwendet werden. Die Nukleinsäurestränge sind ebenso wie in Fig. 15 verwendet, bevorzugterweise am 3'-Ende geblockt, beispielsweise dadurch dass die terminalen Nukleotide 2'-3'-Didesoxynukleotide sind. Die Spaltstelle ist dabei so angeordnet, dass sie zu einer Aufspaltung des zweiten Nukleinsäurestranges des zweiten Adaptermoleküls am 5'-Ende desjenigen Teilbereiches des Nukleinsäurestranges des zweiten Adaptermoleküls führt, der mit der zweiten konstanten Sequenz der Ausgangsnukleinsäure hybridisiert. Das aus dieser Reaktion erhaltene Produkt besteht dabei zum einen aus der Ausgangsnukleinsäure, an deren 5'-Ende der erste Nukleinsäurestrang des ersten Adaptermoleküls und an dessen 3'-Ende der erste Nukleinsäurestrang des zweiten Adaptermoleküls anligiert wurde, wobei der zweite Nukleinsäurestrang des ersten Adaptermoleküls an dem ersten Nukleinsäurestrang an dem nun an die Ausgangsnukleinsäure ligierten ersten Nukleinsäurestrang des ersten Adaptermoleküls sowie die erste konstante Teilsequenz davon hybridisiert und der zweite Nukleinsäurestrang des zweiten Adaptermoleküls an den ersten Nukleinsäurestrang des zweiten Adaptermoleküls und die zweite konstante Teilsequenz der Ausgangsnukleinsäure hybridisiert vorliegt, wobei in dieser doppelsträngigen Nukleinsäure der randomisierte Bereich des Ausgangs-Nukleinsäure nicht enthalten ist.

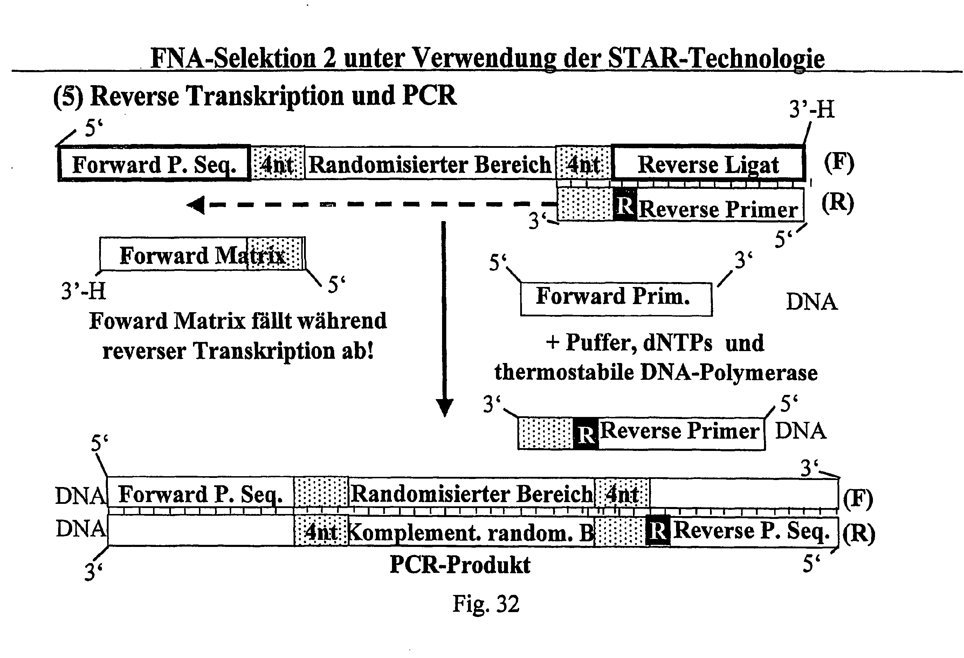

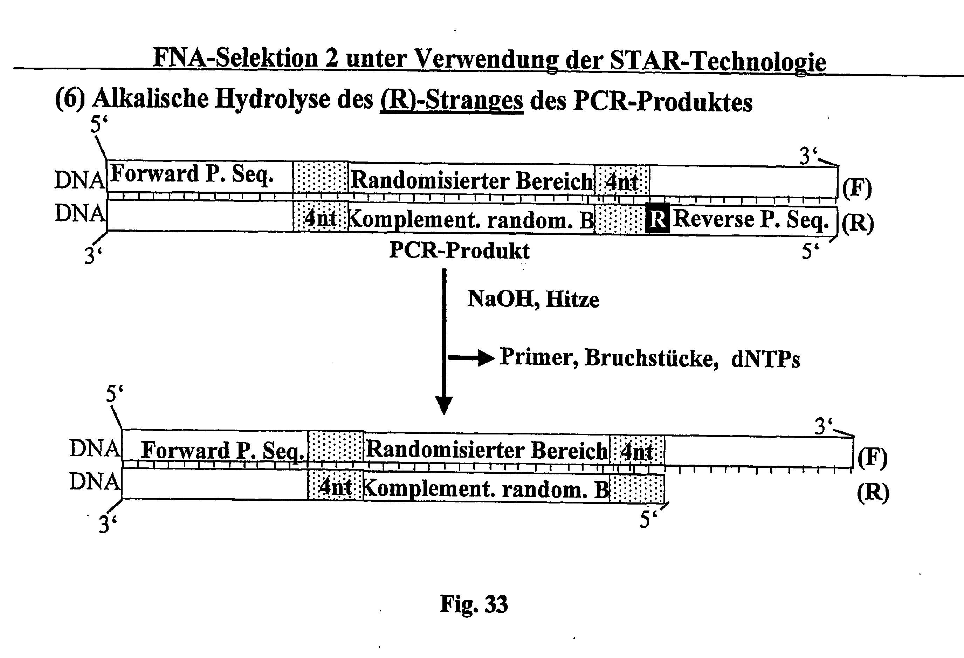

Wie in Fig. 32 dargestellt wird nun ausgehend von dem Ligationsprodukt, d.h. dem mit den ersten Nukleinsäuresträngen des ersten und zweiten Adaptermoleküls ligierten Ausgangsnukleinsäure unter Verwendung des Reverse-Primers eine cDNA mittels reverser Transkriptase hergestellt. Der cDNA-Strang weist dabei infolge der Verwendung des zweiten Nukleinsäurestranges des Adaptermoleküles die Spaltstelle auf. Der reversen Transkription schließt sich eine Amplifikationsreaktion, insbesondere eine Polymerase-Kettenreaktion, an, wobei als Primer der erste Nukleinsäurestrang des ersten Adaptermoleküls sowie der zweite Nukleinsäurestrang des zweiten Adaptermoleküls verwendet wird, wobei eine geeignete Polymerase, insbesondere DNA-Polymerase und bevorzugtererweise eine thermostabile DNA-Polymerase dem Reaktionsansatz hinzugesetzt wird, die die entsprechenden Nukleotide einbaut. Der zweite Strang des ersten Adaptermoleküls wird während der reversen Transkription von dem Ligationsprodukt entfernt. Am Ende der Amplifikationsreaktion wird eine doppelsträngige Nukleinsäure erhalten, wobei dieses hinsichtlich ihres Aufbaues dem Ligationsprodukt entspricht und dem dazu komplementären Strang. Von dem komplementären Strang wird sodann durch Alkali-Behandlung und Hitze-Behandlung derjenige Teil abgespalten, der zur zweiten konstanten Sequenz am 3'-Ende der Ausgangsnukleinsäure nicht komplementär ist. Der Primer sowie die dNTPs werden sodann entfernt (Fig. 33).

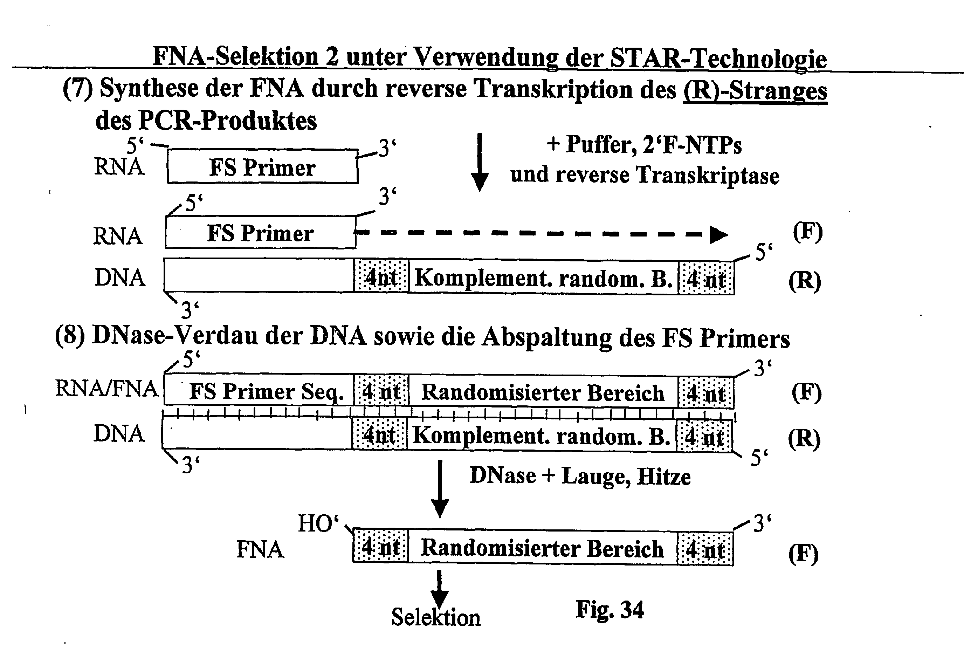

Fig. 34 zeigt die FNA-Synthese mit einem Forward-Synthese-Primer aus RNA, 2.'-F-NTPs und einer reversen Transkriptase. Als Matrizenstrang dient die im vorhergehenden Schritt erhaltene einsträngige Nukleinsäure, von der der nicht mit der zweiten konstanten Teilsequenz hybridisierende Teil des zweiten Nukleinsäurestranges des zweiten Adaptermoleküls entfernt worden ist. Der solchermaßen synthetisierte Strang umfasst die aus RNA bestehende Sequenz des Forward-Synthese-Primers, die erste konstante Teilsequenz, den randomisierten Bereich sowie die zweite Teilsequenz der Ausgangsnukleinsäure. Dieser Strang liegt im Komplex mit dem Matrizenstrang vor, der aus DNA besteht und durch die anschließende DNase-Behandlung entfernt werden kann. Die Sequenz des Forward-Synthese-Primers wird anschließend durch Behandlung mit RNase oder alkalische Behandlung bei erhöhter Temperatur oder Kombination beider entfernt, um eine Nukleinsäure zu ergeben, die der Ausgangs-Nukleinsäure zumindest im Wesentlichen entspricht.

Figs. 35-38 zeigen das erfindungsgemäße Verfahren zur Amplifikation von einzelsträngiger DNA (ssDNA), eingebettet in den Kontext einer *in vitro* Selektion. Das Verfahren beginnt mit einer Amplifikation der ssDNA als Ausgangsnukleinsäure durch PCR, wobei diese bei genügend Material durch eine Zweitstrangsynthese ersetzt werden kann, der sich eine Amplifikation des Gegenstranges anschließt, die durch eine *in vitro* Transkription erfolgt. Das Transkriptionsprodukt dient dann als Matrize für die Synthese der ssDNA mittels einer reversen Transkriptase, einem DNA-Primer und dNTPs. Die Matrize der reversen Transkription kann durch alkalische Hydrolyse in seine Mononukleoside (2'-3'-zyklisches Phosphat, 5'-OH) zerlegt werden. Diese können beispielsweise durch Filterung mit einem Molekularsieb, alkoholische Fällung mit Linearem Polyacrylamid und Ammoniumacetat leicht von dem DNA-Strang abgetrennt werden.

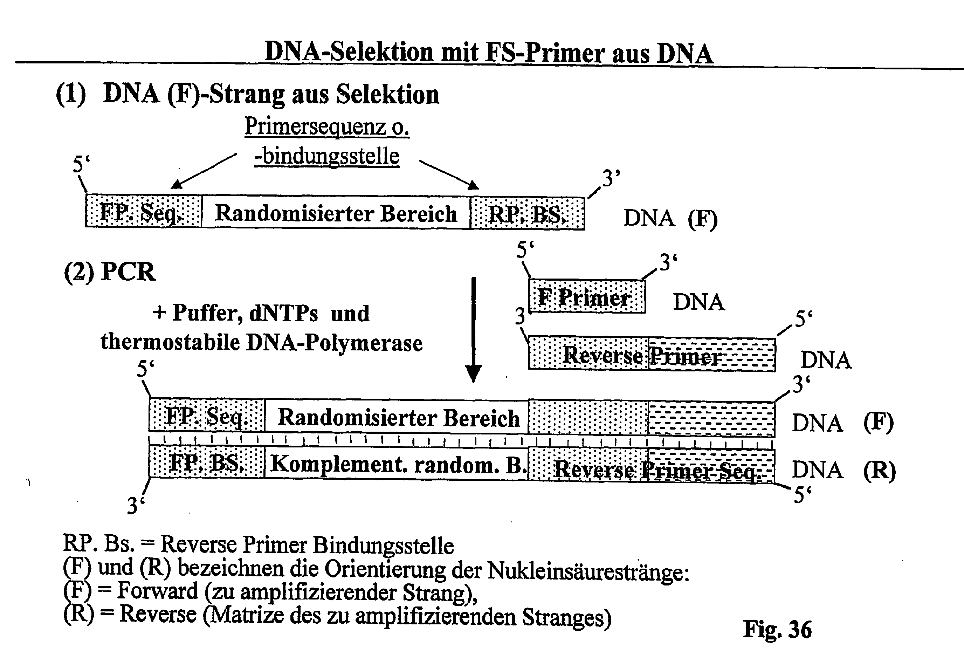

Fig. 36 zeigt die PCR-Amplifikation der zu amplifizierenden ssDNA (Forward Strang). Der Reverse Primer enthält einen 5'-überhängenden Teil, der einen T7 RNA-Polymerase-Promotor enthält. Das PCR-Produkt besteht aus einem Forward- und einem Reverse-Strang.

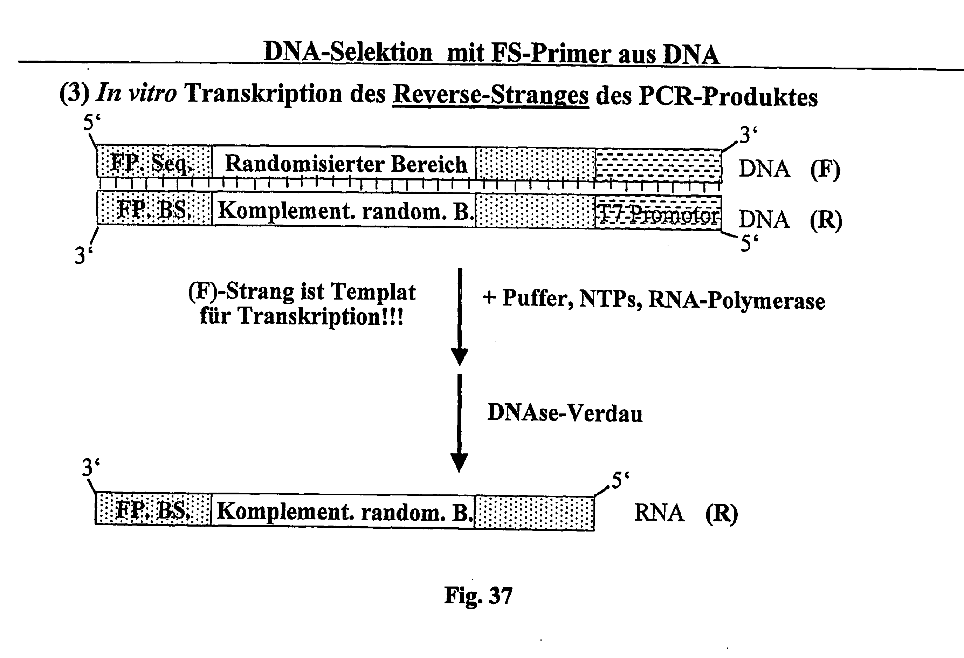

Fig. 37 zeigt die *in vitro* Transkription des Reverse Stranges, wobei der Forward-Strang als Matrize für die RNA-Synthese dient. Der Promotorbereich wird dabei nicht transkribiert. Es schließt sich ein DNase-Verdau des PCR-Produktes und der überschüssigen Primer an.

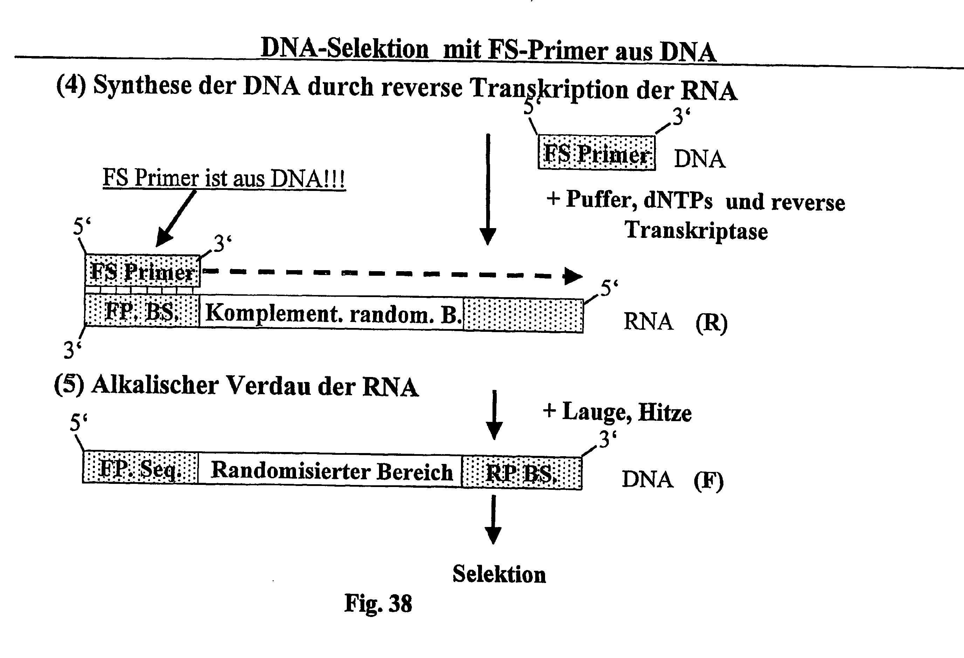

Fig. 38 zeigt die Synthese der einzelsträngigen DNA an der RNA-Matrize mittels einer reversen Transkriptase, einem geeigneten DNA-Primer und dNTPs. Die Matrize wird anschließend durch Lauge und Hitze hydrolysiert, so dass die einzelsträngige DNA lediglich von Nukleotiden, nicht aber von einem Gegenstrang ähnlicher oder gleicher Länge befreit werden muss.

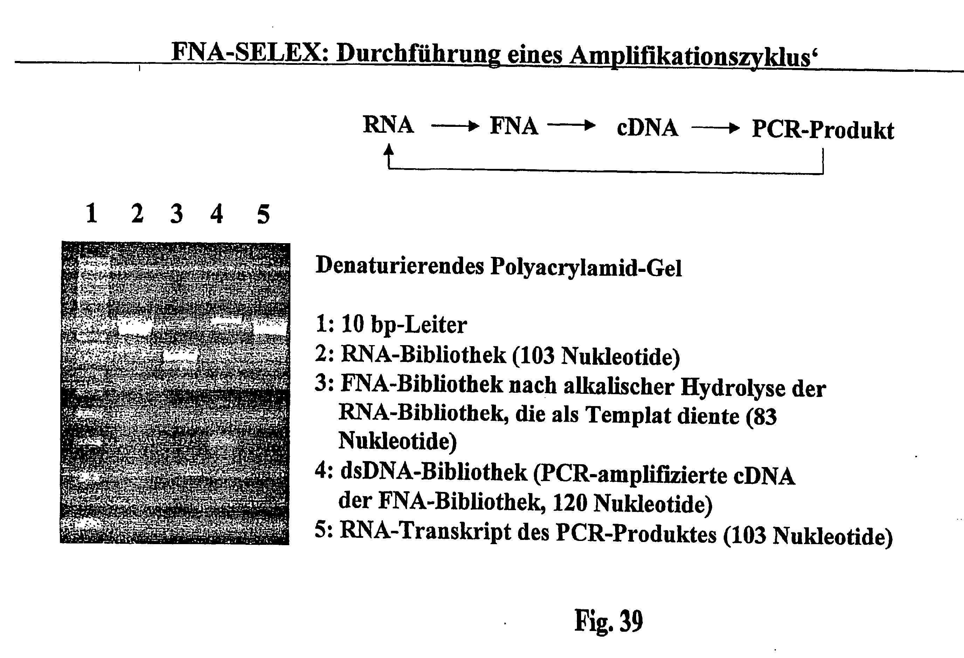

Fig. 39 zeigt experimentell die Durchführung eines Amplifikationszyklus', mit den Schritten der FNA-Selektion 1. Ausgangsmaterial war in eine RNA-Bibliothek. Diese wurde dann durch eine reverse Transkriptase mit einem DNA-Primer und FNTPs in eine FNA-Bibliothek umgeschrieben wurde (mit DNA-Primer am 5'-Ende). Nach Reverser Transkription mit dNTPs und einem überhängenden Reverse Primer (T7 RNA-Polymerase-Promotor-Bereich hängt über) wurde eine PCR durchgeführt. Das PCR-Produkt diente schließlich wieder als Ausgangsprodukt für eine *in vitro* Transkription. Damit wurde der Zyklus geschlossen. Die Reaktionen sind in Beispiel 1 unter "FNA-Bibliothek zum Durchlaufen des Amplifikationszyklus'" näher beschrieben.

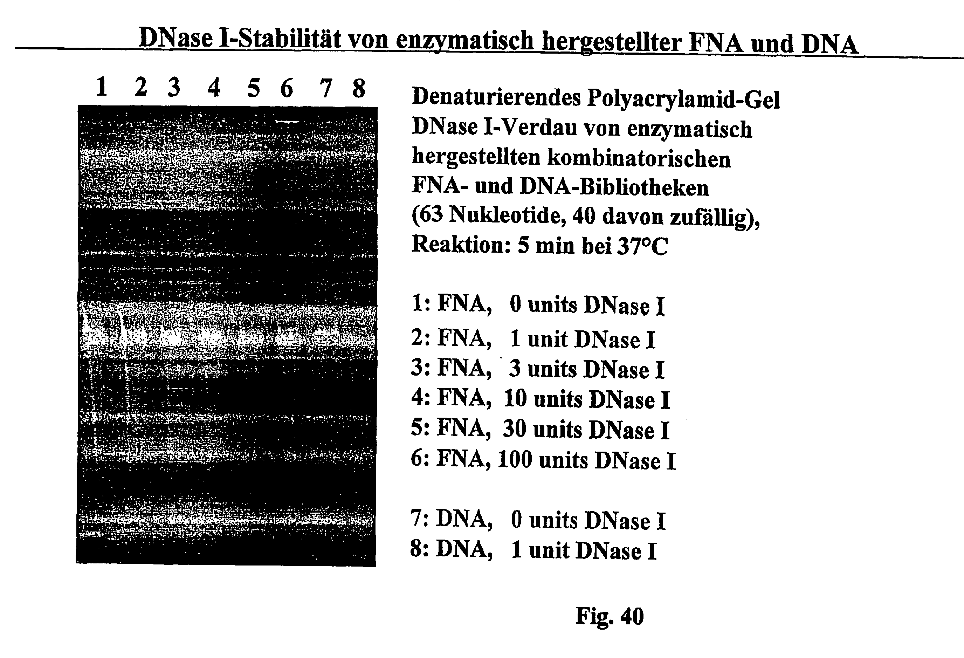

Fig. 40 zeigt die DNase-Stabilität einer FNA-Bibliothek, die zuvor mit Hilfe der Reversen Transkriptase aus RNA hergestellt worden war. Der DNA/RNA Primer wurde vor der DNase-Inkubation durch alkalische Hydrolyse von der FNA entfernt. Bei gleicher Inkubationsdauer ist die FNA deutlich mehr als 100x DNase-stabiler als eine gleich lange DNA, die auf gleiche Weise hergestellt wurde. Die Durchführung ist in Beispiel I unter "Herstellung der FNA-Bibliothek für Stabilitäts-Studien" und Beispiel 2 "DNaseI-Stabilität" beschrieben.

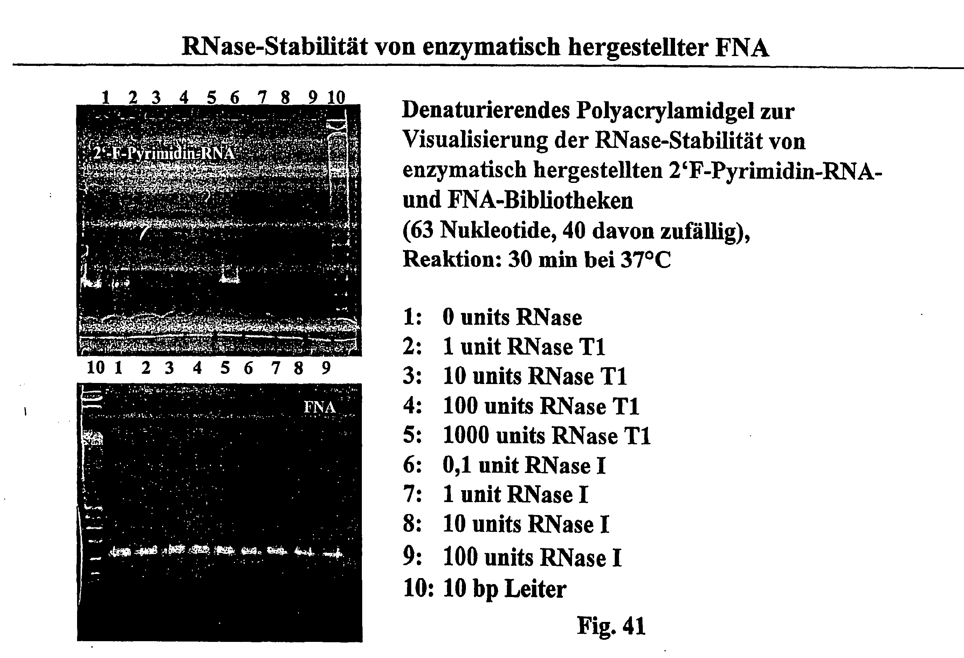

Fig. 41 zeigt die Stabilität der mittels der reversen Transkriptase hergestellten FNA-Bibliothek und einer durch *in vitro* Transkription hergestellten 2'-F-Pyrimidin-RNA gegenüber RNase T1 und RNase I. Die Durchführung ist in Beispiel 3 "RNase-Stabilität" beschrieben

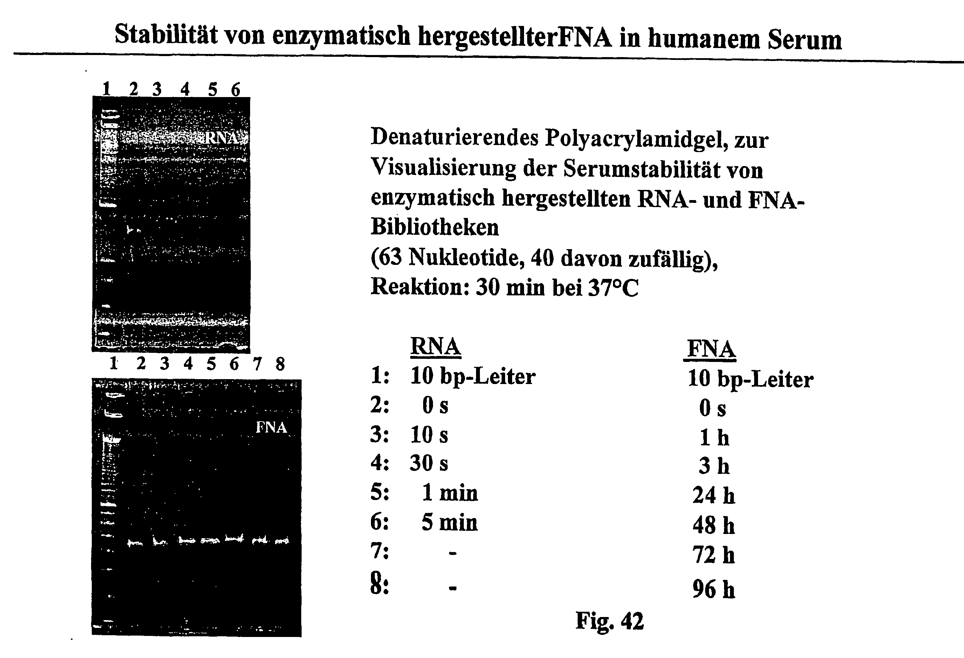

Fig. 42 zeigt die Stabilität der mittels der reversen Transkriptase hergestellten FNA-Bibliothek und einer durch *in vitro* Transkription hergestellten RNA im menschlichem Serum Die Durchführung ist in Beispiel 4 unter "RNase-Stabilität" beschrieben.

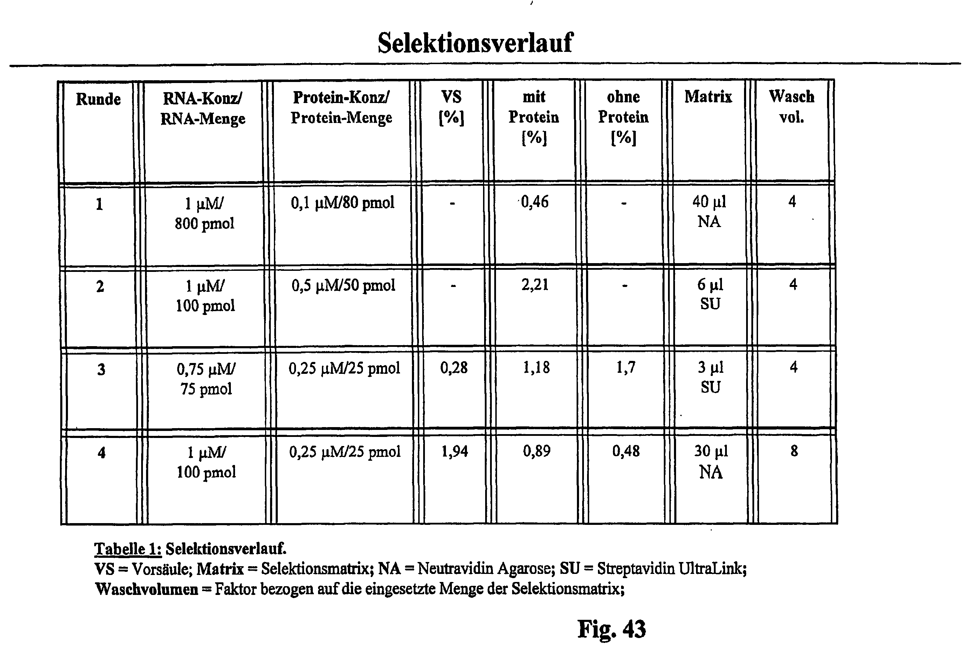

Fig. 43 zeigt den Selektionsverlauf der in Beispiel 1 beschriebenen *in vitro* Selektion.

### Beispiel 1: Verwendung von FNA für die Selektion von Protein-bindenden Nukleinsäuren zur Validierung des Verfahrens

Für die hierin beschriebenen Untersuchungen wurden die folgenden Materialien verwendet, wobei die Angaben zu den Herstellern der im Folgenden aufgeführten Substanzen, Lösungen und Enzyme bei den entsprechenden Textpassagen angeführt werden. Sofern nicht anders erwähnt, kamen die Reagenzien von Merck (Darmstadt, Deutschland). In allen Fällen wurde LiChromosolv Wasser von Merck (Darmstadt, Deutschland) verwendet.

Das zur Selektion verwendete Protein (basisch, pI von 9, 9 kDa, nicht Nukleinsäure-bindend) wurde an mindestens einer der zugänglichen Amino-Funktionen der Aminosäuren unter Verwendung der Biotinylierungs-Kits "EZ-Link Sulfo-NHS-LC-LC-Biotin" (Pierce, Rockford, USA) mit einem Biotin-Linker entsprechend den Herstellerangaben versehen, um die Trennung von ungebundenen Nukleinsäuren mittels der Biotin-Streptavidin- oder Biotin-Neutravidin-Anbindung zu ermöglichen. Hierfür wurde als Selektiosmatrix Neutravidin-Agarose und UltraLink Plus Immobilized Streptavidin Gel (beide von Pierce, Rockford, USA) verwendet. Überschüssige Linker wurden durch ein Molekularsieb, der Filtereinheit "YM-3" (Ausschlussgrenze MW 3000, Amicon/Milliore, Bedford, USA), entsprechend den Herstellerangaben abgetrennt.

Die verwendeten Oligonukleotide, wie Primer und die initiale DNA-Bibliothek wurden alle bei der NOXXON Pharma AG mit Standard-Phosphoramiditchemie synthetisiert. Die Sequenzen finden sich in Beispiel 6.

### Herstellung der FNA-Bibliothek

### Herstellung des Templates für die FNA-Synthese: Fill-in-Reaktion + In Vitro Transkription

Zunächst wurden 3 nmol einer synthetische DNA-Bibliothek *BSA-1C initiale Bibliothek* mittels einer Fill-in-Reaktion unter Verwendung des *BSA 1C-Reverse Primer T7* und der Vent-(exo)-DNA-Polymerase (NEB, Frankfurt a.M., Deutschland) doppelsträngig gemacht. Durch diese Reaktion mit dem *BSA 1C-Reverse Primer T7* wurde auch der T7 RNA-Polymerase-Promotor eingeführt.

### Fill-in-Reaktion

| **Komponente** | **finale Konzentration** |
|---|---|
| 10x Puffer (NEB) | 1x |
| Betain | 0,5 M |
| dNTPs (Larova, Teltow, Deutschland) | 0,5 mM |
| *BSA-1C Reverse Primer T7* | 6 µM |
| *BSA-1C initiale Bibliothek* | 2 µM |
| Vent-(exo⁻)-DNA-Polymerase (NEB) | 10 U/100 µl_{Reaktionsansatz} |

Zunächst wurde der Ansatz ohne Enzym bei 95°C für 10 min. denaturiert, dann für 5 min. auf Eis gestellt, das Enzym hinzugegeben und 2 Stunden bei 63°C inkubiert.

Danach wurde die dsDNA durch EtOH-Fällung (20 µg GlycoBlue (Ambion) und 2,5 Vol. absolutem Ethanol, 30 min bei -80°C, 30 min zentrifugiert bei 13.200 rpm (16.100 g), 4°C; Pellet 1x mit 70 %igem EtOH gewaschen) entsalzt.

Die dsDNA-Bibliothek diente dann als Templat für eine *in vitro* Transkription.

### In vitro Transkription

| **Komponente** | **finale Konzentration** |
|---|---|
| Transkriptionspuffer (80 mM HEPES/KOH, pH 7,5, 22 mM MgCl₂; 1 mM Spermidin) | 1x |
| DTT | 10 mM |
| NTPs (Larova, Teltow, Deutschland) | 4,0 mM |
| RNaseOUT (Invitrogen, Carlsbad, USA) | 1 µl/100 µl_{Reaktionsansatz} |
| Fill-in-Reaktionsprodukt | 1 µM |
| T7 RNA-Polymerase (Stratagene, La Jolla, USA) | 50 U/100 µl_{Reaktionsansatz} |

Die Reaktion lief für 2 bis 12 Stunden bei 37°C.

Das verbliebene DNA-Templat wurde im Anschluss an die *in vitro*-Transkription mit 20 Einheiten DNAse I (Sigma) innerhalb von 20 min verdaut. Nach Zugabe von Beladungspuffer (engl. "loading buffer") (7M Harnstoff, Xylencyanol, Bromphenolblau) wurde der Ansatz denaturiert und über ein 10 %iges denaturierendes Polyacrylamidgel aufgereinigt. Die Bande des Transkriptes wurde per *UV-Shadowing* aus dem Gel ausgeschnitten und mittels Crush and Soak eluiert. Das resultierende Eluat wurde Ethanol-präzipitiert und das Pellet nach einmaligem Waschen mit eiskaltem 70 %igen Ethanol in Wasser resuspendiert.

Die RNA-Bibliothek stellte das Templat für die FNA-Synthese mittels einer Reversen Transkriptase dar.

### FNA-Synthese und Markierung der FNA

### FNA-Synthese

| **Komponente** | | | **finale Konzentration** |
|---|---|---|---|
| RNA-Bibliothek | | | 1 µM |
| *BSA-1C FS Primer 3rG* | | | 5 µM |
| Q-solution (Qiagen, Hilden, Deutschland) | | | 1x |
| **5 min bei 95°C, 5 min auf Eis** | | | |
| 1st strand buffer (Invitrogen, Carlsbad, USA) | | | 1x |
| DTT (Invitrogen, Carlsbad, USA) | | | 10 mM |
| FNTPs (TriLink Biotech, San Diego, USA) | | | 0,5 mM |
| Superscript II (Invitrogen, Carlsbad, USA) | | | 10 U/µl |
| | | | |

| **Temperaturbedingungen:** | | | |
|---|---|---|---|
| | 1.51°C | 20 min | |
| | 2.54°C | 10 min | |

Nach der FNA-Synthese wurde der Templat-Strang (RNA) alkalisch hydrolysiert.

### Alkalische Hydrolyse des Templat-Stranges

| **Komponente** | | **finale Konzentration** |
|---|---|---|
| FNA-Synthese-Reaktionsprodukt | | alles |
| NaOH | | 0,3 M |
| **10 min bei 95°C, 5 min auf Eis** | | |
| NaOAc | | 0,1 M |
| HCl | zum Neutralisieren | entspr. NaOH |

Danach wurde die FNA-Bibliothek durch EtOH-Fällung (20 µg GlycoBlue (Ambion) und 2,5 Vol. absolutem Ethanol, 30 min bei -80°C, 30 min zentrifugiert bei 13.200 rpm (16.100 g), 4°C; Pellet 1x mit 70 %igem EtOH gewaschen) entsalzt. Nach Zugabe von Loading Buffer (7M Harnstoff, Xylencyanol, Bromphenolblau) wurde der Ansatz denaturiert und über ein 10 %iges denaturierendes Polyacrylamidgel aufgereinigt. Die Bande der FNA wurde per *UV-Shadowing* aus dem Gel ausgeschnitten und mittels Crush and Soak eluiert. Das resultierende Eluat wurde Ethanol-präzipitiert und das Pellet nach einmaligem Waschen mit eiskaltem 70 %igen Ethanol in Wasser resuspendiert.

Um die Bindung der FNA-Bibliothek während der *in vitro* Selektion zu verfolgen, wurde diese an der 5'-OH-Gruppe durch Kinasierung radioaktiv mit ³²P markiert.

### Kinasierung der FNA

| **Komponente** | **finale Konzentration** |
|---|---|
| FNA | 10 µM |
| "Forward"-Puffer (Invitrogen) | 1x |
| γ-³²PATP | 1 µl/10 µl_{Reaktionsansatz} |
| T4 Polynukleotid-Kinase (Invitrogen) | 1 U/µl_{Reaktionsansatz} |

Die Reaktion verlief für eine Stunde bei 37°C und wurde bei 65°C (10°min.) gestoppt.

Nach Zugabe von Loading Buffer (7M Harnstoff, Xylencyanol, Bromphenolblau) wurde der Ansatz denaturiert und über ein 10 %iges denaturierendes Polyacrylamidgel aufgereinigt. Die FNA wurde per *UV-Shadowing* aus dem Gel ausgeschnitten und mittels Crush and Soak eluiert. Das resultierende Eluat wurde Ethanol-präzipitiert und das Pellet nach einmaligem Waschen mit eiskaltem 70 %igen Ethanol in Wasser resuspendiert.

### Selektionsschritte

### Denaturierung und Faltung der FNA

Alle nicht-enzymatischen Schritte der Selektion - mit Ausnahme der Denaturierung - wurden in Selektionspuffer (20 mM Tris pH 7,4; 150 mM NaCl; 4 mM KCl; 1 mM MgCl₂ (alle Ambion, Austin, USA); 1 mM CaCl₂ (Merck, Darmstadt, Deutschland) und 0,1 % Tween-20 (Roche Diagnostics, Mannheim, Deutschland) durchgeführt. Die Denaturierung erfolgte für 5 Minuten bei 95°C in Selektionspuffer. Nach der Denaturierung wurde die FNA zunächst für 15 Minuten bei 37°C auf 37°C abgekühlt.

### Anbindung

Im Anschluss an die Faltung wurde die FNA zunächst bei 37°C für 30 Minuten ohne Protein mit der Selektionsmatrix (entweder Neutravidin-Agarose oder mit UltraLink Plus Immobilized Streptavidin, Pierce, USA) inkubiert. Diese sogenannte Vorselektion diente der Abtrennung von potentiellen Matrixbindem. Nach diesem Inkubationsschritt wurde die Selektionsmatrix durch Zentrifugation sedimentiert und die ungebundene FNA im Überstand abgenommen. Ein Teil der FNA wurde in einem Ansatz mit biotinyliertem Protein versetzt und für eine Stunde bei 37°C inkubiert. Der andere Teil der FNA wurde ohne biotinyliertes Protein (erst ab Runde 3) für eine Stunde bei 37°C inkubiert. Daraufhin wurde dem Bindungsansatz die biotinbindende Selektionsmatrix zugesetzt. Die Selektionsmatrix mit den daran gebundenen Protein-FNA-Komplexen wurde nach 30 min Inkubation bei 37°C von der Lösung durch Zentrifugation getrennt und mit Selektionspuffer gewaschen.

### Elution der bindenden FNA-Moleküle

Hierzu wurde die nach dem Waschen auf der Selektionsmatrix verbliebene FNA zweimal mit jeweils 200µl 8 M Harnstoff mit 10 mM EDTA (beide von Ambion, Austin, USA) für 15 Minuten im ersten Schritt bei 65°C und im zweiten Schritt bei 95°C vom Matrixmaterial eluiert. Die- eluierte FNA wurde mit 400 µl Phenol/(Chloroform/Isoamylalkohol)-Gemisch (1:(1:1/24)) (Applichem, Darmstadt, Deuschland) versetzt, 5 min bei 13.000 rpm bei Raumtemperatur zentrifugiert, die wässrige Phase (Überstand) abgenommen, die phenolische Phase einmal mit 100 µl Wasser re-extrahiert, die wässrigen Phasen vereinigt und mit 500µl Chloroform-Isoamylyalkohol-Gemisch (24:1) (Applichem, Darmstadt, Deuschland) geschüttelt, 5 min bei 13000 rpm bei Raumtemperatur zentrifugiert und die obere wässrige Phase abgenommen.

Die wässrige Phase wurde daraufhin mit 2,5 fachen Volumen 100% Ethanol (Merck, Darmstadt, Deutschland), 0,3 M Natriumacetat, pH 5,5 (Ambion, Austin, USA) und 1 µl Glycogen (Roche Diagnostics, Mannheim, Deuschland) für 30 min bei -80°C gefällt und für 30 min bei 14.000 rpm (4°C) zentrifugiert. Das Pellet wurde einmal mit eiskaltem 70 %igen Ethanol (Merck, Darmstadt, Deutschland) gewaschen.

### Amplifikation

### Synthese der cDNA

Um die FNA-Bibliothek mit Hilfe einer PCR-Reaktion amplifizieren zu können, wurde die FNA in DNA umgeschrieben.

### cDNA-Synthese

| **Komponente** | **finale Konzentration** |
|---|---|
| FNA aus Selektion | max. 0,5 µM |
| *BSA-1C Reverse Primer T7* | 2,5 µM |
| Q-solution (Qiagen, Hilden, Deutschland) | 1x |
| **5 min bei 95°C, 5 min auf Eis** | |
| 1st strand buffer (Invitrogen, Carlsbad, USA) | 1x |
| DTT | 10 mM |
| dNTPs (Larova, Teltow, Deutschland) | 0,5 mM |
| Superscript II (Invitrogen, Carlsbad, USA) | *20U*/*µl* |

| **Temperaturbedingungen:** | | |
|---|---|---|
| | 1.51°C | 20 min |
| | 2.54°C | 10 min |
| | 3.4°C | unendlich |

### PCR

| **Komponente** | **finale Konzentration** | |
|---|---|---|
| PCR-Puffer (NEB) | 1x | |
| *BSA-1C Forward-Primer* | 5 µM | |
| *BSA-1C Reverse Primer T7* | µM | |
| dNTPs (Larova, Teltow, Deuschland) | 0,2 mM | |
| vent-(exo⁻)-DNA-Polymerase (NEB) | 0,05 U/µl | |
| cDNA | 0,1-0,5 µM | |

| **Temperaturbedingungen:** | | |
|---|---|---|
| | 1. 95°C | 4 min |
| | 2. 95°C | 1 min |
| | 3. 68°C | 1 min |
| | 4. 72°C | 1 min |
| | 5. Gehe zu 2 | 11x |
| | 6. 72°C | 6 min |
| | 7.4°C | bis Stopp |

Es schloss sich eine Ethanol-Fällung an, um die dsDNA für die sich anschließende *in vitro* Transkription vorzubereiten.

Im Folgenden wurde die in der *in vitro* Transkription hergestellte RNA in FNA umgeschrieben, die als Templat dienende RNA und der *BSA-1C FS- Primer 3rG* durch alkalische Hydrolyse fragmentiert, über ein Molekularsieb (YM-Filtereinheiten, Amicon/Millipore, Bedford, USA) abgetrennt und die FNA radioaktiv markiert (wie bereits beschrieben). Die so angereicherte FNA-Bibliothek wurde in die nächste Selektionsrunde eingesetzt.

In Figur 39 sind die einzelnen Produkte der während einer Selektionsrunde durchzuführenden Amplifikationsschritte dargestellt. Dazu wurden mittels denaturierender Polyacrylamid-Gelelektrophorese (10 % Polyacrylamid, 39:1 Bisacrylamid 7 M Harnstoff, 1x TBE) Aliquots der Produkte der einzelnen Reaktionen aufgetrennt und nach Ethidiumbromid-Färbung auf einem UV-Transilluminator visualisiert. Auf das Gel wurden 5 pmol RNA-Templat, und ein Aliquot der FNA-Synthese nach alkalischer Hydrolyse des Templatstranges, das bei 100 % Umsatz ebenfalls 5 pmol entspräche, aufgetragen. Weiterhin wurden ca. 5 pmol der PCR-Reaktion und 0,5 µl der abschließenden Transkription aufgetragen (**Fig. 39**).

### Selektionsverlauf

Während der Selektion wurde mit radioaktiv markierter FNA gearbeitet und die Anbindung als prozentualer Wert bezogen auf die eingesetzte Menge an FNA ermittelt. Die Radioaktivität wurde mit einem Scintillationszähler (Beckman, Fullerton,USA) bestimmt. Der Selektionsverlauf ist in Figur 43 dargestellt. Nach vier Selektionsrunden konnte erstmalig eine Zielmolekül-spezifische Anreicherung (0,89% in Anwesenheit des Proteins) mit einem Faktor von 1,85 gegenüber der Kontrolle (0,48% ohne Protein) gemessen werden.

### Beispiel 2: Herstellung einer FNA-, DNA-, 2'-F-Pyrimidin-RNA und RNA-Bibliothek für die Stabilitäts-Studien

### FNA-Bibliothek/DNA-Bibliothek

Zunächst wurde die synthetische DNA-Bibliothek *BSA-1A initiale Bibliothek* mittels einer Fill-in-Reaktion unter Verwendung des *BSA-1A Reverse Primer T7* doppelsträngig gemacht. Die dsDNA wurde dann in die *in vitro* Transkription eingesetzt. Als Primer für die FNA-Synthese der FNA-Bibliothek wurde der *BSA-1A FS-Primer 3rG* + 15 eingesetzt. Nach der FNA-Synthese wurde der RNA-Templat-Strang und der Primer durch alkalische Hydrolyse entfernt, so dass eine 63 Nukleotide lange FNA-Bibliothek mit 40 randomisierten Nukleotiden am 5'-Ende entstand ((N)40-CACGAGTGAAGTCTGAGCTCC-3'). Abschließend erfolgte eine Ethanol-Fällung zur Entsalzung der Probe. Die Komponenten und Bedingungen entsprechen der oben beschriebenen FNA-Synthese, der alkalischen Hydrolyse und der Ethanolfällung.

Parallel wurde zum Vergleich DNA hergestellt. An Stelle der FNA-Synthese wurde eine DNA-Synthese mit dem FNA-Synthese-Protokoll durchgeführt, wobei die FNTPs durch dNTPs ersetzt wurden (Protokolle aller Reaktionen unter Verwendung der hier aufgeführten Oligonukloetide, siehe Beispiel 1)

### RNA/2'-F-Pyrimidin-RNA-Bibliothek

Als weitere Kontrollen dienten Bibliotheken aus RNA und 2'-F-Pyrimidin-RNA, die durch *in vitro* Transkriptionen erzeugt wurden. Dazu wurde ausgehend vom *BSA-1A initiale Bibliothek* eine PCR unter Verwendung des *BSA-1 FS-Primer 3'G* und des *BSA1 Reverse Primer T7* durchgeführt (Protokoll siehe Beispiel 1), das PCR-Produkt alkalisch hydrolysiert (analog wie in Beispiel 1 für die alkalische Hydrolyse nach der FNA-Synthese) und eine *in vitro* Transkription zur Herstellung der RNA-Bibliothek (Protokoll siehe Beispiel 1) und eine zur Herstellung der 2'-F-Pyrimidin-Bibliothek (siehe unten) durchgeführt (Länge der Bibliotheken: 63 Nukleotide). Nach der Transkription wurde der RNase-Inhibitor "RNaseOut" (Invitrogen) durch Phenol-Chloroform-Extraktion mit anschließender Ethanolfällung entfernt, um eine unerwünschte Inhibition der RNasen zu verhindern.

Die 2'-F-Pyrimidin-Synthese wurde folgendermaßen durchgeführt:

| **Komponente** | **finale Konzentration** |
|---|---|
| Txn-Puffer mit 6mM MgCl2 (Epicentre, Madison, | |
| USA) | 1x |
| DTT (Merck, Darmstadt, Deuschland) | 10 mM |
| MgCl2 (Ambion, Austin, USA | 5 mM |
| ATP (Larova, Teltow, Deutschland) | 1,5 mM |
| GTP (Larova, Teltow,Deutschland) | 1,5 mM |
| 2'-F-CTP (TriLink Biotech, San Diego, USA) | 1,5 mM |
| 2'-F-UTP (Noxxon Pharma AG, Berlin, DE) | 1,5 mM |
| PCR-Produkt BSA-1A Pool | 1 µM |
| T7 R&DNA-Polymerase (Epicentre, Madison, USA) | 1,5 U/µl |

Die Reaktion lief für 4 bis 12 Stunden bei 37°C.

### Beispiel 3: DNase I-Stabilität

Je ca. 4 pmol FNA-Bibliothek und gleichermaßen hergestellte DNA-Bibliothek (siehe Beispiel 2) wurden in 10 µl in dem vom Hersteller empfohlenen Puffer (100 mM NaOAc, 5 mM MgCl₂) mit unterschiedlichen Mengen DNase I (Sigma) für 5 min bei 37°C inkubiert. DNase I-Verdünnungen wurden unmittelbar vor Verwendung in demselben Puffer hergestellt.

Der DNase-Verdau wurde durch Zugabe von 6 M Harnstoff/15 mM EDTA und Denaturieren (10 min bei 95°C) beendet.

Die Ansätze wurden auf einem 10 %igen denaturierenden Polyacrylamid-Gel aufgetrennt und nach Färbung mit Ethidiumbromid auf einem UV-Transilluminator visualisiert (Fig. 40). Es zeigte sich, dass FNA mindestens 100x DNase I-stabiler ist als DNA.

### Beispiel 4: RNase-Stabilität

Zur weiteren Charakterisierung der FNA wurde auch die RNase-Stabilität der enzymatisch hergestellten FNA-Bibliothek im Vergleich zu RNA und 2'-F-Pyrimidin-RNA untersucht (Herstellung siehe Beispiel 2). Je ca. 4 pmol FNA, RNA oder 2'-Pyrimidin-RNA-Bibliothek wurden in 10 µl des vom Hersteller empfohlenen Puffers mit unterschiedlichen Mengen RNase T1 (1 - 1000 units) und RNase I (0,1 - 100 units) bei 37°C für 30 min inkubiert. RNase T1 stammt aus Aspergillus oryzae und schneidet nach Gs. RNase I stammt aus *E. coli* und schneidet RNA unspezifisch. Beide RNasen wurden von Ambion, Austin, Texas, USA rekombinant in E. coli hergestellt. RNase T1-Puffer: 10 mM Tris, pH 8, 100 mM NaCl; RNase I-Puffer: 50 mM Tris, pH 7,5, 1 mM EDTA.).

Die Reaktion wurde durch Zugabe von 20 µl Stopp-Lösung (2 % SDS, 50 mM EDTA, 6 M Harnstoff) und Schockgefrieren in Trockeneis-Ethanol-Gemisch gestoppt. Die Proben blieben anschließend bis zum Denaturieren (5 min bei 95°C) auf Eis, wurden nach dem Denaturieren auf Eis schnell abgekühlt und auf ein analytisches, denaturierendes 10%iges Polyacrylamid-Gel aufgetragen.

Nach elektrophoretischer Trennung wurden die Banden mit Ethidiumbromid gefärbt und mit UV-Licht auf einem Transilluminator visualisiert (**Fig. 41**). Es zeigte sich, dass FNA im Gegensatz zu 2'-F-Pyrimidin-RNA und RNA (nicht dargestellt) unter den beschriebenen Bedingungen nicht von den genannte RNase angegriffen wird.

### Beispiel 5: Serumstabilität von FNA

Die Stabilität von FNA und RNA (Herstellung siehe Beispiel 2). gegenüber Serumnukleasen wurde in humanem Serum evaluiert Um pH-Verschiebungen im Laufe der viertätigen Versuchsdauer zu minimieren wurden dem Serum 50 mM Natrium-Phosphatpuffer zugesetzt. Je 4 pmol Nukleinsäure wurden bei 37°C für die in Abbildung 42 angegebene Dauer in 20 µl Ansätzen mit 14 µl humanem Serum (70 %iges Serum) inkubiert. Nach Ablauf der Reaktionszeit wurden die Proben nach Zugabe von 20 µl Stopp-Lösung ((2 % SDS, 50 mM EDTA, 6 M Harnstoff in einem Ethanol-Trockeneis-Bad schockgefroren und bei -80°C bis zum Ende des Versuches gelagert. Im Laufe der 96-stündigen Versuchsdauer verschob sich der pH-Wert des gepufferten Serums von pH 7,4 auf pH 8,5. Die Proben wurden am letzten Versuchstag Phenol-Chloroform extrahiert. Alle Arbeitsschritte wurden auf Eis oder in einer Kühlzentrifuge (4°C) durchgeführt. Die Proben wurde für 5 min. bei 95°C denaturiert, auf Eis abgekühlt und auf 10 %ige denaturierende Polyacrylamid-Gele aufgetragen. Nach gel-elektrophoretischer Trennung wurden die Banden mit Ethidiumbromid angefärbt und mittels UV-Licht visualisiert. Wie in Fig. 42 zu sehen ist, wird die FNA im Gegensatz zu RNA unter den gewählten Bedingungen nicht von Serumnukleasen angegriffen.

### Beispiel 6: Verwendete Oligonukleotide

Für die verschiedenen Reaktionen, wie sie in den Beispielen hierin verwendet wurden, wurden die folgenden Oligonukleotide verwendet.

### FNA-Selektion 1

### FNA-Selektion 1 mit FS Primer aus FNA (A)

### unterstrichen: T7 RNA-Polymerase-Promotor oder SP6-RNA-Polymerase-Promotor N_{OH}: Ribo-Nukleotid

**BSA-1A FNA-Pool (FNA, 83 nt)**
   GTGGAACCGACAGTGGTACGTG-N₄₀-CACGAGTGAAGTCTGAGCTCC
**BSA-1A initiale Bibliothek (DNA, 83 nt)**
   GTGGAACCGACAGTGGTACGTG-N₄₀-CACGAGTGAAGTCTGAGCTCC
**BSA-1A Forward Primer (DNA, 20 nt)**
   GTG GAA CCG ACA GTG GTA CG
**BSA-1A FS-Primer (FNA, zur FNA-Synthese, 20 nt)**
   GTG GAA CCG ACA GTG GTA CG
**BSA1 Reverse Primer T7 (DNA, 38 nt)**
   TCT AAT ACG ACT CAC TAT AGG AGC TCA GAC TTC ACT CG

### zu Testzwecken:

**BSA1 FS-Primer 3'rG (DNA, 15 nt)**
   5'-ACC GAC AGT GGT ACG_{OH}-3'
**BSA1 FS Primer 3rG +15 (DNA, 35 nt)**
   GTC CTA CCG_{OH} TCA GAT G_{OH}TG GAA CCG_{OH} ACA GTG_{OH} GTA CG_{OH}

### FNA-Selektion 1 (B und C) mit FS Primer aus RNA oder DNA

### (unter Verwendung von zwei überhängenden Primer und einem Forward-Synthese-Primer aus RNA oder aus DNA mit 3'-terminalem Ribonukleotid)

### unterstrichen: T7 RNA-Polymerase-Promotor oder SP6-RNA-Polymerase-Promotor N_{OH}: Ribo-Nukleotid

**BSA-1B FNA-Pool (FNA, 74 nt)**
   ACAGTGGTACGTG-N₄₀-CACGAGTGAAGTCTGAGCTCC
**BSA-1B initiale Bibliothek (DNA, 87 nt)**
   TGATGTGGAACCGACAGTGGTACGTG-N₄₀-CACGAGTGAAGTCTGAGCTCC
**BSA-1B Forward Primer (DNA, 24 nt)**
   TGA TGT GGA ACC GAC AGT GGT ACG TG
**BSA-1B Reverse Primer T7 (DNA, 38 nt)**
   TCT AAT ACG ACT CAC TAT AGG AGC TCA GAC TTC ACT CG
**BSA-1B FS-Primer (RNA, 13 nt)**
   UGA UGU GGA ACC G
**BSA-1B FS-Primer (DNA mit Ribonukleotiden,13 nt)**
   TGA TG U_{OH} GGA ACC G_{OH}
**BSA-1C FNA-Pool (FNA, 83nt)**
   GTGGAACCGACAGTGGTACGTGN40CACGAGTGAAGTCTGAGCTCC
**BSA-1C initiale Bibliothek (DNA, 83nt)**
   GTGGAACCGACAGTGGTACGTGN40CACGAGTGAAGTCTGAGCTCC
**BSA-1C Forward Primer (DNA, 40 nt)**
   AAT TGT CCT ACT CGT CAG ATG TGG AAC CGA CAG TGG TAC G
**BSA-1C Reverse Primer T7 (DNA, 38 nt)**
   TCT AAT ACG ACT CAC TAT AGG AGC TCA GAC TTC ACT CG
**BSA-1C FS Primer 3'-rG (DNA mit Ribonukleotiden, 21 nt)**
   AAT TGT CCT ACT CGT CAG ATG_{OH}
**BSA-1C FS-Primer 3-rG (DNA mit Ribonukleotiden, 21 nt)**
   AAT TG_{OH}T CCT ACT CG_{OH}T CAG ATG_{OH}

### FNA-Selektion 1 unter Verwendung der STAR-Technologie

### (unter Verwendung von zwei überhängenden Primem aus RNA oder aus DNA mit 3'-terminalem Ribonukleotid)

### kursiv: Nukleotide der Bibliotheken und der Ligationsmatrizen, die miteinander hybridisieren

### unterstrichen: T7 RNA-Polymerase-Promotor oder SP6-RNA-Polymerase-Promotor

N_{OH}: Ribo-Nukleotid
pN: 5'-Phosphat eines Nukleotids
3' dN: 2'-3'-didesoxy-Nukleotid
**BSA-1 STAR Strang während der Ligation** 
GCGAGTTCCTCTCAGCGT'GGGA-(dN)40—GTCC'TATAGTGAGTCGTATTAGTAG
TCGC
GGAGAGTCGCA.CCCT CAGG.ATATCACTCAGCATAATCATC AGCG
**BSA-1 STAR Bibliothek (FNA, 48 nt)**
   GGGA-(dN)₄₀-GTCC
**BSA-1 STAU Forward-Primer (DNA, 18 nt)**
   GCG AGT TCC TCT CAG CGT
**BSA-1 STAR Reverse-Primer (DNA, mit T7 RNA-Polymerase-Promotor, 29 nt)**
   GCG ACT ACT AAT ACG ACT CAC TAT A*GG AC*
**BSA-1 STAR Forward Matrix (DNA, 15 nt)**
   TCC CAC GCT GAG AG 3'dG
**BSA-1 STAR Reverse-Ligat (DNA, 25 nt)**
   pTAT AGT GAG TCG TAT TAG TAG TCG 3'dC
**BSA-1 STAR DNA FS-Primer (DNA mit Ribonukleotiden, 18 nt)**
   GCG AGT U_{OH} CC TCT CAG CG U_{oH}
**BSA-1 STAR RNA FS-Primer (RNA, 18 nt)**
   GCG AGU UCC UCU CAG CGU

### FNA-Selektion 2

### FNA-Selektion 2 mit FS-Primer als FNA (A)

### (unter Verwendung von Forward Synthese Primer aus FNA)

**BSA-2A FNA-Pool (FNA, 83 nt)**
   GTGGAACCGACAGTGGTACGTG-N₄₀-CACGAGTGAAGTCTGAGCTCC
**BSA-2A initiale Bibliothek (DNA, 83 nt)**
   GTGGAACCGACAGTGGTACGTG-N₄₀-CACGAGTGAAGTCTGAGCTCC
**BSA-2A Forward Primer (DNA, 20 nt)**
   GTG GAA CCG ACA GTG GTA CG
**BSA-2A FS-Primer (FNA zur FNA-Synthese, 20 nt)**
   GTG GAA CCG ACA GTG GTA CG
**BSA-2A Reverse Primer (DNA, 19 nt)**
   GG AGC TCA GAC TTC ACT CG

### FNA-Selektion 2 mit FS-Primer aus DNA oder RNA (B)

### (unter Verwendung von einem überhängenden Primem aus RNA oder aus DNA mit 3'-terminalem Ribonukleotid)

N_{OH}: Ribo-Nukleotid
**BSA- 2B FNA-Pool (FNA, 74 nt)**
   ACAGTGGTACGTG-N₄₀-CACGAGTGAAGTCTGAGCTCC
**BSA- 2B initiale Bibliothek (DNA, 87 nt)**
   TGATGTGGAACCGACAGTGGTACGTG-N₄₀-CACGAGTGAAGTCTGAGCTCC
**BSA-2B Forward Primer (DNA, 24 nt)**
   TGA TGT GGA ACC GAC AGT GGT ACG TG
**BSA-2B Reverse Primer (DNA, 19 nt)**
   GG AGC TCA GAC TTC ACT CG
**BSA-2B RNA FS-Primer (RNA, 13 nt)**
   UGA UGU GGA ACC G
**BSA-2B DNA FS-Primer (DNA mit Ribonukleotiden, 13 nt)**
   TGA TG U_{OH} GGA ACC **G_{OH}**

### FNA-Selektion 2 unter Verwendung der STAR-Technologie

### (unter Verwendung von einem überhängenden Primem aus RNA oder aus DNA mit 3'-terminalem Ribonukleotid)

### kursiv: Nukleotide der Bibliotheken und der Ligationsmatrizen, die miteinander hybridisieren

N_{OH}: Ribo-Nukleotid
pN: 5'-Phosphat eines Nukleotids
3'dN: 2'-3'-didesoxy-Nukleotid
**BSA-2 STAR Strang während der Ligation** 
GCGAGTTCCTCTCAGCGT'GGGA-(dN)40—
GTCC'TATAGTGAGTCGTATTAGTAGTCGC
GGAGAGTCGCA.CCCT CAGG.ATATCACTCAGCATAATCATCAGCG
**BSA-2 STAR Bibliothek (FNA, 48 nt)**
   GGGA-(dN)₄₀-GTCC
**BSA-2 STAR Forward-Primer (DNA, 18 nt)**
   GCG AGT TCC TCT CAG CGT
**BSA-2 STAR Reverse-Primer (DNA mit Ribonukleotiden, 29 nt)**
   GCG ACT ACT AAU_{OH} ACG ACT CAC TAT I_{OH} *GG AC*
**BSA-2 STAR Forward Matrix (DNA, 15 nt)**
   TCC CAC GCT GAG AG 3'dG
**BSA-2 STAR Reverse-Ligat (DNA, 25 nt)**
   pTAT AGT GAG TCG TAT TAG TAG TCG 3'dC
**BSA-2 STAR RNA FS Primer (RNA, 18 nt)**
   GCG AGU UCC UCU CAG CGU
**BSA-2 STAR DNA FS-Primer (DNA mit Ribonukleotiden, 18 nt)**
   GCG AGT U_{OH}CC TCT CAG CGU_{OH}

### FNA-Selektion 3

**BSA-3 FNA-Pool (FNA, 83 nt)**
   5'-GTGGAACCGACAGTGGTACGTG-N₄₀-CACGAGTGAAGTCTGAGCTCC-3'
**BSA-3 initiale Bibliothek (DNA, 83 nt)**
   5'-GTGGAACCGACAGTGGTACGTG-N₄₀-CACGAGTGAAGTCTGAGCTCC-3'
**BSA-3 Forward Primer (FNA, 20 nt)**
   5'-GTG GAA CCG ACA GTG GTA CG-3'
**BSA-3 Reverse Primer (DNA, 19 nt)**
   5'Biotin-GG AGC TCA GAC TTC ACT CG-3'
**BSA-3 Forward Sequenzierungs-Primer (DNA, 20 nt)**
   5'-GTG GAA CCG ACA GTG GTA CG-3'
**BSA-3 Reverse Sequenzierungs-Primer (DNA, 19 nt)**
   5'-GG AGC TCA GAC TTC ACT CG-3'

### DNA-Selektion mit FS Primer aus DNA

### unterstrichen: T7 RNA-Polymerase-Promotor oder SP6-RNA-Polymerase-Promotor

**DNA-Pool (DNA, 83 nt)**
   GTGGAACCGACAGTGGTACGTG-N₄₀-CACGAGTGAAGTCTGAGCTCC
**DNA initiale Bibliothek (DNA, 83 nt)**
   GTGGAACCGACAGTGGTACGTG-N₄₀-CACGAGTGAAGTCTGAGCTCC
**DNA Forward Primer (DNA, 20 nt)**
   GTG GAA CCG ACA GTG GTA CG
**DNA Reverse Primer T7 (DNA, 38 nt)**
   TCT AAT ACG ACT CAC TAT AGG AGC TCA GAC TTC ACT CG

### Literatur

Die verschiedenen, hierin enthaltenen Literaturzitate lauten vollständig wie folgt, wobei deren Offenbarung hierin unter Bezugnahme aufgenommen wird.
Aurup, H., Williams, D.M. and Eckstein, F. (1992) 2'-Fluoro- and 2'-amino-2'-deoxynucleoside 5'-triphosphates as substrates for T7 RNA polymerase. Biochemistry, 31, 9636-9641.
Bell, C., Lynam, E., Landfair, D.J., Janjic, N. and Wiles, M.E. (1999) Oligonucleotide NX1838 inhibits VEGF165-mediated cellular responses in vitro. In Vitro Cell Dev Biol Anim, 35, 533-542.
Bock, L.C., Griffin, L.C., Latham, J.A., Vermaas, E.H. and Toole, J.J. (1992) Selection of single-stranded DNA molecules that bind and inhibit human thrombin. Nature, 355, 564-566.
Chiu, Y.L. and Rana, T.M. (2003) siRNA function in RNAi: A chemical modification analysis. Rna, 9, 1034-1048.
Cummins, L.L., Owens, S.R., Risen, L.M., Lesnik, E.A., Freier, S.M., McGee, D., Guinosso, C.J. and Cook, P.D. (1995) Characterization of fully 2'-modified oligoribonucleotide hetero- and homoduplex hybridization and nuclease sensitivity. Nucleic Acids Res, 23, 2019-2024.
Eaton, B.E. and Pieken, W.A. (1995) Ribonucleosides and RNA. Annu Rev Biochem, 64, 837-863.
Green, L.S., Jellinek, D., Bell, C., Beebe, L.A., Feistner, B.D., Gill, S.C., Jucker, F.M. and Janjic, N. (1995) Nuclease-resistant nucleic acid ligands to vascular permeability factor/vascular endothelial factor. Chem Biol, 2, 683-695.
Green, L.S., Jellinek, D., Jenison, R., Ostman, A., Heldin, C.H. and Janjic, N. (1996) Inhibitory DNA ligands to platelet-derived growth factor B-chain. Biochemistry, 35, 14413-14424.
Griffin, L.C., Tidmarsh, G.F., Bock, L.C., Toole, J.J. and Leung, L.L. (1993) In vivo anticoagulant properties of a novel nucleotide-based thrombin inhibitor and demonstration of regional anticoagulation in extracorporeal circuits. Blood, 81, 3271-3276.
Henry, S.P., Geary, R.S., Yu, R. and Levin, A.A. (2001) Drug properties of second-generation antisense oligonucleotides: how do they measure up to their predecessors? Curr Opin Investig Drugs, 2, 1444-1449.
Jellinek, D., Green, L.S., Bell, C., Lynott, C.K., Gill, N., Vargeese, C., Kirschenheuter, G., McGee, D.P.C., Abesinghe, P., Pieken, W.A., Shapiro, R., Rifkin, D.B., Moscatelli, D. and Janjic, N. (1995) Potent 2'-amino-2'deoxypyrimidine RNA inhibitors of basic fibroblast growth factor. Biochensistry, 34, 11363-11372.
Jhaveri, S., Olwin, B. and Ellington, A.D. (1998) In vitro selection of phosphorothiolated aptamers. Bioorg Med Chem Lett, 8, 2285-2290.
King, D.J., Bassett, S.E., Li, X., Fennewald, S.A., Herzog, N.K., Luxon, B.A., Shope, R. and Gorenstein, D.G. (2002) Combinatorial selection and binding of phosphorothioate aptamers targeting human NF-kappa B RelA(p65) and p50. Biochemistry, 41, 9696-9706.
Kujau, M.J., Siebert, A. and Wolfl, S. (1997) Design of leader sequences that improve the efficiency of the enzymatic synthesis of 2'-amino-pyrimidine RNA for in vitro selection. J Biochem Biophys Methods, 35, 141-151.
Kusser, W. (2000) Chemically modified nucleic acid aptamers for in vitro selections: evolving evolution. J Biotechnol, 74, 27-38.
Leva, S., Lichte, A., Burmeister, J., Muhn, P., Jahnke, B., Fesser, D., Erfurth, J., Burgstaller, P. and Klussmann, S. (2002) GnRH binding RNA and DNA Spiegelmers: a novel approach toward GnRH antagonism. Chem Biol, 9, 351-359.
Lin, Y., Qiu, Q., Gill, S.C. and Jayasena, S.D. (1994) Modified RNA sequence pools for in vitro selection. Nucleic Acids Research, 22, 5229-5234.
Manoharan, M. (1999) 2'-carbohydrate modifications in antisense oligonucleotide therapy: importance of conformation, configuration and conjugation. Biochim Biophys Acta, 1489, 117-130.
Meador, J.W., 3rd, McElroy, H.E., Pasloske, B.L., Milburn, S.C. and Winkler, M.M. (1995) pTRIPLEscript: a novel cloning vector for generating in vitro transcripts from tandem promoters for SP6, T7 and T3 RNA polymerase. Biotechniques, 18, 152-157.
Milligan, J.F. and Uhlenbeck, O.C. (1989) Synthesis of small RNAs using T7 RNA polymerase. Methods Enzymol, 180, 51-62.
Murphy, M.B., Fuller, S.T., Richardson, P.M. and Doyle, S.A. (2003) An improved method for the in vitro evolution of aptamers and applications in protein detection and purification. Nucleic Acids Res, 31, e110.
Ono, T., Scalf, M. and Smith, L.M. (1997) 2'-Fluoro modified nucleic acids: polymerase-directed synthesis, properties and stability to analysis by matrix-assisted laser desorption/ionization mass spectrometry. Nucleic Acids Res, 25, 4581-4588.
Padilla, R. and Sousa, R. (1999) Efficient synthesis of nucleic acids heavily modified with non-canonical ribose 2'-groups using a mutantT7 RNA polymerase (RNAP). Nucleic Acids Res, 27, 1561-1563.
Padilla, R. and Sousa, R. (2002) A Y639F/H784A T7 RNA polymerase double mutant displays superior properties for synthesizing RNAs with non-canonical NTPs. Nucleic Acids Res, 30, e138.
Richardson, F.C., Kuchta, R.D., Mazurkiewicz, A. and Richardson, K.A. (2000) Polymerization of 2'-fluoro- and 2'-O-methyl-dNTPs by human DNA polymerase alpha, polymerase gamma, and primase. Biochem Pharmacol, 59, 1045-1052.
Stryer, L. (1995) Biochemistry. W. H. Freeman and Company, New York.
Williams, K.P., Liu, X.H., Schumacher, T.N., Lin, H.Y., Ausiello, D.A., Kim, P.S. and Bartel, D.P. (1997) Bioactive and nuclease-resistant L-DNA ligand of vasopressin. Proc Natl Acad Sci USA, 94, 11285-11290.
Xu, Y., Zhang, H.Y., Thormeyer, D., Larsson, O., Du, Q., Helmen, J., Wahlestedt, C. and Liang, Z. (2003) Effective small interfering RNAs and phosphorothioate antisense DNAs have different preferences for target sites in the luciferase mRNAs. Biochem Biophys Res Commun, 306, 712-717.
Zhang, H.Y., Mao, J., Zhou, D., Xu, Y., Thonberg, H., Liang, Z. and Wahlestedt, C. (2003) mRNA accessible site tagging (MAST): a novel high throughput method for selecting effective antisense oligonucleotides. Nucleic Acids Res, 31, e72.

## Patentansprüche

1. Verfahren zur Synthese einer Nukleinsäure, wobei die Nukleinsäure modifizierte Nukleotide umfasst, umfassend die Schritte:
- Bereitstellen eines Matrizenstranges;
- Bereitstellen eines zumindest teilweise an dem Matrizenstrang hybridisierenden Primers;
- Bereitstellen von Nukleosidtriphosphaten, wobei ein Teil der Nukleosidtriphosphate modifizierte Nukleosidtriphosphate sind;
- Bereitstellen einer Polymerase-Aktivität; und
- Inkubation des Matrizenstranges, des Primers, der Nukleosidtriphosphate zur Synthese einer zum Matrizenstrang im wesentlichen komplementären Nukleinsäure,
**dadurch gekennzeichnet, dass** die Polymerase-Aktivität eine reverse Transkriptase ist, die ausgewählt ist aus der Gruppe, die Reverse Transkriptasen von Murine Moloney Leukemia Virus (MMLV), Avian Myeloblastosis Virus (AMV), thermostabile Reverse Transkriptasen, DNA-Polymerase von *Carboxydothermus hydrogenoformans* jeweilige Mutanten davon und Gemische davon umfasst, und wobei
die modifizierten Nukleosidtriphosphate ausgewählt sind aus der Gruppe, die 2'-Fluoro-modifizierte Nukleosidtriphosphate, 2'-Amino-modifizierte Nukleosidtriphosphate, 2'-Azido-modifizierte Nukleosidtriphosphate, 2'-O-Methyl-modifizierte Nukleosidtriphosphate, 2'-Alkyl-modifizierte Nukleosidtriphosphate und 2'-Allyl-modifizierte Nukleosidtriphosphate umfasst.

2. Verfahren nach Anspruch 1, **dadurch gekennzeichnet, dass** die modifizierten Nukleosidtriphosphate 2'-Fluoro-Nukleosidtriphosphate sind.

3. Verfahren nach einem der Ansprüche 1 bis 2, **dadurch gekennzeichnet, dass** die bereitgestellten Nukleosidtriphosphate ausschließlich modifizierte Nukleosidtriphosphate sind und die synthetisierte Nukleinsäure ausschließlich aus modifizierten Nukleotiden besteht.

4. Verfahren nach einem der Ansprüche 1 bis 3, **dadurch gekennzeichnet, dass** der Matrizenstrang aus einer 2'-Fluoro-Nukleinsäure besteht.

5. Verfahren nach einem der Ansprüche 1 bis 4, **dadurch gekennzeichnet, dass** die Nukleotide des Primers von denen der zu synthetisierenden Nukleinsäure verschieden sind.

6. Verfahren nach einem der Ansprüche 1 bis 5, **dadurch gekennzeichnet, dass** der Primer aus modifizierten Nukleosidphosphaten besteht, wobei die Modifikation der Nukleosidphosphate des Primers die gleiche Modifikation wie die der bereitgestellten Nukleosidtriphosphate ist.

7. Verfahren nach einem der Ansprüche 1 bis 6, **dadurch gekennzeichnet, dass** der Primer aus DNA besteht, wobei mindestens das 3'-terminale Nukleotid des Primers ein Ribonukleotid ist.

8. Verfahren nach einem der Ansprüche 1 bis 7, **dadurch gekennzeichnet, dass** der Primer oder ein Teil davon von der durch die Polymerase-Aktivität synthetisierten Nukleinsäure entfernt wird.

9. Verfahren nach einem der Ansprüche 1 bis 8, **dadurch gekennzeichnet, dass** der Matrizenstrang und/oder der Primer verdaut oder gespalten wird nach der Synthese der zum Matrizenstrang im wesentlichen komplementären Nukleinsäure.

10. Verfahren nach einem der Ansprüche 8 bis 9, **dadurch gekennzeichnet, dass** das Entfernen und/oder das Spalten oder der Verdau durch alkalische Spaltung oder enzymatische Aktivität erfolgt.

11. Verwendung einer reversen Transkriptase zur Synthese einer Nukleinsäure, wobei die Nukleinsäure mindestens ein modifiziertes Nukleosidphosphat enthält, und das modifizierte Nukleosidtriphosphat ausgewählt ist aus der Gruppe, die 2'-Fluoro-modifizierte Nukleosidtriphosphate, 2'-Amino-modifizierte Nukleosidtriphosphate, 2'-Azido-modifizierte Nukleosidtriphosphate, 2'-O-Methyl-modifizierte Nukleosidtriphosphate, 2'-Alkyl-modifizierte Nukleosidtriphosphate und 2'-Allyl-modifizierte Nukleosidtriphosphate umfasst, und
die reverse Transkriptase ausgewählt ist aus der Gruppe, die Reverse Transkriptasen von Murine Moloney Leukemia Virus (MMLV), Avian Myeloblastosis Virus (AMV), thermostabile Reverse Transkriptasen, DNA-Polymerase von *Carboxydothermus hydrogenofornrans*, Mutanten davon und Gemische davon umfasst.

12. Verwendung nach Anspruch 11, **dadurch gekennzeichnet, dass** das modifizierte Nukleosidtriphosphat 2'-Fluoro-modifiziertes Nukleosidtriphosphat ist.

13. Verwendung nach einem der Ansprüche 11 bis 12, **dadurch gekennzeichnet, dass** die Nukleinsäure vollständig aus modifizierten Nukleosidphosphaten besteht.

## Claims

1. A method for the synthesis of a nucleic acid, whereby the nucleic acid comprises modified nucleotides, comprising the steps of:
- providing a template strand;
- providing a primer which is at least partially hybridizing to the template strand;
- providing nucleoside triphosphates, whereby a portion of the nucleoside triphosphates are modified nucleoside triphosphates;
- providing a polymerase activity; and
- incubating the template strand, the primers, the nucleoside triphosphates for the synthesis of a nucleic acid which is essentially complementary to the template strand,
**characterized in that** the polymerase activity is a reverse transcriptase activity, whereby the reverse transcriptase is selected from the group comprising reverse transcriptases of murine moloney leukemia virus (MMLV), avian myeloblastosis virus (AMV), thermostable reverse transcriptases, DNA polymerase of *Carboxydothermus hydrogenoformans*, respective mutants thereof, and mixtures thereof and
wherein the modified nucleoside triphosphates are selected from the group comprising 2'-fluoro-modified nucleoside triphosphates, 2'-amino-modified nucleoside triphosphates, 2'-azido-modified nucleoside triphosphates, 2'-O-methyl-modified nucleoside triphosphates, 2'-alkyl-modified nucleoside triphosphates and 2'-allyl-modified nucleoside triphosphates.

2. The method according to claim 1, **characterized in that** the modified nucleoside triphosphates are 2'-fluoro nucleoside triphosphates.

3. The method according to any one of claims 1 to 2, **characterized in that** the nucleoside triphosphates provided are only modified nucleoside triphosphates and that the synthesized nucleic acid consists solely of modified nucleotides.

4. The method according to any one of claims 1 to 3, **characterized in that** the template strand consists of a 2'-fluoro nucleic acid.

5. The method according to any one of claims 1 to 4, **characterized in that** the nucleotides of the primer are different from the nucleotides of the nucleic acid to be synthesized.

6. The method according to any one of claims 1 to 5, **characterized in that** the primer consists of modified nucleoside phosphates, whereby the modification of the nucleoside phosphates of the primer is the same modification as the one of the nucleoside triphosphates provided.

7. The method according to any one of claims 1 to 6, **characterized in that** the primer consists of DNA, whereby at least the 3' terminal nucleotide of the primer is a ribonucleotide.

8. The method according to any one of claims 1 to 7, **characterized in that** the primer or a part thereof is removed from the nucleic acid which is synthesized by the polymerase activity.

9. The method according to any one of claims 1 to 8, **characterized in that** the template strand and/or the primer is/are digested or cleaved after the synthesis of the nucleic acid which is essentially complementary to the template strand.

10. The method according to any one of claims 8 to 9, **characterized in that** the separation and/or the cleavage or the digestion is performed by alkaline cleavage or enzymatic activity.

11. Use of a reveres transcriptase for the synthesis of a nucleic acid, whereby the nucleic acid comprises at least a modified nucleoside phosphate, whereby the modified nucleoside triphosphate is selected from the group comprising 2'-fluoro-modified nucleoside triphosphates, 2'-amino-modified nucleoside triphosphates, 2'-azido-modified nucleoside triphosphates, 2'-O-methyl-modified nucleoside triphosphates, 2'-alkyl-modified nucleoside triphosphates and 2'-allyl-modified nucleoside triphosphates, and whereby the reverse transcriptase is selected from the group comprising reverse transcriptases of murine moloney leukemia virus (MMLV), avian myeloblastosis virus (AMV), thermostable reverse transcriptases, DNA polymerase of *Carboxydothermus hydrogenoformans*, mutants thereof, and mixtures thereof.

12. Use according to claim 11, **characterized in that** the modified nucleoside triphosphate is 2'-fluoro nucleoside triphosphate.

13. Use according to any one of claims 11 to 12, **characterized in that** the nucleic acid consists completely of modified nucleoside phosphates.

## Revendications

1. Procédé de synthèse d'un acide nucléique, pour lequel l'acide nucléique comprend des nucléotides modifiés, comprenant les étapes suivantes:
- fournir un brin matrice;
- fournir un primaire qui s'hybridise au moins partiellement avec le brin matrice;
- fournir des triphosphates de nucléoside, pour lesquels une partie des triphosphates de nucléoside sont des triphosphates de nucléoside modifiés;
- fourni une activité polymérase; et
- faire incuber le brin matrice, les primaires, les triphosphates de nucléoside pour la synthèse d'un acide nucléique qui est essentiellement complémentaire au brin matrice,
**caractérisé en ce que** l'activité polymérase est une activité transcriptase inverse pour laquelle la transcriptase inverse est sélectionnée dans le groupe comprenant les transcriptases inverses du virus de la leucémie murine de Moloney (MMLV), du virus de la myéloblastose aviaire (AMV), les transcriptases inverses thermostables, les ADN polymérases de *Carboxydothermus hydrogenoformans*, des mutants respectifs de ceux-ci, et des mélanges de ceux-ci, et
dans lequel les triphosphates de nucléoside modifiés sont sélectionnés dans le groupe comprenant les triphosphates de nucléoside modifiés en 2'-fluoro, les triphosphates de nucléoside modifiés en 2'-amino, les triphosphates de nucléoside modifiés en 2'-azido, les triphosphates de nucléoside modifiés en 2'-O-méthyle, les triphosphates de nucléoside modifiés en 2'-alkyle et les triphosphates de nucléoside modifiés en 2'-allyle.

2. Procédé selon la revendication 1, **caractérisé en ce que** les triphosphates de nucléoside modifiés sont des triphosphates de nucléoside modifiés en 2'-fluoro.

3. Procédé selon l'une quelconque des revendications 1 à 2, **caractérisé en ce que** les triphosphates de nucléoside fournis sont uniquement des triphosphates de nucléoside modifiés et **en ce que** l'acide nucléique synthétisé se compose uniquement de nucléotides modifiés.

4. Procédé selon l'une quelconque des revendications 1 à 3, **caractérisé en ce que** le brin matrice se compose d'un acide nucléique en 2'-fluoro.

5. Procédé selon l'une quelconque des revendications 1 à 4, **caractérisé en ce que** les nucléotides du primaire sont différents des nucléotides de l'acide nucléique à synthétiser.

6. Procédé selon l'une quelconque des revendications 1 à 5, **caractérisé en ce que** le primaire se compose de phosphates de nucléoside modifiés, pour lesquels la modification des phosphates de nucléoside du primaire est la même modification que celle des triphosphates de nucléoside fournis.

7. Procédé selon l'une quelconque des revendications 1 à 6, **caractérisé en ce que** le primaire se compose de ADN, pour lequel au moins le nucléotide terminal 3' du primaire est un ribonucléotide.

8. Procédé selon l'une quelconque des revendications 1 à 7, **caractérisé en ce que** le primaire ou une partie de celui-ci est enlevé de l'acide nucléique qui est synthétisé par l'activité polymérase.

9. Procédé selon l'une quelconque des revendications 1 à 8, **caractérisé en ce que** le brin matrice et/ou le primaire est/sont digéré(s) ou clivé(s) après la synthèse de l'acide nucléique qui est essentiellement complémentaire au brin matrice.

10. Procédé selon l'une quelconque des revendications 8 à 9, **caractérisé en ce que** la séparation et/ou le clivage ou la digestion est effectué(e) par clivage alcalin ou par activité enzymatique.

11. Utilisation d'une transcriptase inverse pour la synthèse d'un acide nucléique, pour laquelle l'acide nucléique comprend au moins un phosphate de nucléoside modifié, pour laquelle le triphosphate de nucléoside modifié est sélectionné dans le groupe comprenant les triphosphates de nucléoside modifiés en 2'-fluoro, les triphosphates de nucléoside modifiés en 2'-amino, les triphosphates de nucléoside modifiés en 2'-azido, les triphosphates de nucléoside modifiés en 2'-O-méthyle, les triphosphates de nucléoside modifiés en 2'-alkyle et les triphosphates de nucléoside modifiés en 2'-allyle, et pour laquelle la transcriptase inverse est sélectionnée dans le groupe comprenant les transcriptases inverses du virus de la leucémie murine de Moloney (MMLV), du virus de la myéloblastose aviaire (AMV), les transcriptases inverses thermostables, les ADN polymérases de *Carboxydothermus hydrogenoformans*, des mutants respectifs de ceux-ci, et des mélanges de ceux-ci.

12. Utilisation selon la revendication 11, **caractérisée en ce que** le triphosphate de nucléotide modifié est un triphosphate de nucléoside modifié en 2'-fluoro.

13. Utilisation selon l'une quelconque des revendications 11 à 12, **caractérisée en ce que** l'acide nucléique est entièrement constitué de phosphates de nucléoside modifiés.
